# EUROPEAN PATENT APPLICATION

(11) **EP 4 825 397 A1**
(43) Date of publication of application: **21.10.2026**
(21) Application number: 25171234.5
(22) Date of filing: 17.04.2025
(51) Int. Cl.: A61K 40/11, A61K 40/31, A61K 40/42

(54) **STABILIZED CHIMERIC ANTIGEN RECEPTORS**

(71) Applicant: Kemijski Institut, 1000 Ljubljana (SI)
(72) Inventor: JERALA, Roman, 1000 Ljubljana (SI); FINK, Tina, 1000 Ljubljana (SI); KLEMENCIC , Kaja, 1000 Ljubljana (SI)
(74) Representative: Mewburn Ellis LLP

(57) **Abstract**

The present application describes novel chimeric antigen receptors (CARs) comprising novel interaction domains. In some embodiments, the CARs are stabilized by one or more disulfide bonds between cysteine residues of interaction domains. Also provided are cells comprising the CAR, a nucleic acid or a plurality of nucleic acids encoding the CAR, an expression vector or a plurality of expression vectors comprising said nucleic acids, methods of making such molecules, and the use of such molecules in a method of medical treatment or prophylaxis.

## Description

### Field of the Invention

The present disclosure relates to the fields of molecular biology, more specifically chimeric antigen receptor (CAR) technology. The present disclosure also relates to methods of medical treatment and prophylaxis.

### Background

CAR T cell therapy has emerged as a transformative treatment for hematologic malignancies, utilizing genetically engineered T cells to recognize and kill tumor cells through chimeric antigen receptors (CARs). Many CAR constructs employ a single-chain variable fragment (scFv) derived from antibodies, comprising a variable light (V_{L}) and variable heavy (V_{H}) chain, to specifically recognize tumor-associated antigens. The affinity of the scFv plays a key role in determining the function of a CAR for its target antigen and target cells. Generally, CAR T cells with a high-affinity scFv domains demonstrate stronger anti-tumor activity but are more likely to trigger on-target, off-tumor toxicity. Alternatively, lowering the affinity of the scFv can improve tumor specificity and reduce toxicity to normal tissues; however, it can result in a weaker anti-tumor response (1-3). Besides the scFv, traditional second-generation CARs contain a signaling domain, which is essential for T-cell activation, persistence, and the effective elimination of cancer cells (4). In addition to cancer immunotherapy, CAR-based therapeutic cells can be used to treat other diseases by targeting specific cell types involved in pathology, such as autoimmune diseases (e.g., lupus or diabetes) (5).

When CAR T cells engage target cells, an immunological synapse is formed - a physically active structure capable of exerting mechanical force. Cells utilize these synaptic forces to regulate the activity of outgoing chemical signals and to enhance their effector responses, such as cytotoxic activity (6). However, scFvs are less stable than antibodies due to the absence of constant immunoglobulin domains, making them more prone to unfolding or aggregation (7). In antibodies, an intermolecular disulfide bond stabilizes the constant heavy 1 (C_{H}1) and constant light (C_{L}) chains, whereas the majority of native V_{H}-V_{L} pair lacks such a stabilizing covalent bond, resulting in lower stability (8). Consequently, when CAR T cells engage target cancer cells, the mechanical force exerted during immune synapse formation may cause scFv domain unfolding. This can impair antigen recognition, particularly when targeting cancer cells with low antigen density, thereby reducing the effectiveness of CAR T cells.

Therefore, there is a compelling need for CAR designs that enhance the mechanical stability of the antigen-binding domain to sustain effective antigen engagement and immune activation, even under mechanical stress. Disulfide-stabilized scFvs have been engineered by introducing a non-native interdomain disulfide bond between V_{H} and V_{L} through site-specific cysteine substitutions, which are not always possible and mutations in the core of the protein may affect the affinity of scFv for the target antigen. These scFvs exhibit increased stability and are less prone to adopting the open (disassembled) state (8-11). However, this stabilization strategy requires a customized approach, as the V_{H}-V_{L} disulfide bridge must be designed individually for each scFv domain.

The present invention has been devised in light of the above considerations.

### Summary of the Invention

In a first aspect, the present disclosure provides a polypeptide comprising (i) a first interaction domain comprising a cysteine, (ii) an antigen-binding domain which binds specifically to a target antigen, and (ii) a second interaction domain comprising a cysteine.

In some embodiments, the first interaction domain and the second interaction domain interact through a disulfide bond between cysteine residues.

In some embodiments, the polypeptide comprises: (i) a first interaction domain comprising a cysteine, (ii) an antigen-binding domain which binds specifically to a target antigen, and (ii) a second interaction domain comprising a cysteine, wherein the first interaction domain and the second interaction domain interact through a disulfide bond between cysteine residues.

In a first aspect, the present disclosure provides a (CAR) comprising (i) a first interaction domain comprising a cysteine, (ii) an antigen-binding domain which binds specifically to a target antigen, and (ii) a second interaction domain comprising a cysteine.

In some embodiments, the CAR comprises:
(i) a first interaction domain comprising a cysteine,
(ii) an antigen-binding domain which binds specifically to a target antigen,
(iii) a second interaction domain comprising a cysteine,
(iv) a hinge,
(v) a transmembrane domain, and
(vi) a signalling domain.

In some embodiments, the first interaction domain and the second interaction domain interact through a disulfide bond between cysteine residues.

In some embodiments, the CAR comprises:
(i) a first interaction domain comprising a cysteine,
(ii) an antigen-binding domain which binds specifically to a target antigen,
(iii) a second interaction domain comprising a cysteine,
(iv) a hinge,
(v) a transmembrane domain, and
(vi) a signalling domain,
wherein the first interaction domain and the second interaction domain interact through a disulfide bond between cysteine residues.

In some embodiments, the first interaction domain comprises two cysteines and the second interaction domain comprises two cysteines.

In some embodiments, the first interaction domain and the second interaction domain interact through two disulfide bonds.

In some embodiments, the first interaction domain and the second interaction domain interact to form a coiled-coil heterodimer.

In some embodiments, the interaction domain comprises a heptad repeat,

In some embodiments, the interaction domain comprises a cysteine at position d of the heptad repeat.

In some embodiments, the interaction domain comprises less than 50 amino acid residues. In some embodiments, the interaction domain comprises less than 30 amino acid residues.

In some embodiments, the interaction domain comprises at least 7 amino acid residues. In some embodiments, the interaction domain comprises at least 14 amino acid residues. In some embodiments, the interaction domain comprises at least 21 amino acid residues.

In some embodiments, the interaction domain comprises at least 7 amino acid residues.
first interaction domain and/or second interaction domain comprises an amino acid sequence having at least 70% amino acid sequence identity to any one of SEQ ID NOs: 1-8.

In some embodiments,
(i) the first interaction domain comprises SEQ ID NO:1, and the second interaction domain comprises SEQ ID NO:2,
(ii) the first interaction domain comprises SEQ ID NO:2, and the second interaction domain comprises SEQ ID NO:1,
(iii) the first interaction domain comprises SEQ ID NO:3, and the second interaction domain comprises SEQ ID NO:4,
(iv) the first interaction domain comprises SEQ ID NO:4, and the second interaction domain comprises SEQ ID NO:3,
(v) the first interaction domain comprises SEQ ID NO:5, and the second interaction domain comprises SEQ ID NO:6,
(vi) the first interaction domain comprises SEQ ID NO:6, and the second interaction domain comprises SEQ ID NO:5,
(vii) the first interaction domain comprises SEQ ID NO:7, and the second interaction domain comprises SEQ ID NO:8, or
(viii) the first interaction domain comprises SEQ ID NO:8, and the second interaction domain comprises SEQ ID NO:7.

In some embodiments, the antigen-binding domain comprises an antibody-derived antigen-binding molecule.

In some embodiments, the antigen-binding domain comprises a single chain variable fragment (scFv) or a variable domain of the heavy chain of a heavy-chain antibody (VHH).

In some embodiments, the CAR is a multispecific CAR.

In some embodiments, the CAR is a multispecific CAR, wherein each antigen binding domain is independently stabilized by two interaction domains.

A multispecific chimeric antigen receptor (CAR) is provided, the multispecific CAR comprising:
(i) a first interaction domain comprising a cysteine,
(ii) a first antigen-binding domain which binds specifically to a target antigen,
(ii) a second interaction domain comprising a cysteine,
(iv) a linker
(v) a third interaction domain comprising a cysteine,
(vi) a second antigen-binding domain which binds specifically to a target antigen,
(vii) a fourth interaction domain comprising a cysteine,
(viii) a hinge,
(ix) a transmembrane domain, and
(x) a signalling domain,
wherein the first interaction domain and the second interaction domain interact through a disulfide bond between cysteine residues, and the third interaction domain and the fourth interaction domain interact through a disulfide bond between cysteine residues.

In some embodiments, the hinge comprises an amino acid sequence which is, or which is derived from, the amino acid sequence of a CD8α hinge
In some embodiments, the transmembrane domain comprises an amino acid sequence which is, or which is derived from, the amino acid sequence of a CD8α transmembrane domain.

In some embodiments, the hinge comprises or consists of an amino acid sequence having at least 70% amino acid sequence identity to SEQ ID NO:41.

In some embodiments, the transmembrane domain comprises or consists of an amino acid sequence having at least 70% amino acid sequence identity to SEQ ID NO:42
In some embodiments, the signalling domain comprises an amino acid sequence which is, or which is derived from, the amino acid sequence of a CD3ζ signalling domain.

In some embodiments, the signalling domain comprises an amino acid sequence which is, or which is derived from, the amino acid sequence of a 4-1BB costimulatory domain
In some embodiments, the signalling domain comprises an amino acid sequence having at least 70% amino acid sequence identity to any one of SEQ ID NOs 43, 44, 45, and/or 46.

In some embodiments, the signalling domain comprises an amino acid sequence having at least 70% amino acid sequence identity to SEQ ID NO:45, and comprises an amino acid sequence having at least 70% amino acid sequence identity to SEQ ID NO:46.

In some embodiments, the target antigen is a tumor associated antigen or an antigen expressed on the surface of a cell involved in autoimmune disease.

In some embodiments, the target antigen is CD19 or BCMA.

A nucleic acid, or a plurality of nucleic acids, encoding a polypeptide or CAR according to the present disclosure, is also provided.

An expression vector, or a plurality of expression vectors, comprising a nucleic acid or a plurality of nucleic acids according to the present disclosure, is also provided.

A method comprising culturing a cell under conditions suitable for expression of an CAR by the cell, is also provided.

A cell comprising a polypeptide according to the present disclosure, a CAR according to the present disclosure, a nucleic acid according to the present disclosure, or an expression vector or a plurality of expression vectors according to the present disclosure, is also provided.

A composition comprising a polypeptide according to the present disclosure, a CAR according to the present disclosure, a nucleic acid or a plurality of nucleic acids according to the present disclosure, an expression vector or a plurality of expression vectors according to the present disclosure, or a cell according to the present disclosure, and a pharmaceutically acceptable carrier, diluent, excipient or adjuvant.

A polypeptide according to the present disclosure, a CAR according to the present disclosure, a nucleic acid or a plurality of nucleic acids according to the present disclosure, an expression vector or a plurality of expression vectors according to the present disclosure, a cell according to the present disclosure, or a composition according to the present disclosure, for use in a method of medical treatment or prophylaxis.

A polypeptide according to the present disclosure, a CAR according to the present disclosure, a nucleic acid or a plurality of nucleic acids according to the present disclosure, an expression vector or a plurality of expression vectors according to the present disclosure, a cell according to the present disclosure, or a composition according to the present disclosure, for use in the treatment or prevention of a cancer or an autoimmune disease.

A method of treating or preventing a cancer or an autoimmune disease, the method comprising administering a therapeutically- or prophylactically-effective amount of a polypeptide according to the present disclosure, a CAR according to the present disclosure, a nucleic acid or a plurality of nucleic acids according to the present disclosure, an expression vector or a plurality of expression vectors according to the present disclosure, a cell according to the present disclosure, or a composition according to the present disclosure.

Use of a polypeptide according to the present disclosure, a CAR according to the present disclosure, a nucleic acid or a plurality of nucleic acids according to the present disclosure, an expression vector or a plurality of expression vectors according to the present disclosure, a cell according to the present disclosure, or a composition according to the present disclosure, in the manufacture of a medicament for the treatment or prevention of a cancer or an autoimmune disease.

Use of a polypeptide according to the present disclosure, a CAR according to the present disclosure, a nucleic acid or a plurality of nucleic acids according to the present disclosure, an expression vector or a plurality of expression vectors according to the present disclosure, a cell according to the present disclosure, or a composition according to the present disclosure to deplete or increase killing of cells expressing the target antigen for the CAR.

An *in vitro* complex, optionally isolated, comprising a polypeptide according to the present disclosure, a CAR according to the present disclosure, or a cell according to the present disclosure, bound to the target antigen for the CAR.

The invention includes the combination of the aspects and preferred features described except where such a combination is clearly impermissible or expressly avoided.

### Summary of the Figures

Embodiments and experiments illustrating the principles of the invention will now be discussed with reference to the accompanying figures in which:

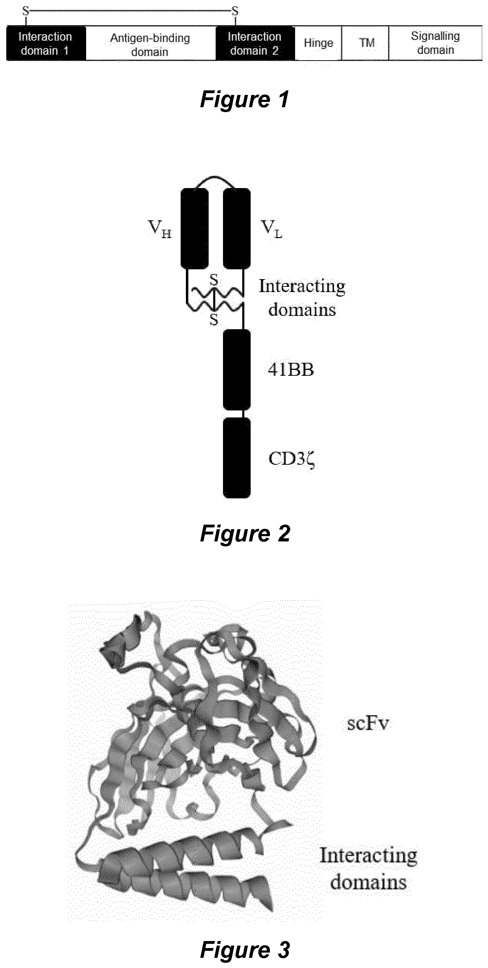

**Figure 1****.** Schematic representation of a CAR construct with disulfide-stabilized antigen-binding domain. A scheme of a CAR construct incorporating engineered interaction domains at the N- and C-termini of the antigen-binding domain. The interaction domains include cysteine residues that can form a disulfide bond between the interaction domains.
**Figure 2****.** Diagram illustrating a CAR construct with an interaction domain disulfide-stabilized scFv. A diagram illustrating a CAR construct incorporating a coiled-coil peptide heterodimer with cysteine residues to facilitate disulfide bond formation. Coiled-coil-forming interaction domains are located at the N- and C-termini of the scFv, which consists of a variable light (VL) and a variable heavy (VH) chain.
**Figure 3****.** Molecular model of an scFv stabilized by a coiled-coil peptide heterodimer. A molecular model of a CD19-targeting scFv with coiled-coil-forming interaction domains added at both ends, where a disulfide is formed between the cysteine residues introduced within the coiled-coil segments.

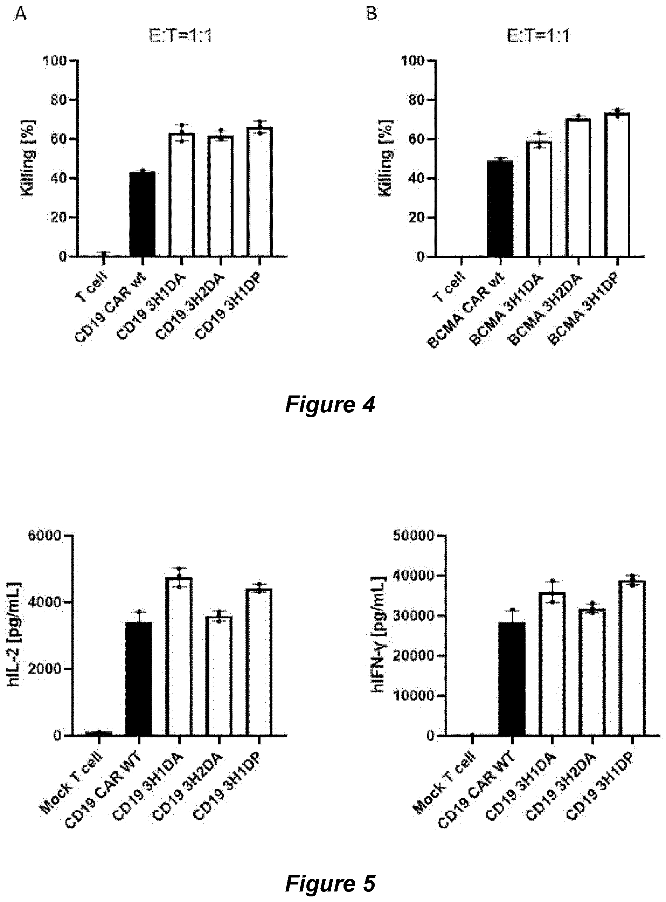

**Figure 4A-B** . Killing efficacy of CAR T cells targeting CD19 and BCMA antigens. Comparison of tumor cell killing by interaction domain disulfide-stabilized CAR T cells after 24 hours at an effector-to-target ratio of 1:1. CAR constructs incorporate coiled-coil peptide heterodimers with cysteine residues, composed of three heptad repeats, as the interacting heterodimerization domains. The 3H1DA interaction domains enable the formation of one disulfide bond, while the 3H2DA interaction domains form two disulfide bonds. The 3H1DP heterodimer forms one disulfide but is further modified to include polar and charged amino acid residues at the solvent-exposed surface. (A) Targeting CD19 on RAJI cells. (B) Targeting BCMA on RAJI cells. In constructs targeting BCMA, all components remain unchanged except for the targeting scFv region, which is specific to the different target antigen.
**Figure 5A-B** . Cytokine secretion by CAR T cells in response to the CD19 target antigen. (A) Human IL-2 levels in supernatants from co-cultures of CD19-targeting CAR T cells and RAJI cells at an effector-to-target ratio of 1:1 after 24 hours. (A) Human IFN-γ levels in supernatants from co-cultures of CD19-targeting CAR T cells and RAJI cells at an effector-to-target ratio of 1:1 after 24 hours.

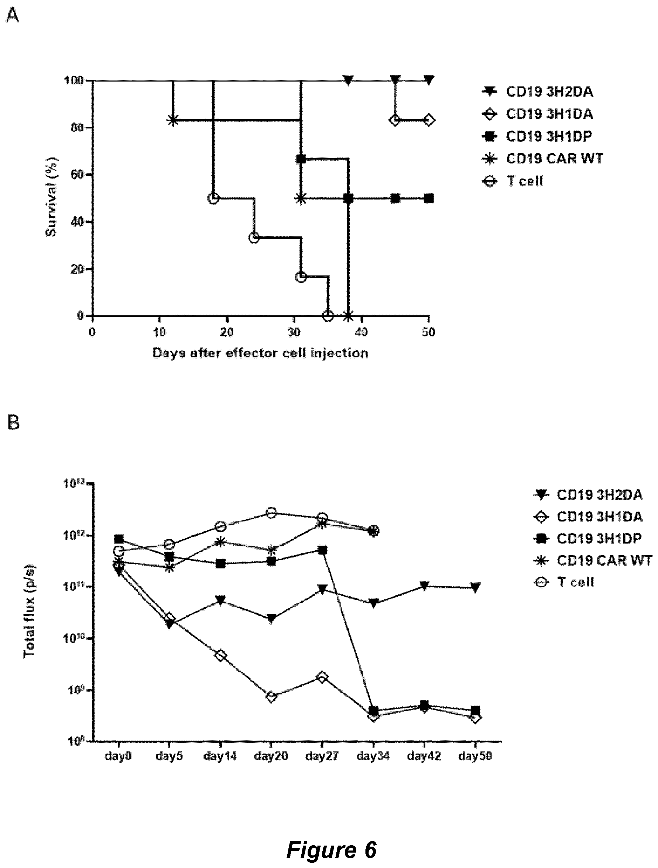

**Figure 6A-B** . In vivo efficacy of disulfide-stabilized CAR T cells in tumor-bearing mice. Results of the in vivo study, where tumor models were established in NOD.CB17-Prkdcscid IL2rgtm1/BcgenHsd mice through intravenous injection of 1 × 10^6 RAJI-FLuc cells per mouse. Following tumor establishment, CAR T cells (5 × 10^6 per mouse) were administered intravenously to assess therapeutic efficacy. (A) Survival curve depicting the percentage of surviving animals over time post-CAR T cell injection. (B) Total flux (p/s) measured over time, indicating tumor burden based on bioluminescent imaging of RAJI-FLuc cells.

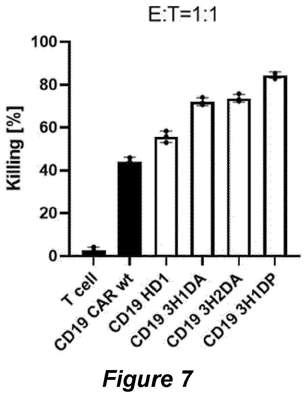

**Figure 7****.** Killing efficacy of CAR T cells targeting CD19 on RAJI cells. Comparison of tumor cell killing by interaction domain disulfide-stabilized CAR T cells after 24 hours at an effector-to-target ratio of 1:1. CAR constructs incorporate a peptide heterodimer HD1 or coiled-coil peptide heterodimers (3H1DA, 3H2DA, 3H1DP) as the interacting heterodimerization domains. The HD1 peptide heterodimer consists of an α-helix and a β-hairpin, connected by two interchain disulfide bonds.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

The present invention addresses the aforementioned challenges by stabilizing the antigen-binding domain within CAR constructs. In some embodiments, the stabilization may be achieved by insertion of interaction domains at the N- and C-termini of the antigen-binding domain. These interaction domains comprise a cysteine residue, and are capable of forming one or several disulfide bonds between each other. This fortifies the structural integrity of the antigen-binding domain, and can prevent mechanical unfolding during target antigen engagement.

The invention represents a significant advancement in CAR-based cell therapy by establishing a universally applicable stabilized CAR format. This enhances the therapeutic efficacy of CAR expressing cells, improving their functionality and adaptability to different antigens. Furthermore, it broadens the therapeutic potential of CAR technology, extending its application beyond typical cancers, to other diseases where highly selective elimination of specific cell types is required.

An exemplary embodiment employs coiled-coil heterodimers, wherein specific interacting residues within the coiled-coil-forming segments are substituted with cysteine residues to facilitate disulfide bond formation. This design significantly enhances the mechanical stability of the antigen-binding domain, thereby increasing the target cell killing efficacy of CAR T cells and enabling effective targeting of cancer cells with low antigen density.

Additionally, other small heterodimeric domains beyond coiled-coil motifs can be utilized to achieve similar stabilization effects, broadening the versatility of the invention. A heterodimeric domain comprising or consisting of 50 or fewer amino acids may be described as a 'small heterodimeric domain'. A heterodimeric domain/small heterodimeric domain may comprise or consist of α-helices. A heterodimeric domain/small heterodimeric domain may comprise or consist of β-sheets. A heterodimeric domain/small heterodimeric domain may comprise or consist of a combination of α-helices and β-sheets. A heterodimeric domain/small heterodimeric domain may contain cysteine residues that enable the formation of one or several disulfide bonds between the domains.

The stabilization strategies described herein are universally applicable across target antigens and antigen-binding domains, as demonstrated with a diverse panel of CAR constructs targeting, for example, CD19 and B-cell maturation antigen (BCMA). Furthermore, this approach extends to tandem CAR configurations, which incorporate two distinct antigen-binding domain, each stabilized by an independent disulfide bond encompassing and stabilizing each antigen-binding domain separately. This allows for the simultaneous recognition of multiple antigens while resisting the mechanical stress exerted by interacting cells.

The present disclosure provides a polypeptide comprising, from N-terminus to C-terminus, (i) a first interaction domain comprising a cysteine, (ii) an antigen-binding domain which binds specifically to a target antigen, and (ii) a second interaction domain comprising a cysteine.

The present disclosure also provides a CAR comprising, from N-terminus to C-terminus, (i) a first interaction domain comprising a cysteine, (ii) an antigen-binding domain which binds specifically to a target antigen, (iii) a second interaction domain comprising a cysteine, (iv) a hinge, (v) a transmembrane domain, and (vi) a signalling domain.

### Chimeric antigen receptors (CARs)

Aspects of the present disclosure relate to chimeric antigen receptors (CARs). CARs are recombinant receptors that provide both antigen-binding and T cell activating functions. CAR structure and engineering is reviewed, for example, in Dotti et al. (Immunol Rev (2014) 257(1)) and Jayaraman et al. (EBioMedicine (2020) 58:102931), both hereby incorporated by reference in their entirety. Many CARs comprise an antigen-binding domain linked via a transmembrane domain to a signalling domain. An optional spacer domain may provide separation between the antigen-binding domain and transmembrane domain and may act as a flexible linker. When expressed by a cell, the antigen-binding domain is provided in the extracellular space, and the signalling domain is intracellular.

The antigen-binding domain mediates binding to the target antigen for which the CAR is specific. The antigen-binding domain of a CAR may be based on the antigen-binding region of an antibody which is specific for the antigen to which the CAR is targeted. For example, the antigen-binding domain of a CAR may comprise amino acid sequences for the complementarity-determining regions (CDRs) of an antibody which binds specifically to the target antigen. The antigen-binding domain of a CAR may comprise or consist of the light chain and heavy chain variable region amino acid sequences of an antibody which binds specifically to the target antigen. The antigen-binding domain may be provided as a single chain variable fragment (scFv) comprising the sequences of the light chain and heavy chain variable region amino acid sequences of an antibody.

Hinge domains, or spacer domains, may be utilised to provide separation between the antigen-binding domain and the transmembrane domain, and may act as a flexible linker. Such domains may be, or may comprise, flexible regions allowing the binding moiety to orient in different directions.

The transmembrane domain is provided between the antigen-binding domain and the signalling domain of the CAR. The transmembrane domain anchors the CAR to the cell membrane of a cell expressing a CAR, with the antigen-binding domain in the extracellular space and signalling domain inside the cell.

Transmembrane domains of CARs may be derived from transmembrane region sequences for cell membrane-bound proteins (e.g. CD8, CD28, CD4, CD3-ζ, *etc.*)*.*

The signalling domain comprises amino acid sequences required for activation of immune cell function. The CAR signalling domains may comprise the amino acid sequence of the intracellular domain of CD3-ζ, which provides immunoreceptor tyrosine-based activation motifs (ITAMs) for phosphorylation and activation of the CAR-expressing cell. Signalling domains comprising sequences of other ITAM-containing proteins have also been employed in CARs, such as domains comprising the ITAM containing region of FcγRI (Haynes et al., 2001 J Immunol 166(1):182-187). CARs comprising a signalling domain derived from the intracellular domain of CD3-ζ are often referred to as first generation CARs.

The signalling domains of CARs often also comprise the signalling domain of a costimulatory protein (*e.g.,* 4-1BB, CD28 *etc.*)*,* for providing a costimulation signal to enhance immune cell activation and effector function. CARs having a signalling domain including additional costimulatory sequences are often referred to as second generation CARs. In some cases, CARs are engineered to provide for costimulation of different intracellular signalling pathways. For example, CD28 costimulation preferentially activates the phosphatidylinositol 3-kinase (P13K) pathway, whereas 4-1BB costimulation triggers signalling is through TNF receptor associated factor (TRAF) adaptor proteins. Signalling domains of CARs therefore sometimes contain costimulatory sequences derived from signalling domains of more than one costimulatory molecule. CARs comprising a signalling domain with multiple costimulatory sequences are often referred to as third generation CARs.

Throughout this specification, polypeptides, domains and amino acid sequences which are 'derived from' a reference polypeptide/domain/amino acid sequence have at least 60%, preferably one of 70%, 75%, 80%, 85%, 90%, 91%, 92%, 93%, 94%, 95%, 96%, 97%, 98%, 99% or 100% amino acid sequence identity to the amino acid sequence of the reference polypeptide/domain/amino acid sequence. Polypeptides, domains and amino acid sequences which are 'derived from' a reference polypeptide/domain/amino acid sequence preferably retain the functional and/or structural properties of the reference polypeptide/domain/amino acid sequence.

By way of illustration, an amino acid sequence derived from the intracellular domain of 4-1BB may comprise an amino acid sequence having 60%, preferably one of 70%, 75%, 80%, 85%, 90%, 91%, 92%, 93%, 94%, 95%, 96%, 97%, 98%, 99% or 100% amino acid sequence identity to the intracellular domain of 4-1BB, e.g. as shown in SEQ ID NO:45. Furthermore, an amino acid sequence derived from the intracellular domain of 4-1BB preferably retains a functional properties of the amino acid sequence of SEQ ID NO:45, *e.g.,* the ability to trigger signalling is through TRAF adaptor proteins.

The amino acid sequence of a given polypeptide or domain thereof can be retrieved from, or determined from a nucleic acid sequence retrieved from, databases known to the person skilled in the art. Such databases include GenBank, EMBL and UniProt.

Through engineering to express a CAR specific for a particular target antigen, immune cells (typically T cells, but also other immune cells such as NK cells) can be directed to kill cells expressing the target antigen. Binding of a CAR-expressing T cell (CAR-T cell) to the target antigen for which it is specific triggers intracellular signalling, and consequently activation of the T cell. The activated CAR-T cell is stimulated to divide and produce factors resulting in killing of the cell expressing the target antigen.

### Interaction domains

The present disclosure provides novel methods for producing stabilized CARs. The CARs according to the present disclosure comprise at least two interaction domains. These interaction domains (e.g., a first interaction domain and a second interaction domain) interact with each other through disulfide bonds. These disulfide bonds stabilize the structure of the CAR, and prevent mechanical unfolding during target antigen engagement.

The CAR may comprise two interaction domains. In some embodiments, an interaction domain is provided on the N-terminal side of the antigen-binding domain. In some embodiments, an interaction domain is provided on the C-terminal side of the antigen-binding domain.

Disulfide bonds may spontaneously form between interaction domains positioned at the N-and C-terminal ends of an antigen-binding domain. Spontaneous formation of disulfide bonds may be achieved, for example, by genetically encoded fusion of interaction domains at both ends of the target protein domain.

In some embodiments, the CAR comprises a first interaction domain, and a second interaction domain. In some embodiments, the first interaction domain is provided on the N-terminal side of the antigen-binding domain. In some embodiments, the second interaction domain is provided on the C-terminal side of the antigen-binding domain. In some embodiments, the first interaction domain is provided on the N-terminal side of the antigen-binding domain, and the second interaction domain is provided on the C-terminal side of the antigen-binding domain. Figure 1 shows a schematic representation of a CAR construct comprising a first interaction domain at the N-terminal side of the antigen-binding domain, and a second interaction domain at the N-terminal side of the antigen-binding domain.

In some embodiments, an interaction domain is provided directly adjacent to an antigen-binding domain. In some embodiments, amino acids are provided between an interaction domain and an antigen-binding domain.

In some embodiments, 0 to 150 amino acids are provided between an interaction domain and an antigen-binding domain, for example, 0 to 1, 0 to 2, 0 to 3, 0 to 4, 0 to 5, 0 to 6, 0 to 7, 0 to 8, 0 to 9, 0 to 10, 0 to 11, 0 to 12, 0 to 13, 0 to 14, 0 to 15, 0 to 16, 0 to 17, 0 to 18, 0 to 19, 0 to 20, 0 to 30, 0 to 40, 0 to 50, 0 to 60, 0 to 70, 0 to 80, 0 to 90, 0 to 100, 0 to 110, 0 to 120, 0 to 130, 0 to 140, or 0 to 150 amino acids are provided between an interaction domain and an antigen-binding domain. In some embodiments, 1 to 150 amino acids are provided between an interaction domain and an antigen-binding domain, for example, 1 to 2, 1 to 3, 1 to 4, 1 to 5, 1 to 6, 1 to 7, 1 to 8, 1 to 9, 1 to 10, 1 to 11, 1 to 12, 1 to 13, 1 to 14, 1 to 15, 1 to 16, 1 to 17, 1 to 18, 1 to 19, 1 to 20, 1 to 30, 1 to 40, 1 to 50, 1 to 60, 1 to 70, 1 to 80, 1 to 90, 1 to 100, 1 to 110, 1 to 120, 1 to 130, 1 to 140, or 1 to 150 amino acids are provided between an interaction domain and an antigen-binding domain.

In some embodiments, at least one amino acid is provided between an interaction domain and an antigen-binding domain, for example, at least 2, 3, 4, 5, 6, 7, 8, 9, 10, 11, 12, 13, 14, 15, 16, 17, 18, 19, 20, 30, 40, 50, 60, 70, 80, 90, 100, 110, 120, 130, 140, or at least 150 amino acids are provided between an interaction domain and an antigen-binding domain. In some embodiments, 1, 2, 3, 4, 5, 6, 7, 8, 9, 10, 11, 12, 13, 14, 15, 16, 17, 18, 19, 20, 30, 40, 50, 60, 70, 80, 90, 100, 110, 120, 130, 140, or 150 amino acids are provided between an interaction domain and an antigen-binding domain.

The interaction domain comprises at least one cysteine residue. In some embodiments, the interaction comprises at least two cysteine residues. In some embodiments, the interaction comprises at least three cysteine residues. In some embodiments, the interaction comprises at least four cysteine residues. In some embodiments, the interaction comprises at least five cysteine residues. In some embodiments, an interaction domain comprises 1, 2, 3, 4, 5, 6, 7, 8, 9, or 10 cysteine residues.

The presence of cysteine residues in the interaction domains enables spontaneous formation of disulfide bonds between interaction domains. In some embodiments, interaction domains interact through one disulfide bond. In some embodiments, interaction domains interact through one disulfide bond. In some embodiments, interaction domains interact through two disulfide bonds. In some embodiments, interaction domains interact through three disulfide bonds. In some embodiments, interaction domains interact through four disulfide bonds. In some embodiments, interaction domains interact through five disulfide bonds. In some embodiments, interaction domains interact through at least one disulfide bond. In some embodiments, interaction domains interact through at least two disulfide bonds. In some embodiments, interaction domains interact through at least three disulfide bonds. In some embodiments, interaction domains interact through at least four disulfide bonds. In some embodiments, interaction domains interact through at least five disulfide bonds.

Disulfide bonds will not form in certain ex vivo conditions, e.g., storage at low pH (e.g., at or below pH 3-4). In some embodiments, the polypeptide comprises a disulfide bond between interaction domains. In some embodiments, the polypeptide comprises two disulfide bonds between interaction domains. In some embodiments, the polypeptide comprises three disulfide bonds between interaction domains. In some embodiments, the polypeptide comprises four disulfide bonds between interaction domains. In some embodiments, the polypeptide comprises five disulfide bonds between interaction domains. The polypeptide may be a CAR. In some embodiments, the CAR comprises a disulfide bond between interaction domains. In some embodiments, the CAR comprises two disulfide bonds between interaction domains. In some embodiments, the CAR comprises three disulfide bonds between interaction domains. In some embodiments, the CAR comprises four disulfide bonds between interaction domains. In some embodiments, the CAR comprises five disulfide bonds between interaction domains.

In some embodiments, the CAR comprises a disulfide bond between interaction domains at neutral pH (e.g. around pH7). In some embodiments, the CAR comprises a disulfide bond between interaction domains at neutral pH (e.g. around pH7) at around 37 °C. In some embodiments, the CAR comprises a disulfide bond between interaction domains in the presence of dissolved oxygen. In some embodiments, the CAR comprises a disulfide bond between interaction domains at neutral pH and in the presence of dissolved oxygen under normal physiological conditions. In some embodiments, the disulfide bond between interaction domains of the CAR forms/was formed spontaneously during biosynthesis within the cell. In some embodiments, the disulfide bond between interaction domains of the CAR forms/was formed spontaneously during biosynthesis during biosynthesis within the endoplasmic reticulum or at the extracellular location of the CAR.

The interaction domain may comprise at least 7 amino acids, for example, the interaction domain may comprise at least 8, 9, 10, 11, 12, 13, 14, 15, 16, 17, 18, 19, 20, 21, 22, 23, 24, 25, 26, 27, 28, 29, or at least 30 amino acids. In some embodiments, the interaction domain comprises 7, 8, 9, 10, 11, 12, 13, 14, 15, 16, 17, 18, 19, 20, 21, 22, 23, 24, 25, 26, 27, 28, 29, or 30 amino acids. In some embodiments, the interaction domain comprises 14 amino acids. In some embodiments, the interaction domain comprises 21 amino acids. In some embodiments, the interaction domain comprises 28 amino acids.

The interaction domain may comprise less than 50 amino acids, for example, the interaction domain may comprise less than 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, or less than 49 amino acids.

The interaction domain may comprise between 7 and 50 amino acids, for example the interaction domain may comprise between 7-10, 7-20, 7-30, 7-40, 7-50, 10-20, 10-30, 10-40, 10-50, 15-20, 15-30, 15-40, 15-50, 20-30, 20-40, 20-50, 25-30, 25-40, 25-50, 30-40, 30-50, 35-40, 35-50, 40-50, or between 45-50 amino acids.

Cysteine residues are positioned to enable disulfide bond formation between interaction domains. For example, the exemplified interaction domain denoted 3H1DA.1 (SEQ ID NO:1) comprises a cysteine at position 5 of its amino acid sequence, and the exemplified interaction domain denoted 3H1DA.2 (SEQ ID NO:2) comprises a cysteine at position 5 of its amino acid sequence, and a disulfide bond can be formed between these cysteines in polypeptides comprising these two interaction domains (*e.g.*, as seen in SEQ ID NO:9 and SEQ ID NO:13). By further way of example, the exemplified interaction domain denoted 3H2DA.1 (SEQ ID NO:3) comprises a cysteine at position 5 and another cysteine at position 19 of its amino acid sequence, and the exemplified interaction domain denoted 3H2DA.2 (SEQ ID NO:4) comprises a cysteine at position 5 and another cysteine at position 19 of its amino acid sequence, and two disulfide bonds can be formed between the cysteines in polypeptides comprising these two interaction domains (*e.g*., as seen in SEQ ID NO:10 and SEQ ID NO:14).

The term disulfide bond used herein relates to a covalent bond formed between the sulfur atoms of two cysteine residues, enhancing the structural stability of proteins.

In some embodiments, the interaction domains form a coiled-coil heterodimer. The term coiled-coil peptide heterodimer used herein relates to a structural motif formed by two complementary alpha-helical peptides that interact to form a stable dimeric structure. Coiled-coil peptide heterodimers may include cysteine residues for disulfide bond formation. In some embodiments, the interaction domain comprises an alpha-helix. In some embodiments, the first interaction domain comprises an alpha-helix, and the second interaction domain comprises an alpha-helix. In some embodiments, the first interaction domain comprises an alpha-helix, and the second interaction domain comprises an alpha-helix, and the first and second interaction domains interact to form a coiled-coil dimer. In some embodiments, the first interaction domain comprises an alpha-helix, and the second interaction domain comprises an alpha-helix, and the first and second interaction domains interact to form a coiled-coil heterodimer.

Coiled-coil peptides are found in proteins throughout all three kingdoms of life. Coiled-coil peptides are known to the skilled person, and are reviewed in the art, for example, in Truebestein and Leonard (Bioessays. 2016 Aug 5;38(9):903-916), which is hereby incorporated by reference in its entirety. The canonical coiled-coil comprises a heptad repeat. Heptad repeats comprise seven amino acids labeled '*a-b-c-d-e-f-g',* in which hydrophobic amino acids at positions a and d are typically conserved (Truebestein and Leonard. Bioessays. 2016 Aug 5;38(9):903-916). In some embodiments, the interaction domain comprises a coiled-coil. In some embodiments, the first interaction domain comprises a coiled-coil, and the second interaction domain comprises a coiled-coil. In some embodiments, the first interaction domain comprises a coiled-coil, and the second interaction domain comprises a coiled-coil, and the first and second interaction domains interact to form a coiled-coil dimer. In some embodiments, the first interaction domain comprises a coiled-coil, and the second interaction domain comprises a coiled-coil, and the first and second interaction domains interact to form a coiled-coil heterodimer.

An interaction domain may comprise a heptad repeat. In some embodiments, an interaction domain comprises two heptad repeats. In some embodiments, an interaction domain comprises three heptad repeats. In some embodiments, an interaction domain comprises four heptad repeats. In some embodiments, an interaction domain comprises five heptad repeats.

Typical heptad repeats which are found in nature comprise a repeating pattern of hydrophobic, charged, and polar amino acids. Positions 'a' and 'd' of heptad repeats are typically hydrophobic residues (e.g., ). Positions 'b', 'c', and 'f' of heptad repeats are typically polar residues (e.g., ). Positions 'e' and 'g' of heptad repeats are typically charged residues (e.g., ). In wild-type polypeptides, the 'd' position(s) of heptad repeats is often occupied by leucine or phenylalanine residue.

Coiled coil peptides comprising heptad repeats can be identified easily from the literature and freely available databases (e.g., Uniprot). In some embodiments, the interaction domain comprises a mutant heptad repeat comprising a substitution at position 'd'. In some embodiments, the interaction domain comprises a mutant heptad repeat comprising a cysteine substitution at position 'd'.

In some embodiments, the interaction domain comprises a heptad repeat, wherein the heptad repeat comprises a cysteine at position 'd'. In some embodiments, the interaction domain comprises a heptad repeat, wherein the heptad repeat comprises a hydrophobic amino acid at position 'a'.

In some embodiments, the interaction domain comprises a heptad repeat, wherein the heptad repeat comprises a polar amino acid at position(s) 'b', 'c', and/or 'f'. In some embodiments, the interaction domain comprises a heptad repeat, wherein the heptad repeat comprises a charged amino acid at position(s) 'e' and/or 'g'. In some embodiments, the interaction domain comprises a heptad repeat, wherein the heptad repeat comprises a cysteine at position 'd', a hydrophobic amino acid at position 'a', a polar amino acid at positions 'b', 'c', and 'f', and a charged amino acid at positions 'e' and 'g'.

In some embodiments, the interaction domain comprising polar and charged amino acid residues at its solvent-exposed surface comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:1.

In some embodiments, the interaction domain comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:2.

In some embodiments, the interaction domain comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:3.

In some embodiments, the interaction domain comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:4.

In some embodiments, the interaction domain comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:5.

In some embodiments, the interaction domain comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:6.

In some embodiments, the interaction domain comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:7.

In some embodiments, the interaction domain comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:8.

In some embodiments, the first interaction domain comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:1, 3, 5, or 7.

In some embodiments, the second interaction domain comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:2, 4, 6, or 8.

In some embodiments, the first interaction domain comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:1, 3, 5, or 7, and the second interaction domain comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:2, 4, 6, or 8.

In some embodiments, the interaction domain comprises polar and charged amino acid residues at its solvent-exposed surface. In some embodiments, the interaction domain comprises amino acid residues at its solvent-exposed surface which increase solubility. In some embodiments, the interaction domain comprises amino acid residues at its solvent-exposed surface which allow for/increase translocation to the plasma membrane. In some embodiments, the interaction domain comprises amino acid residues at its solvent-exposed surface which decrease/prevent degradation, constitutive activity and/or internalisation. The presence of polar and charged amino acid residues at the solvent-exposed surface of the interaction domain may facilitate functionality of the CAR by increasing the solubility and translocation to the plasma membrane, and preventing aggregation that may lead to degradation, constitutive activity and/or internalization of the CAR.

In some embodiments, the interaction domain comprising polar and charged amino acid residues at its solvent-exposed surface comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:7.

In some embodiments, the interaction domain comprising polar and charged amino acid residues at its solvent-exposed surface comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:8.

In some embodiments, the polypeptide/CAR comprises:
(i) an interaction domain which comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:1, and
(ii) an interaction domain which comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:2.

In some embodiments, the polypeptide/CAR comprises:
(i) an interaction domain which comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:3, and
(ii) an interaction domain which comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:4.

In some embodiments, the polypeptide/CAR comprises:
(i) an interaction domain which comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:5, and
(ii) an interaction domain which comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:6.

In some embodiments, the polypeptide/CAR comprises:
(i) an interaction domain which comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:7, and
(ii) an interaction domain which comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:8.

In some embodiments, the polypeptide/CAR according to the present disclosure comprises a first interaction domain other than a coiled-coil peptide, and a second interaction domain other than a coiled-coil peptide. In some embodiments, the polypeptide/CAR according to the present disclosure comprises a first interaction domain derived from a heterodimeric domain (e.g. a small heterodimeric domain), and a second interaction domain derived from the heterodimeric domain. In some embodiments, polypeptide or CAR according to the present disclosure comprises a first interaction domain of HD1 (e.g. HD1.1) and a second interaction domain of HD1 (e.g. HD1.2).

### Antigen-binding domain

An 'antigen-binding domain' refers to a domain which is capable of binding to a target antigen. The target antigen may *e.g.* be a peptide/polypeptide, glycoprotein, lipoprotein, glycan, glycolipid, lipid, or fragment thereof. Antigen-binding domains according to the present disclosure may be derived from antibodies (*i.e.* immunoglobulins (Igs)) and antigen-binding fragments thereof. As used herein, 'antibodies' include monoclonal antibodies, polyclonal antibodies, monospecific and multispecific (*e.g.*, bispecific, trispecific, *etc.*) antibodies, and antibody-derived antigen-binding molecules such as scFv, scFab, diabodies, triabodies, scFv-Fc, minibodies, single domain antibodies (*e.g.* VhH), *etc.*)*.* Antigen-binding fragments of antibodies include e.g. Fv, Fab, F(ab')₂ and F(ab') fragments. In some embodiments, an antigen-binding domain according to the present disclosure may comprise or consist of an antibody or an antigen-binding fragment thereof.

In some embodiments, the antigen-binding domain comprises an antibody heavy chain variable region (VH) and an antibody light chain variable region (VL) of an antibody capable of specific binding to the target antigen. An antigen-binding domain formed by a VH and a VL may also be referred to herein as an Fv.

The antigen-binding domains of the present disclosure may comprise a VH and a VL of an antibody capable of specific binding to the target antigen. Antibodies generally comprise six complementarity-determining regions CDRs; three in the heavy chain variable region (VH): HC-CDR1, HC-CDR2 and HC-CDR3, and three in the light chain variable region (VL): LC-CDR1, LC-CDR2, and LC-CDR3. The six CDRs together define the paratope of the antibody, which is the part of the antibody which binds to the target antigen. The VH region and VL region comprise framework regions (FRs) either side of each CDR, which provide a scaffold for the CDRs. From N-terminus to C-terminus, VHs comprise the following structure: N term-[HC-FR1]-[HC-CDR1]-[HC-FR2]-[HC-CDR2]-[HC-FR3]-[HC-CDR3]-[HC-FR4]-C term; and VLs comprise the following structure: N term-[LC-FR1]-[LC-CDR1]-[LC-FR2]-[LC-CDR2]-[LC-FR3]-[LC-CDR3]-[LC-FR4]-C term.

VH and VL sequences may be provided in any suitable format provided that the antigen-binding domain can be linked to the other domains of the CAR, e.g., in a format described in Carter, Nat. Rev. Immunol (2006), 6: 343-357, such as scFv, dsFV, (scFv)₂ diabody, triabody, tetrabody, Fab, minibody, and F(ab)₂ formats.

In some embodiments, the antigen-binding domain comprises the CDRs of an antibody/antibody fragment which is capable of binding to the target antigen. In some embodiments, the antigen-binding domain comprises the VH region and the VL region of an antibody/antibody fragment which is capable of binding to the target antigen. A moiety comprised of the VH and a VL of an antibody may also be referred to herein as a variable fragment (Fv). The VH and VL may be provided on the same polypeptide chain, and joined via a linker sequence; such moieties are referred to as single-chain variable fragments (scFvs). Suitable linker sequences for the preparation of scFv are known to the skilled person, and may comprise serine and glycine residues.

In some embodiments, the antigen-binding domain comprises the CDRs of a known CAR T-cell therapy (*e.g.,* an approved CAR T-cell therapy). Numerous CAR T-cell therapies are known to the skilled person, and multiple CAR T-cell therapies are approved for use in treatments by government agencies, such as the U.S. Food and Drug Administration (FDA) and the European Medicines Agency (EMA). For example, Kymriah (tisagenlecleucel), YESCARTA (axicabtagene ciloleucel), TECARTUS (brexucabtagene autoleucel), BREYANZI (lisocabtagene maraleucel), ABECMA (idecabtagene vicleucel), CARVYKTI (ciltacabtagene autoleucel), and AUCATZYL (obecabtagene autoleucel, obe-cel, AUTO1) are CAR T-cell therapies approved for the treatment of cancer by the FDA. Kymriah (tisagenlecleucel), YESCARTA (axicabtagene ciloleucel), TECARTUS (brexucabtagene autoleucel), BREYANZI (lisocabtagene maraleucel), and AUCATZYL (obecabtagene autoleucel, obe-cel, AUTO1) all target CD19. ABECMA (idecabtagene vicleucel) and CARVYKTI (ciltacabtagene autoleucel) target BCMA.

Antigen-binding domains according to the present disclosure include antibody-derived antigen-binding molecules, *e.g.* molecules comprising an antigen-binding region/domain derived from an antibody. Antibody-derived antigen-binding molecules may comprise an antigen-binding region/domain that comprises, or consists of, the antigen-binding region of an antibody (*e.g*. an antigen-binding fragment of an antibody). In some embodiments, the antigen-binding region/domain of an antibody-derived antigen-binding molecule may be or comprise the Fv (*e.g.* provided as an scFv) or the Fab region of an antibody. In some embodiments, the antigen-binding domain comprises, or consists of, Fv capable of binding to the target antigen. The antigen-binding domain may be provided with any suitable format, *e.g*. scFv, scFab, *etc.* In some embodiments, the antigen-binding domain comprises, or consists of, a scFv capable of binding to the target antigen.

In some embodiments, the antigen-binding domain comprises an antibody heavy chain variable region (VH). In some embodiments, the moiety capable of binding to a target antigen comprises a Variable domain of the Heavy chain of a Heavy-chain antibody (VHH). VHH antibodies are formed of a single antibody variable domain which comprises three CDRs. VHH antibodies further comprise framework regions (FRs) either side of each CDR, which provide a scaffold for the CDRs. The designation 'VHH' is an acronym for 'Variable Heavy domain of Heavy chain'. VHH antibodies are also known as 'VHHs', 'single domain antibodies (sdAbs)', 'nanobodies', 'single variable domain on a heavy chain antibodies', and 'heavy chain only antibodies (HcAbs)', and are described *e.g.* in Henry and MacKenzie, Front Immunol. (2018) 9:41, Bever et al., Anal Bioanal Chem. (2016) 408(22): 5985-6002, and Tang et al. Int J Mol Sci. 2023 Feb; 24(4): 4176, all of which are hereby incorporated by reference in their entirety.

The target antigen for which the antigen-binding domain (and thus the CAR) is specific may be any target antigen. In some embodiments, the target antigen is an antigen whose expression/activity, or whose upregulated expression/activity, is positively associated with a disease or disorder (*e.g.* a cancer, an infectious disease or an autoimmune disease). The target antigen is preferably expressed at the cell surface of a cell expressing the target antigen. It will be appreciated that the CAR directs effector activity of the cell expressing the CAR against cells/tissues expressing the target antigen for which the CAR comprises a specific antigen-binding domain.

In some embodiments, a target antigen may be a cancer cell antigen. A cancer cell antigen is an antigen which is expressed or over-expressed by a cancer cell. A cancer cell antigen may be any peptide/polypeptide, glycoprotein, lipoprotein, glycan, glycolipid, lipid, or fragment thereof. A cancer cell antigen's expression may be associated with a cancer. A cancer cell antigen may be abnormally expressed by a cancer cell (*e.g.* the cancer cell antigen may be expressed with abnormal localisation), or may be expressed with an abnormal structure by a cancer cell. A cancer cell antigen may be capable of eliciting an immune response. In some embodiments, the antigen is expressed at the cell surface of the cancer cell (*i.e.* the cancer cell antigen is a cancer cell surface antigen). In some embodiments, the part of the antigen which is bound by the antigen-binding molecule described herein is displayed on the external surface of the cancer cell (*i.e*. is extracellular). The cancer cell antigen may be a cancer-associated antigen. In some embodiments the cancer cell antigen is an antigen whose expression is associated with the development, progression or severity of symptoms of a cancer. The cancer-associated antigen may be associated with the cause or pathology of the cancer, or may be expressed abnormally as a consequence of the cancer. In some embodiments, the cancer cell antigen is an antigen whose expression is upregulated (*e.g*. at the RNA and/or protein level) by cells of a cancer, *e.g.* as compared to the level of expression of by comparable non-cancerous cells (*e.g*. non-cancerous cells derived from the same tissue/cell type). In some embodiments, the cancer-associated antigen may be preferentially expressed by cancerous cells, and not expressed by comparable non-cancerous cells (*e.g*. non-cancerous cells derived from the same tissue/cell type). In some embodiments, the cancer-associated antigen may be the product of a mutated oncogene or mutated tumor suppressor gene. In some embodiments, the cancer-associated antigen may be the product of an overexpressed cellular protein, a cancer antigen produced by an oncogenic virus, an oncofetal antigen, or a cell surface glycolipid or glycoprotein.

Cancer cell antigens are reviewed by Zarour HM, DeLeo A, Finn OJ, et al. Categories of Tumor Antigens. In: Kufe DW, Pollock RE, Weichselbaum RR, et al., editors. Holland-Frei Cancer Medicine. 6th edition. Hamilton (ON): BC Decker; 2003. Cancer cell antigens include oncofetal antigens: CEA, Immature laminin receptor, TAG-72; oncoviral antigens such as HPV E6 and E7; overexpressed proteins: BING-4, calcium-activated chloride channel 2, cyclin-B1, 9D7, Ep-CAM, EphA3, HER2/neu, telomerase, mesothelin, SAP-1, survivin; cancer-testis antigens: BAGE, CAGE, GAGE, MAGE, SAGE, XAGE, CT9, CT10, NY-ESO-1, PRAME, SSX-2; lineage restricted antigens: MART1, Gp100, tyrosinase, TRP-1/2, MC1R, prostate specific antigen; mutated antigens: β-catenin, BRCA1/2, CDK4, CML66, Fibronectin, MART-2, p53, Ras, TGF-βRII; post-translationally altered antigens: MUC1, idiotypic antigens: Ig, TCR. Other cancer cell antigens include heat-shock protein 70 (HSP70), heat-shock protein 90 (HSP90), glucose-regulated protein 78 (GRP78), vimentin, nucleolin, feto-acinar pancreatic protein (FAPP), alkaline phosphatase placental-like 2 (ALPPL-2), siglec-5, stress-induced phosphoprotein 1 (STIP1), protein tyrosine kinase 7 (PTK7), and cyclophilin B.

In some embodiments the cancer cell antigen is a cancer cell antigen described in Zhao and Cao, Front Immunol. (2019) 10: 2250, which is hereby incorporated by reference in its entirety. In some embodiments, a cancer cell antigen is selected from CD19, BCMA, CD30, CD20, CD22, ROR1R, CD4, CD7, CD38, Mesothelin, EGFR, GPC3, MUC1, HER2, GD2, CEA, EpCAM, LeY and PSCA.

In some embodiments, a cancer cell antigen is an antigen expressed by cells of a hematological malignancy. In some embodiments, a cancer cell antigen is selected from CD19, BCMA, CD30, CD20, CD22, ROR1R, CD4, CD7, and CD38. In some embodiments, a cancer cell antigen is an antigen expressed by cells of a solid tumor. In some embodiments, a cancer cell antigen is selected from Mesothelin, EGFR, GPC3, MUC1, HER2, GD2, CEA, EpCAM, LeY and PSCA.

The antigen-binding domains of the present disclosure may be designed and prepared using the sequences of antibodies capable of binding to a target antigen of interest. The skilled person is readily able to produce/obtain further antibodies that bind to a given target antigen (*e.g.* CD19, BCMA, CD30, CD20, CD22, ROR1R, CD4, CD7, CD38, Mesothelin, EGFR, GPC3, MUC1, HER2, GD2, CEA, EpCAM, LeY and/or PSCA), in view of techniques that are well-known in the art. For example, Park and Smolen, Advances in Protein Chemistry (2001) 56: 369-421 describes approaches to the production of monoclonal antibodies suitable for use in humans, including raising xenogeneic antibodies by immunisation of non-human animals and their subsequent chimerisation and/or humanisation, identification by human antibody gene-phage display, and production of antibodies in transgenic mice harbouring human antibody genes.

Mouse/human chimeric antibodies can be prepared from mouse antibodies by the process of chimerisation, *e.g.* as described in Human Monoclonal Antibodies: Methods and Protocols, Michael Steinitz (Editor), Methods in Molecular Biology 1060, Springer Protocols, Humana Press (2014), in Chapter 8 thereof, in particular section 3 of Chapter 8. Humanised antibodies can be prepared from mouse antibodies by the process of humanisation, *e.g.* as described in Human Monoclonal Antibodies: Methods and Protocols, Michael Steinitz (Editor), Methods in Molecular Biology 1060, Springer Protocols, Humana Press (2014), in Chapter 7 thereof, in particular section 3.1 of Chapter 7 entitled 'Antibody Humanization'. Techniques for antibody humanisation are also described *e.g.* in Safdari et al., Biotechnol Genet Eng Rev (2013) 29:175-86. In the human antibody gene-phage display technique, genes encoding the VH and VL chains are generated by PCR amplification and cloning from 'naive' human lymphocytes, and assembled into a library from which they can be expressed either as disulfide-linked Fab fragments or as single-chain Fv (scFv) fragments. The Fab- or scFv-encoding genes are fused to a surface coat protein of filamentous bacteriophage and Fab or scFv capable of binding to the target antigen can then be identified by screening the library with the target antigen/a fragment thereof. Molecular evolution or affinity maturation procedures can be employed to enhance the affinity of the Fab/scFv fragment. In the transgenic mouse technique, mice in which the endogenous murine Ig gene loci have been replaced by homologous recombination with their human homologues are immunised with the relevant target antigen/a fragment thereof, and a monoclonal antibody can then by produced by conventional hybridoma technology, to yield a fully-human monoclonal antibody. Techniques for generating antibodies suitable for use in therapy are also reviewed in Lu et al., J Biomed Sci. (2020) 27:1, which is hereby incorporated by reference in its entirety.

In some embodiments, the antigen-binding domain (and thus the CAR) is multispecific. By 'multispecific' it is meant that the antigen-binding domain displays specific binding to more than one target. In some embodiments the antigen-binding domain is a bispecific antigen-binding domain. In some embodiments the antigen-binding molecule comprises at least two different antigen-binding moieties (*i.e*. at least two antigen-binding moieties, *e.g.* comprising non-identical VHs and VLs). Individual antigen-binding moieties of multispecific antigen-binding domains may be connected, *e.g.* via linker sequences.

In some embodiments the antigen-binding domain binds to at least two, non-identical target antigens, and so is at least bispecific. The term 'bispecific' means that the antigen-binding domain is able to bind specifically to at least two distinct antigenic determinants. In some embodiments, at least one of the target antigens for the multispecific antigen-binding domain/CAR is CD19 and/or BCMA.

Each of the target antigens may independently be a target antigen as described herein. In some embodiments each target antigen is independently a cancer cell antigen as described herein.

In some embodiments, the antigen-binding domain may comprise or consist of a single chain variable fragment (scFv) comprising a VH sequence and a VL sequence as described herein. The VH sequence and VL sequence may be covalently linked. In some embodiments, the VH and the VL sequences are linked by a flexible linker sequence, *e.g.* a flexible linker sequence as described herein. The flexible linker sequence may be joined to ends of the VH sequence and VL sequence, thereby linking the VH and VL sequences.

In some embodiments, the VH and VL are joined via a linker sequence comprising one or more copies of an amino acid sequence according to SEQ ID NO:34, 35, 36, or 37. In some embodiments, the linker sequence comprises at least 1, 2, 3 or 4 copies of an amino acid sequence according to SEQ ID NO: 34, 35, 36, or 37. In some embodiments, the VH and VL are joined via a linker sequence comprising one or more copies of an amino acid sequence according to SEQ ID NO:35. In some embodiments, the linker sequence comprises at least 1, 2, 3 or 4 copies of an amino acid sequence according to SEQ ID NO:35.

In some embodiments, the VH and VL are joined via a linker sequence comprising, or consisting of, an amino acid sequence having at least 80%, 85%, 86%, 87%, 88%, 89%, 90%, 91%, 92%, 93%, 94%, 95%, 96%, 97%, 98%, 99%, or 100% sequence identity to the amino acid sequence of SEQ ID NO:38, 39, or 40.

It will be appreciated that an antigen-binding domain according to the present disclosure (*e.g.* a multispecific antigen-binding domain) comprises antigen-binding moieties capable of binding to the target(s) for which the antigen-binding domain is specific. For example, an antigen-binding domain which is capable of binding to CD19 and an antigen other than CD19 may comprise: (i) an antigen-binding moiety which is capable of binding to CD19, and (ii) an antigen-binding moiety which is capable of binding to a target antigen other than CD19 (*e.g*., BCMA).

In aspects and embodiments of the present disclosure, the target antigen is CD19. Accordingly, in some aspects and embodiments of the present disclosure the antigen-binding domain is a CD19-binding domain.

CD19 (also known as B-lymphocyte surface antigen B4) is the protein identified by UniProt: P15391. CD19 is a marker of B cells, and is a useful target for the treatment of e.g. B cell lymphomas, acute lymphoblastic leukemia (ALL), and chronic lymphocytic leukemia (CLL) - see e.g. Wang et al., Exp Hematol Oncol. (2012) 1:36.

In this specification "CD19" refers to CD19 from any species and includes CD19 isoforms (*e.g*. P15391-2), fragments, variants (including mutants) or homologues from any species. As used herein, a 'fragment', 'variant' or 'homologue' of a reference protein may optionally be characterised as having at least 60%, preferably one of 70%, 75%, 80%, 85%, 90%, 91%, 92%, 93%, 94%, 95%, 96%, 97%, 98%, 99% or 100% amino acid sequence identity to the amino acid sequence of the reference protein (*e.g.* a reference isoform). In some embodiments fragments, variants, isoforms and homologues of a reference protein may be characterised by ability to perform a function performed by the reference protein.

CD19 is a cell-surface molecule that is found in most B cell malignancies. CD19 function is described *e.g.* in de Rie et al. (Cell Immunol. 1989 Feb;118(2):368-81), Carter and Fearon (Science. 1992 Apr 3;256(5053):105-7), and Vanshylla et al. (Sci Rep. 2018 Mar 9;8(1):4244), all of which are hereby incorporated by reference in their entirety.

In some embodiments, the CD19 is from a mammal (*e.g*. a primate (rhesus, cynomolgous, or human) and/or a rodent (*e.g.* rat or murine) CD19). In preferred embodiments the CD19 is a human CD19. Isoforms, fragments, variants or homologues may optionally be characterised as having at least 70%, preferably one of 80%, 85%, 90%, 91%, 92%, 93%, 94%, 95%, 96%, 97%, 98%, 99% or 100% amino acid sequence identity to the amino acid sequence of an immature or mature CD19 isoform from a given species, e.g. human. A fragment of CD19 may have a minimum length of one of 10, 20, 30, 40, 50, 100, 200, 300, 400, 500 or 590 amino acids, and may have a maximum length of one of 10, 20, 30, 40, 50, 100, 200, 300, 400, 500 or 595 amino acids.

Human CD19 may be identified by UniProt: P15391. Alternative splicing of mRNA encoded by the human CD19 gene yields two main isoforms: isoform 1 (SEQ ID NO:23), isoform 2 (SEQ ID NO:24).

Human CD19 isoform 1 comprises an N-terminal signal peptide (SEQ ID NO:25), followed by an extracellular domain (SEQ ID NO:26), transmembrane domain (SEQ ID NO:27), and a cytoplasmic domain (SEQ ID NO:28).

In some embodiments, the CD19 comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:23, 24, 25, 26, 27 or 28.

In some embodiments, the CD19 comprises an amino acid sequence having at least 70%, preferably one of 80%, 85%, 90%, 91%, 92%, 93%, 94%, 95%, 96%, 97%, 98%, 99% or 100% amino acid sequence identity to SEQ ID NO: 23 or 24. In some embodiments, a fragment of CD19 comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO: 25, 26, 27 or 28.

The CD19-binding domain of the CAR of the present disclosure preferably displays specific binding to CD19 or a fragment thereof. The CD19-binding domain of the CAR of the present disclosure preferably displays specific binding to the extracellular domain of CD19. The CD19-binding domain may be derived from an anti-CD19 antibody or other CD19-binding agent, *e.g.* a CD19-binding peptide or CD19-binding small molecule.

The CD19-binding domain may be derived from the antigen-binding moiety of an anti-CD19 antibody. Anti-CD19 antibodies are reviewed in the art, for example, in Zinzani and Minotti (J Cancer Res Clin Oncol. 2022 Jan;148(1):177-190).

In some embodiments a CD19-binding domain according to the present disclosure comprises the CDRs of an anti-CD19 antibody. In some embodiments a CD19-binding domain according to the present disclosure comprises the VH and VL regions of an anti-CD19 antibody. In some embodiments a CD19-binding domain according to the present disclosure comprises a scFv comprising the VH and VL regions of an anti-CD19 antibody.

Anti-CD19 antibodies include Blinatumomab (DrugBank Accession Number: DB09052), Coltuximab ravtansine (SAR3419; DrugBank Accession Number: DB06342), Inebilizumab (DrugBank Accession Number: DB12530), and Tafasitamab (DrugBank Accession Number: DB15044). The skilled person is readily able to identify further anti-CD19 antibodies in the art.

In some embodiments a CD19-binding domain according to the present disclosure comprises the CDRs of an anti-CD19 CAR. In some embodiments a CD19-binding domain according to the present disclosure comprises the VH and VL regions of an anti-CD19 CAR. In some embodiments a CD19-binding domain according to the present disclosure comprises a scFv comprising the VH and VL regions of an anti-CD19 CAR.

Anti-CD19 CARs include Kymriah (tisagenlecleucel), YESCARTA (axicabtagene ciloleucel), TECARTUS (brexucabtagene autoleucel), BREYANZI (lisocabtagene maraleucel), and AUCATZYL (obecabtagene autoleucel, obe-cel, AUTO1).

In some embodiments a CD19-binding domain according to the present disclosure comprises the CDRs of an anti-CD19 antibody. In some embodiments a CD19-binding domain according to the present disclosure comprises the VH and VL regions of an anti-CD19 antibody. In some embodiments a CD19-binding domain according to the present disclosure comprises a scFv comprising the VH and VL regions of an anti-CD19 antibody.

There are several different conventions for defining antibody CDRs and FRs, such as those described in Kabat et al., Sequences of Proteins of Immunological Interest, 5th Ed. Public Health Service, National Institutes of Health, Bethesda, MD (1991), Chothia et al., J. Mol. Biol. 196:901-917 (1987), and VBASE2, as described in Retter et al., Nucl. Acids Res. (2005) 33 (suppl 1): D671-D674. The CDRs and FRs of the VH regions and VL regions of the antibody clones described herein are defined according to the international IMGT (ImMunoGeneTics) information system (LeFranc et al., Nucleic Acids Res. (2015) 43 (Database issue):D413-22), which uses the IMGT V-DOMAIN numbering rules as described in Lefranc et al., Dev. Comp. Immunol. (2003) 27:55-77.

In some embodiments the antigen-binding domain of the present disclosure comprises:
a VH incorporating the following CDRs:
HC-CDR1 having the amino acid sequence of SEQ ID NO:59
HC-CDR2 having the amino acid sequence of SEQ ID NO:60
HC-CDR3 having the amino acid sequence of SEQ ID NO:61,
or a variant thereof in which one or two or three amino acids in one or more of HC-CDR1, HC-
CDR2, or HC-CDR3 are substituted with another amino acid;
   and
a VL incorporating the following CDRs:
   LC-CDR1 having the amino acid sequence of SEQ ID NO:63
   LC-CDR2 having the amino acid sequence of SEQ ID NO:64
   LC-CDR3 having the amino acid sequence of SEQ ID NO:65,
   or a variant thereof in which one or two or three amino acids in one or more of LC-CDR1, LC-CDR2, or LC-CDR3 are substituted with another amino acid.

In some embodiments the antigen-binding domain comprises:
a VH comprising, or consisting of, an amino acid sequence having at least 80% sequence identity (*e.g.* at least 85%, 86%, 87%, 88%, 89%, 90%, 91%, 92%, 93%, 94%, 95%, 96%, 97%, 98%, 99% or 100%) to the amino acid sequence of SEQ ID NO:58;
   and
a VL comprising, or consisting of, an amino acid sequence having at least 80% sequence identity (*e.g.* at least 85%, 86%, 87%, 88%, 89%, 90%, 91%, 92%, 93%, 94%, 95%, 96%, 97%, 98%, 99% or 100%) to the amino acid sequence of SEQ ID NO:62.

In some embodiments, a CD19-binding domain may comprise or consist of a single chain variable fragment (scFv) comprising a VH sequence and a VL sequence as described herein. The VH sequence and VL sequence may be covalently linked. In some embodiments, the VH and the VL sequences are linked by a flexible linker sequence, *e.g.* a flexible linker sequence as described herein. The flexible linker sequence may be joined to ends of the VH sequence and VL sequence, thereby linking the VH and VL sequences.

In some embodiments, the CD19-binding domain comprises, or consists of, an amino acid sequence having at least 80%, 85% 86%, 87%, 88%, 89%, 90%, 91%, 92%, 93%, 94%, 95%, 96%, 97%, 98%, 99%, or 100% sequence identity to the amino acid sequence of SEQ ID NO:66.

In aspects and embodiments of the present disclosure, the target antigen is BCMA. Accordingly, in some aspects and embodiments of the present disclosure the antigen-binding domain is a BCMA-binding domain.

BCMA (also known as Tumor necrosis factor receptor superfamily member 17) is the protein identified by UniProt: Q02223.

BCMA is a nonglycosylated integral membrane type I protein that is preferentially expressed in mature B lymphocytes, and it promotes B-cell survival and plays a role in the regulation of humoral immunity. BCMA structure and function is described e.g. in Hatzoglou et al (J Immunol. 2000 Aug 1;165(3):1322-30) and Gross et al. (Nature. 2000 Apr 27;404(6781):995-9), both of which are hereby incorporated by reference in their entirety.

In this specification 'BCMA' refers to BCMA from any species and includes BCMA isoforms, fragments, variants or homologues from any species. As used herein, a 'fragment', 'variant' or 'homologue' of a reference protein may optionally be characterised as having at least 60%, preferably one of 70%, 75%, 80%, 85%, 90%, 91%, 92%, 93%, 94%, 95%, 96%, 97%, 98%, 99% or 100% amino acid sequence identity to the amino acid sequence of the reference protein (*e.g*. a reference isoform). In some embodiments fragments, variants, isoforms and homologues of a reference protein may be characterised by ability to perform a function performed by the reference protein.

In some embodiments, the BCMA is from a mammal (*e.g*. a primate (rhesus, cynomolgous, or human) and/or a rodent (*e.g*. rat or murine) BCMA). In preferred embodiments the BCMA is a human BCMA. Isoforms, fragments, variants or homologues may optionally be characterised as having at least 70%, preferably one of 80%, 85%, 90%, 91%, 92%, 93%, 94%, 95%, 96%, 97%, 98%, 99% or 100% amino acid sequence identity to the amino acid sequence of an immature or mature BCMA isoform from a given species, *e.g.* human. A fragment of BCMA may have a minimum length of one of 10, 20, 30, 40, 50, 100, 200, 300, 400, 500 or 590 amino acids, and may have a maximum length of one of 10, 20, 30, 40, 50, 100, 200, 300, 400, 500 or 595 amino acids.

Human BCMA may be identified by UniProt: Q02223. Alternative splicing of mRNA encoded by the human *TNFRSF17* gene yields two main isoforms: isoform 1 (SEQ ID NO:29), isoform 2 (SEQ ID NO:30).

Human BCMA isoform 1 comprises an extracellular domain (SEQ ID NO:31), transmembrane domain (SEQ ID NO:32), and a cytoplasmic domain (SEQ ID NO:33).

In some embodiments, the BCMA comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:29, 30, 31, 32 or 33.

In some embodiments, the BCMA comprises an amino acid sequence having at least 70%, preferably one of 80%, 85%, 90%, 91%, 92%, 93%, 94%, 95%, 96%, 97%, 98%, 99% or 100% amino acid sequence identity to SEQ ID NO:29 or 30. In some embodiments, a fragment of BCMA comprises, or consists of, an amino acid sequence having at least 70%, preferably one of 80%, 85%, 90%, 91%, 92%, 93%, 94%, 95%, 96%, 97%, 98%, 99% or 100% amino acid sequence identity to SEQ ID NO: 31, 32 or 33.

The BCMA-binding domain of the CAR of the present disclosure preferably displays specific binding to BCMA or a fragment thereof. The BCMA-binding domain of the CAR of the present disclosure preferably displays specific binding to the extracellular domain of BCMA. The BCMA-binding domain may be derived from an anti-BCMA antibody or other BCMA-binding agent, *e.g.* a BCMA-binding peptide or BCMA-binding small molecule.

In some embodiments a BCMA-binding domain according to the present disclosure comprises the CDRs of an anti-BCMA antibody. In some embodiments a BCMA-binding domain according to the present disclosure comprises the VH and VL regions of an anti-BCMA antibody. In some embodiments a BCMA-binding domain according to the present disclosure comprises a scFv comprising the VH and VL regions of an anti-BCMA antibody.

Anti-BCMA antibodies include Teclistamab (DrugBank Accession Number: DB16655) and Elranatamab (DrugBank Accession Number: DB15395). The skilled person is readily able to identify further anti-BCMA antibodies in the art.

In some embodiments a BCMA-binding domain according to the present disclosure comprises the CDRs of an anti-BCMA CAR. In some embodiments a BCMA-binding domain according to the present disclosure comprises the VH and VL regions of an anti-BCMA CAR. In some embodiments a BCMA-binding domain according to the present disclosure comprises a scFv comprising the VH and VL regions of an anti-BCMA CAR. Anti-BCMA CARs include ABECMA (idecabtagene vicleucel) and CARVYKTI (ciltacabtagene autoleucel).

In some embodiments the antigen-binding domain of the present disclosure comprises:
a VH incorporating the following CDRs:
HC-CDR1 having the amino acid sequence of SEQ ID NO:68
HC-CDR2 having the amino acid sequence of SEQ ID NO:69
HC-CDR3 having the amino acid sequence of SEQ ID NO:70,
or a variant thereof in which one or two or three amino acids in one or more of HC-CDR1, HC-
CDR2, or HC-CDR3 are substituted with another amino acid;
   and
a VL incorporating the following CDRs:
   LC-CDR1 having the amino acid sequence of SEQ ID NO:72
   LC-CDR2 having the amino acid sequence of SEQ ID NO:73
   LC-CDR3 having the amino acid sequence of SEQ ID NO:74,
   or a variant thereof in which one or two or three amino acids in one or more of LC-CDR1, LC-CDR2, or LC-CDR3 are substituted with another amino acid.

In some embodiments the antigen-binding domain comprises:
a VH comprising, or consisting of, an amino acid sequence having at least 80% sequence identity (*e.g.* at least 85%, 86%, 87%, 88%, 89%, 90%, 91%, 92%, 93%, 94%, 95%, 96%, 97%, 98%, 99% or 100%) to the amino acid sequence of SEQ ID NO:67;
   and
a VL comprising, or consisting of, an amino acid sequence having at least 80% sequence identity (*e.g.* at least 85%, 86%, 87%, 88%, 89%, 90%, 91%, 92%, 93%, 94%, 95%, 96%, 97%, 98%, 99% or 100%) to the amino acid sequence of SEQ ID NO:71.

In some embodiments, a BCMA-binding domain may comprise or consist of a single chain variable fragment (scFv) comprising a VH sequence and a VL sequence as described herein. The VH sequence and VL sequence may be covalently linked. In some embodiments, the VH and the VL sequences are linked by a flexible linker sequence, *e.g.* a flexible linker sequence as described herein. The flexible linker sequence may be joined to ends of the VH sequence and VL sequence, thereby linking the VH and VL sequences.

In some embodiments, the BCMA-binding domain comprises, or consists of, an amino acid sequence having at least 80%, 85% 86%, 87%, 88%, 89%, 90%, 91%, 92%, 93%, 94%, 95%, 96%, 97%, 98%, 99%, or 100% sequence identity to the amino acid sequence of SEQ ID NO:75.

The target antigen may be an inflammation-associated antigen. An 'inflammation-associated antigen' refers to an antigen whose presence is indicative of inflammation, or an antigen for which an elevated level of the antigen is positively-correlated with inflammation. The inflammation-associated antigen may be an antigen whose gene and/or protein expression is associated with the development, progression or severity of symptoms of inflammation. The inflammation-associated antigen may be associated with the cause or pathology of the inflammation, or may be expressed abnormally as a consequence of inflammation.

In some embodiments, an inflammation-associated antigen is selected from: an inflammatory cytokine or chemokine, an immune cell surface protein, an acute phase protein, an inflammation-associated enzyme, a pattern recognition receptor (PRR), a complement protein, and an autoimmune-disease-associated antigen.

In some embodiments, an inflammation-associated antigen is an inflammatory cytokine or chemokine. In some embodiments, an inflammatory cytokine or chemokine is selected from: TNFα, IL-1β, IL-6, IL-17, IL-23, IL-8 (CXCL8), IFNγ, MCP-1 (CCL2), RANTES (CCL5), MIP-1α (CCL3), and MIP-1β (CCL4).

In some embodiments, an inflammation-associated antigen is an immune cell surface protein. In some embodiments, an inflammation-associated antigen is an inflammatory immune cell surface protein. In some embodiments, an inflammation-associated antigen is a T cell surface protein, e.g. CD3, CD4, CD8, CD28, CD40L, CTLA-4, ICOS, PD-1, CCR5. In some embodiments, an inflammation-associated antigen is a B cell surface protein, e.g. CD19, CD20, BAFF. In some embodiments, an inflammation-associated antigen is a monocyte and/or macrophage cell surface protein, e.g. CD14, CCR2 or CCR5. In some embodiments, an inflammation-associated antigen is an NK cell surface protein, e.g. CD16 or CD56. In some embodiments, an inflammation-associated antigen is a leukocyte adhesion protein, e.g. CD11b. In some embodiments, an inflammation-associated antigen is an antigen-presenting cell (APC) surface protein, e.g. CD40, CD80, CD86, MHC class I, MHC class I, ICOSL, PD-L1 or PD-L2. In some embodiments, an inflammation-associated antigen is an adhesion molecule, e.g. ICAM-1 (CD54), VLA-4 (integrin α4β1), LPAM-1 (integrin α4β7) or VCAM-1 (CD106).

In some embodiments, an inflammation-associated antigen is an acute phase protein. In some embodiments, an inflammation-associated antigen is C-reactive protein (CRP), serum amyloid A (SAA), fibrinogen or ferritin.

In some embodiments, an inflammation-associated antigen is an inflammation-associated enzyme. In some embodiments, an inflammation-associated antigen is COX-2, iNOS, a matrix metalloproteinase (MMP; e.g. MMP-1, MMP-3, and MMP-9), or 5-LOX.

In some embodiments, an inflammation-associated antigen is a pattern recognition receptor (PRR). In some embodiments, an inflammation-associated antigen is TLR4, TLR2, NOD2 or RAGE.

In some embodiments, an inflammation-associated antigen is a complement protein. In some embodiments, an inflammation-associated antigen is C3, C5a or C1q.

In some embodiments, an inflammation-associated antigen may be an autoimmune-disease-associated antigen. An 'autoimmune-disease-associated antigen' refers to an antigen whose presence is indicative of an autoimmune-disease, or an antigen for which an elevated level of the antigen is positively-correlated with an autoimmune-disease. The autoimmune-disease-associated antigen may be an antigen whose gene and/or protein expression is associated with the development, progression or severity of symptoms of an autoimmune-disease. The autoimmune-disease-associated antigen may be associated with the cause or pathology of the autoimmune-disease, or may be expressed abnormally as a consequence of an autoimmune-disease.

In some embodiments, an autoimmune-disease-associated antigen is an antigen associated with an autoimmune-disease selected from: systemic lupus erythematosus (SLE), type-1 diabetes, arthritis, rheumatoid arthritis (RA), osteoarthritis (OA), psoriatic arthritis, gout, ankylosing spondylitis, juvenile idiopathic arthritis, lupus-related arthritis, type 1 diabetes mellitus, celiac disease, psoriasis, inflammatory bowel disease, Crohn's disease, ulcerative colitis, Graves' disease, Hashimoto's thyroiditis, multiple sclerosis, systemic lupus erythematosus, pernicious anemia, Sjögren's syndrome, vitiligo, autoimmune hepatitis, primary biliary cholangitis, ankylosing spondylitis, myasthenia gravis, Addison's disease, systemic sclerosis, scleroderma, pemphigus vulgaris, dermatomyositis, polymyositis, Guillain-Barré syndrome, antiphospholipid syndrome, granulomatosis with polyangiitis (GPA), immune thrombocytopenic purpura, and Lambert-Eaton myasthenic syndrome.

In some embodiments, an autoimmune-disease-associated antigen is selected from: an anti-nuclear antibody (ANA), double-stranded DNA (dsDNA), Smith (Sm) antigen, ribonucleoprotein (RNP), SSA/Ro, SSB/La, a histone protein, a chromatin component, thyroglobulin, thyroid peroxidase (TPO), TSH receptor, glutamic acid decarboxylase (GAD65), insulin, IA-2, desmoglein-1, desmoglein-3, BP180, BP230, a melanocyte antigen (e.g. tyrosinase), tissue transglutaminase (tTG), intrinsic factor, an anti-Saccharomyces cerevisiae antibody (ASCA), liver-kidney microsome-1 (LKM-1), an anti-mitochondrial antibody (AMA), a smooth muscle antibodies (SMA), aquaporin-4 (AQP4), acetylcholine receptor (AChR), myelin oligodendrocyte glycoprotein (MOG), voltage-gated calcium channel (VGCC), cyclic citrullinated peptide (CCP), rheumatoid factor (RF), anti-Scl-70 (Topoisomerase I), a centromere protein, a platelet glycoprotein (e.g. GPllb/llla), beta-2 glycoprotein I, and a neutrophil cytoplasmic antigen (ANCA).

### Hinge

In some embodiments, the CAR comprises a hinge. The hinge may be provided between the antigen-binding domain and the transmembrane domain (*e.g*., between the second interaction domain and the transmembrane domain - see Figure 1). A hinge may also be referred to as a hinge domain, or a spacer domain. A hinge is an amino acid sequence which provides for flexible linkage of the antigen-binding and transmembrane domains of the CAR.

Hinge domains can lend flexibility to the antigen-binding domain, and can thus enable recognition of target epitopes that would otherwise be sterically inaccessible. For example, in Wilkie et al., J Immunol. (2008) 180:4901-4909 replacement of a CD28 spacer region with a highly-flexible IgD spacer improved binding of the CAR to a sterically hindered epitope of MUC1.

Hinge domains can also be engineered to regulate synaptic cleft distances, and hence modulate signalling. To maintain optimal synapse distance, membrane-proximal epitopes typically require shorter spacers, while membrane-distal epitopes require longer spacers (Hudecek et al., Clin Cancer Res. (2013) 19:3153-3164). Increasing epitope-paratope distance permits the interference by inhibitory phosphatases, and can result in impaired delivery of perforins and granzymes to the target cell, thereby reducing the efficiency of cytolysis. A compact immunological synapse impairs diffusion of lytic granules, enhancing pore formation by perforins and delivery of granzymes (Woodsworth et al., Biophys J. (2015) 109:477-488).

Human IgG1 Fc, IgG4 Fc and IgG2 Fc spacer domains have been employed in CARs, and variants of IgG1 Fc and IgG4 Fc spacers designed to reduce/eliminate binding to FcyRs have been shown to reduce depletion of cells expressing the CARs and improve persistence *in vivo.*

Non-lgG Fc-based hinges such as CD8 and CD28 hinges have also been used in clinically-approved CAR-T cell therapies. A CD28-derived spacer has been shown to result in increased CAR activation compared to a CD8α-derived spacer, which might be a consequence of an increased propensity of the CD28-derived spacer to participate in homotypic associations (Alabanza et al., Mol. Ther. (2017) 25:2452-2465).

In some embodiments, a hinge according to the present disclosure comprises, or consists of, an amino acid sequence derived from the amino acid sequence of one of CD8α, 41BB, OX40, CD96 CD44, IgG2 or IgG1. In some embodiments, a hinge according to the present disclosure comprises, or consists of, an amino acid sequence derived from the amino acid sequence of one of CD8α, 41BB, OX40, CD96 or CD44. In some embodiments, a hinge according to the present disclosure comprises, or consists of, an amino acid sequence derived from the amino acid sequence of CD8α.

In some embodiments, a hinge according to the present disclosure comprises, or consists of, an amino acid sequence having at least 80%, 85%, 86%, 87%, 88%, 89%, 90%, 91%, 92%, 93%, 94%, 95%, 96%, 97%, 98%, 99%, or 100% sequence identity to the amino acid sequence of SEQ ID NO:41.

### Transmembrane domain

The CAR of the present disclosure comprises a transmembrane domain. A transmembrane domain refers to any three-dimensional structure formed by a sequence of amino acids which is thermodynamically stable in a biological membrane, *e.g.* a cell membrane. In connection with the present disclosure, the transmembrane domain may be an amino acid sequence which spans the cell membrane of a cell expressing the CAR.

The transmembrane domain may comprise or consist of a sequence of amino acids which forms a hydrophobic alpha helix or beta-barrel. The amino acid sequence of the transmembrane domain of the CAR of the present disclosure may be, or may be derived from, the amino acid sequence of a transmembrane domain of a protein comprising a transmembrane domain. Transmembrane domains are recorded in databases such as GenBank, UniProt, Swiss-Prot, TrEMBL, Protein Information Resource, Protein Data Bank, Ensembl, and InterPro, and/or can be identified/predicted *e.g.* using amino acid sequence analysis tools such as TMHMM (Krogh et al., 2001 J Mol Biol 305: 567-580).

In some embodiments, the amino acid sequence of the transmembrane domain of the CAR of the present disclosure may be, or may be derived from, the amino acid sequence of the transmembrane domain of a protein expressed at the cell surface. In some embodiments the protein expressed at the cell surface is a receptor or ligand, *e.g.* an immune receptor or ligand. In some embodiments the amino acid sequence of the transmembrane domain may be, or may be derived from, the amino acid sequence of the transmembrane domain of one of CD8α, ICOS, ICOSL, CD86, CTLA-4, CD28, CD80, MHC class I α, MHC class II α, MHC class II β, CD3ε, CD3δ, CD3γ, CD3-ζ, TCRα TCRβ, CD4, CD8β, CD40, CD40L, PD-1, PD-L1, PD-L2, 4-1BB, 4-1BBL, OX40, OX40L, GITR, GITRL, TIM-3, Galectin 9, LAG3, CD27, CD70, LIGHT, HVEM, TIM-4, TIM-1, ICAM1, LFA-1, LFA-3, CD2, BTLA, CD160, LILRB4, LILRB2, VTCN1, CD2, CD48, 2B4, SLAM, CD30, CD30L, DR3, TL1A, CD226, CD155, CD112 and CD276. In some embodiments, the transmembrane is, or is derived from, the amino acid sequence of the transmembrane domain of CD8α, CD28, CD3-ζ, CD8β or CD4. In some embodiments, the transmembrane is, or is derived from, the amino acid sequence of the transmembrane domain of CD8α.

In some embodiments, the transmembrane domain comprises, or consists of, an amino acid sequence having at least 80%, 85%, 86%, 87%, 88%, 89%, 90%, 91%, 92%, 93%, 94%, 95%, 96%, 97%, 98%, 99%, or 100% sequence identity to the amino acid sequence of SEQ ID NO:42.

### Signalling domain

The chimeric antigen receptor of the present disclosure comprises a signalling domain. The signalling domain provides sequences for initiating intracellular signalling in cells expressing the CAR. A signalling domain may alternatively be referred to as a cytoplasmic domain, intracellular domain, cytoplasmic signalling domain, or an intracellular signalling domain.

### ITAM-containing sequence:

The signalling domain comprises an ITAM-containing sequence. An ITAM-containing sequence comprises one or more immunoreceptor tyrosine-based activation motifs (ITAMs). ITAMs comprise the amino acid sequence YXXL/I (SEQ ID NO:43), wherein 'X' denotes any amino acid. In ITAM-containing proteins, sequences according to SEQ ID NO:43 are often separated by 6 to 8 amino acids; YXXL/I(X)₆₋₈YXXL/I (SEQ ID NO:44). When phosphate groups are added to the tyrosine residue of an ITAM by tyrosine kinases, a signalling cascade is initiated within the cell.

In some embodiments, the signalling domain comprises one or more copies of an amino acid sequence according to SEQ ID NO:43 or SEQ ID NO:44. In some embodiments, the signalling domain comprises at least 1, 2, 3, 4, 5 or 6 copies of an amino acid sequence according to SEQ ID NO:43. In some embodiments, the signalling domain comprises at least 1, 2, or 3 copies of an amino acid sequence according to SEQ ID NO:44.

In some embodiments, the signalling domain comprises an ITAM-containing sequence which is, or which is derived from, the amino acid sequence of an ITAM-containing sequence of a protein having an ITAM-containing amino acid sequence. In some embodiments the signalling domain comprises an ITAM-containing sequence which is, or which is derived from, the amino acid sequence of the intracellular domain of one of CD3-ζ, FcyRI, CD3ε, CD3δ, CD3γ, CD79α, CD79β, FcyRIIA, FcyRIIC, FcyRIIIA, FcyRIV or DAP12. In some embodiments the signalling domain comprises an ITAM-containing sequence which is, or which is derived from, the amino acid sequence of the intracellular domain of CD3-ζ.

In some embodiments, the signalling domain comprises an ITAM-containing sequence which comprises, or consists of, an amino acid sequence having at least 80%, 85%, 86%, 87%, 88%, 89%, 90%, 91%, 92%, 93%, 94%, 95%, 96%, 97%, 98%, 99%, or 100% sequence identity to the amino acid sequence of SEQ ID NO:46.

### Costimulatory sequence:

The signalling domain may additionally comprise one or more costimulatory sequences. A costimulatory sequence is an amino acid sequence which provides for costimulation of the cell expressing the CAR of the present disclosure. Costimulation promotes proliferation and survival of a CAR-expressing cell upon binding to the target antigen, and may also promote cytokine production, differentiation, cytotoxic function and memory formation by the CAR-expressing cell. Molecular mechanisms of T cell costimulation are reviewed in Chen and Flies, (2013) Nat Rev Immunol 13(4):227-242.

A costimulatory sequence may be, or may be derived from, the amino acid sequence of a costimulatory protein. In some embodiments the costimulatory sequence is an amino acid sequence which is, or which is derived from, the amino acid sequence of the intracellular domain of a costimulatory protein.

Upon binding of the CAR to the target antigen, the costimulatory sequence provides costimulation to the cell expressing the CAR of the kind which would be provided by the costimulatory protein from which the costimulatory sequence is derived upon ligation by its cognate ligand. By way of example in the case of a CAR comprising a signalling domain comprising a costimulatory sequence derived from CD28, binding to the target antigen triggers signalling in the cell expressing the CAR of the kind that would be triggered by binding of CD80 and/or CD86 to CD28. Thus, a costimulatory sequence is capable of delivering the costimulation signal of the costimulatory protein from which the costimulatory sequence is derived.

In some embodiments, the costimulatory protein may be a member of the TNF receptor superfamily (e.g. 4-1BB, OX40, CD27, DR3, GITR, CD30, HVEM), or a member of the B7-CD28 superfamily (e.g. CD28, ICOS). In some embodiments, the costimulatory sequence is, or is derived from, the intracellular domain of one of 4-1BB, CD28, ICOS, CD27, OX40, HVEM, CD2, SLAM, TIM-1, CD30, GITR, DR3, CD226 and LIGHT. In some embodiments, the costimulatory sequence is, or is derived from, the intracellular domain of CD28.

In some embodiments the signalling domain comprises more than one costimulatory sequence. In some embodiments the signalling domain comprises 1, 2, 3, 4, 5 or 6 costimulatory sequences. Plural costimulatory sequences may be provided in tandem.

Whether a given amino acid sequence is capable of initiating signalling mediated by a given costimulatory protein can be investigated *e.g.* by analysing a correlate of signalling mediated by the costimulatory protein (*e.g*. expression/activity of a factor whose expression/activity is upregulated or downregulated as a consequence of signalling mediated by the costimulatory protein).

Costimulatory proteins upregulate expression of genes promoting cell growth, effector function and survival through several transduction pathways. For example, 4-1BB, OX40, and CD27 recruit TNF receptor associated factor (TRAF) and signal through MAPK pathways, as well as through PI3K. CD28 and ICOS signal through phosphatidylinositol 3 kinase (PI3K) and AKT to upregulate expression of genes promoting cell growth, effector function and survival through NF-κB, mTOR, NFAT and AP1/2. CD28 also activates AP1/2 via CDC42/RAC1 and ERK1/2 via RAS, and ICOS activates C-MAF.

In some embodiments the signalling domain comprises a costimulatory sequence which is, or which is derived from 4-1BB and/or CD28. In some embodiments the signalling domain comprises a costimulatory sequence which is, or which is derived from 4-1BB. In some embodiments the signalling domain comprises a costimulatory sequence which is, or which is derived from CD28.

In some embodiments, the signalling domain comprises, or consists of, an amino acid sequence having at least 80%, 85%, 86%, 87%, 88%, 89%, 90%, 91%, 92%, 93%, 94%, 95%, 96%, 97%, 98%, 99%, or 100% sequence identity to the amino acid sequence of SEQ ID NO:45.

### Linkers and additional sequences

The CARs of the present disclosure may additionally comprise further amino acids or sequences of amino acids.

An amino acid sequence described herein (*e.g*. an amino acid sequence of a domain/region of a CAR described herein) may comprise one or more additional amino acids at one or both ends (*i.e*. the N- or C-terminus) of the reference amino acid sequence. In some embodiments, the peptide/polypeptide comprises *e.g.* 1-5, 1-10, 1-20, 1-30, 1-40, 1-50, 5-10, 5-20, 5-30, 5-40, 5-50, 10-20, 10-30, 10-40, 10-50, 20-30, 20-40 or 20-50 additional amino acids at one or both ends of the reference amino acid sequence. In some embodiments, the additional amino acids correspond to the amino acids provided at those positions relative to the reference amino acid sequence, in the context of the protein from which the reference amino acid sequence is derived. By way of illustration, where a reference amino acid sequence corresponds to amino acid positions 20 to 30 of the amino acid sequence of the protein from which the reference amino acid sequence is derived, and wherein the amino acid sequence an additional 5 amino acids at its N-terminus, those additional 5 amino acids may correspond to positions 15 to 19 of the amino acid sequence of the protein from which the reference amino acid sequence is derived.

The CARs of the present disclosure may comprise one or more linker sequences between sequences of amino acids. For example, a linker sequence may be provided between domains of a CAR (*e.g*. between the antigen-binding domain and spacer domain, and/or between the spacer domain and the transmembrane domain, and/or between the transmembrane domain and the signalling domain). By way of further example, a linker sequence may be provided between subsequences of the domains of a CAR (*e.g.* between VH and VL of an antigen-binding domain, and/or between the costimulatory and sequence and ITAM-containing sequence of a signalling domain).

Linker sequences are known to the skilled person, and are described, for example in Chen et al., Adv Drug Deliv Rev (2013) 65(10): 1357-1369, which is hereby incorporated by reference in its entirety. In some embodiments, a linker sequence may be a flexible linker sequence. Flexible linker sequences allow for relative movement of the amino acid sequences which are linked by the linker sequence. Flexible linkers are known to the skilled person, and several are identified in Chen *et al.,* Adv Drug Deliv Rev

(2013) 65(10): 1357-1369. Flexible linker sequences often comprise high proportions of glycine and/or serine residues.

In some embodiments, the linker sequence comprises at least one glycine residue and/or at least one serine residue. In some embodiments, the linker sequence comprises or consists of glycine and serine residues. In some embodiments, the linker sequence has the structure: (GxS)n or (GxS)nGm ,wherein G = glycine, S = serine, x = 3 or 4, n = 2, 3, 4, 5 or 6, and m = 0, 1, 2 or 3. In some embodiments, the linker sequence comprises one or more (*e.g*. 1, 2, 3, 4, 5 or 6) copies (*e.g*. in tandem) of the sequence motif G₄S (SEQ ID NO:35). In some embodiments, the linker sequence comprises or consists of (G₄S)₄ (SEQ ID NO:40). In some embodiments, the linker sequence has a length of 1-2, 1-3, 1-4, 1-5, 1-10, 1-15, 1-20, 1-25, or 1-30 amino acids.

In some embodiments, the linker sequence comprises one or more copies of an amino acid sequence according to SEQ ID NO:35. In some embodiments, the linker sequence comprises at least 1, 2, 3 or 4 copies of an amino acid sequence according to SEQ ID NO:35.

The CARs of the present disclosure may comprise amino acid sequence(s) to facilitate expression, folding, trafficking, processing, purification or detection of the antigen-binding molecule/polypeptide. For example, antigen-binding molecules and polypeptides of the present disclosure may additionally comprise a sequence of amino acids forming a detectable moiety, *e.g.* as described hereinbelow.

The CARs of the present disclosure may additionally comprise a signal peptide (also known as a leader sequence or signal sequence). Signal peptides normally consist of a sequence of 5-30 hydrophobic amino acids, which form a single alpha helix. Secreted proteins and proteins expressed at the cell surface often comprise signal peptides. Signal peptides are known for many proteins, and are recorded in databases such as GenBank, UniProt and Ensembl, and/or can be identified/predicted *e.g.* using amino acid sequence analysis tools such as SignalP (Petersen et al., 2011 Nature Methods 8: 785-786) or Signal-BLAST (Frank and Sippl, 2008 Bioinformatics 24: 2172-2176).

The signal peptide may be present at the N-terminus of the CAR, and may be present in the newly synthesised CAR. The signal peptide provides for efficient trafficking of the CAR to the cell surface. Signal peptides are often removed by cleavage, and thus are not comprised in the mature CAR expressed at the cell surface.

Signal peptides are known for many proteins, and are recorded in databases such as GenBank, UniProt, Swiss-Prot, TrEMBL, Protein Information Resource, Protein Data Bank, Ensembl, and InterPro, and/or can be identified/predicted *e.g.* using amino acid sequence analysis tools such as SignalP (Petersen et al., 2011 Nature Methods 8: 785-786) or Signal-BLAST (Frank and Sippl, 2008 Bioinformatics 24: 2172-2176).

### Labels and conjugates

In some embodiments, the CAR of the present disclosure additionally comprises a detectable moiety.

In some embodiments, a detectable moiety is a fluorescent label, phosphorescent label, luminescent label, immuno-detectable label (*e.g*. an epitope tag), radiolabel, chemical, nucleic acid or enzymatic label. The CAR may be covalently or non-covalently labelled with the detectable moiety.

Fluorescent labels include *e.g.* fluorescein, rhodamine, allophycocyanin, eosine and NDB, green fluorescent protein (GFP), chelates of rare earths such as europium (Eu), terbium (Tb) and samarium (Sm), tetramethyl rhodamine, Texas Red, 4-methyl umbelliferone, 7-amino-4-methyl coumarin, Cy3, and Cy5. Radiolabels include radioisotopes such as Hydrogen³, Sulfur³⁵, Carbon¹⁴, Phosphorus³², Iodine¹²³, Iodine¹²⁵, Iodine¹²⁶, Iodine¹³¹, Iodine¹³³, Bromine⁷⁷, Technetium^{99m}, Indium¹¹¹, Indium^{113m}, Gallium⁶⁷, Gallium⁶⁸, Ruthenium⁹⁵, Ruthenium⁹⁷, Ruthenium¹⁰³, Ruthenium¹⁰⁵, Mercury²⁰⁷, Mercury²⁰³, Rhenium^{99m}, Rhenium¹⁰¹, Rhenium¹⁰⁵, Scandium⁴⁷, Tellurium^{121m}, Tellurium^{122m}, Tellurium^{125m}, Thulium¹⁶⁵, Thuliuml¹⁶⁷, Thulium¹⁶⁸, Copper⁶⁷, Fluorine¹⁸, Yttrium⁹⁰, Palladium¹⁰⁰, Bismuth²¹⁷ and Antimony²¹¹. Luminescent labels include as radioluminescent, chemiluminescent (*e.g.* acridinium ester, luminol, isoluminol) and bioluminescent labels. Immuno-detectable labels include haptens, peptides/polypeptides, antibodies, receptors and ligands such as biotin, avidin, streptavidin or digoxigenin. Nucleic acid labels include aptamers.

In some embodiments, the CAR comprises an epitope tag, *e.g.* a His, FLAG, c-Myc, StrepTag, haemagglutinin, E, calmodulin-binding protein (CBP), glutathione-s-transferase (GST), maltose-binding protein (MBP), thioredoxin, S-peptide, T7 peptide, SH2 domain, avidin, streptavidin, and haptens (*e.g.* biotin, digoxigenin, dinitrophenol), optionally at the N- or C- terminus of the antigen-binding molecule/polypeptide/CAR.

In some embodiments, the CAR comprises a moiety having a detectable activity, *e.g*. an enzymatic moiety. Enzymatic moieties include *e.g.* luciferases, glucose oxidases, galactosidases (*e.g*. beta-galactosidase), glucorinidases, phosphatases (*e.g.* alkaline phosphatase), peroxidases (*e.g*. horseradish peroxidase) and cholinesterases.

In some embodiments, the CAR of the present disclosure is conjugated to a chemical moiety. The chemical moiety may be a moiety for providing a therapeutic effect, *i.e.* a drug moiety. A drug moiety may be a small molecule (*e.g.* a low molecular weight (< 1000 daltons, typically between ~300-700 daltons) organic compound). Drug moieties are described *e.g.* in Parslow et al., Biomedicines. 2016 Sep; 4(3):14 (hereby incorporated by reference in its entirety). In some embodiments, a drug moiety may be or comprise a cytotoxic agent. In some embodiments, a drug moiety may be or comprise a chemotherapeutic agent. Drug moieties include *e.g.* calicheamicin, DM1, DM4, monomethylauristatin E (MMAE), monomethylauristatin F (MMAF), SN-38, doxorubicin, duocarmycin, D6.5 and PBD.

### Particular exemplary polypeptides and CARs

A polypeptide according to the present disclosure may comprise:
(i) a first interaction domain comprising a cysteine,
(ii) an antigen-binding domain which binds specifically to a target antigen, and
(iii) a second interaction domain comprising a cysteine.

In some embodiments, the polypeptide according to the present disclosure comprises, or consists of:
(i) a first interaction domain comprising or consisting of an amino acid sequence having at least 60%, 65%, 70%, 75%, 80%, 85%, 86%, 87%, 88%, 89%, 90%, 91%, 92%, 93%, 94%, 95%, 96%, 97%, 98%, 99%, or 100% sequence identity to the amino acid sequence of SEQ ID NO:1, 2, 3, 4, 5, 6, 7, or 8,
(ii) an antigen-binding domain which binds specifically to a target antigen,
(iii) a second interaction domain comprising or consisting of an amino acid sequence having at least 60%, 65%, 70%, 75%, 80%, 85%, 86%, 87%, 88%, 89%, 90%, 91%, 92%, 93%, 94%, 95%, 96%, 97%, 98%, 99%, or 100% sequence identity to the amino acid sequence of SEQ ID NO:1, 2, 3, 4, 5, 6, 7, or 8.

In some embodiments, the polypeptide according to the present disclosure comprises, or consists of:
(i) a first interaction domain comprising or consisting of an amino acid sequence having at least 60%, 65%, 70%, 75%, 80%, 85%, 86%, 87%, 88%, 89%, 90%, 91%, 92%, 93%, 94%, 95%, 96%, 97%, 98%, 99%, or 100% sequence identity to the amino acid sequence of SEQ ID NO:1, 2, 3, 4, 5, 6, 7, or 8,
(ii) an antigen-binding domain comprising or consisting of an amino acid sequence having at least 60%, 65%, 70%, 75%, 80%, 85%, 86%, 87%, 88%, 89%, 90%, 91%, 92%, 93%, 94%, 95%, 96%, 97%, 98%, 99%, or 100% sequence identity to the amino acid sequence(s) of: SEQ ID NO:58 and 62; SEQ ID NO:67 and 71; SEQ ID NO:66 or SEQ ID NO:75,
(iii) a second interaction domain comprising or consisting of an amino acid sequence having at least 60%, 65%, 70%, 75%, 80%, 85%, 86%, 87%, 88%, 89%, 90%, 91%, 92%, 93%, 94%, 95%, 96%, 97%, 98%, 99%, or 100% sequence identity to the amino acid sequence of SEQ ID NO:1, 2, 3, 4, 5, 6, 7, or 8.

A CAR according to the present disclosure may comprise, or consist of:
(i) a first interaction domain comprising a cysteine,
(ii) an antigen-binding domain which binds specifically to a target antigen,
(iii) a second interaction domain comprising a cysteine,
(iv) a hinge,
(v) a transmembrane domain, and
(vi) a signalling domain.

In some embodiments, the CAR according to the present disclosure comprises, or consists of:
(i) a first interaction domain comprising or consisting of an amino acid sequence having at least 60%, 65%, 70%, 75%, 80%, 85%, 86%, 87%, 88%, 89%, 90%, 91%, 92%, 93%, 94%, 95%, 96%, 97%, 98%, 99%, or 100% sequence identity to the amino acid sequence of SEQ ID NO:1, 2, 3, 4, 5, 6, 7, or 8,
(ii) an antigen-binding domain which binds specifically to a target antigen,
(iii) a second interaction domain comprising or consisting of an amino acid sequence having at least 60%, 65%, 70%, 75%, 80%, 85%, 86%, 87%, 88%, 89%, 90%, 91%, 92%, 93%, 94%, 95%, 96%, 97%, 98%, 99%, or 100% sequence identity to the amino acid sequence of SEQ ID NO:1, 2, 3, 4, 5, 6, 7, or 8,
(iv) a hinge,
(v) a transmembrane domain, and
(vi) a signalling domain.

In some embodiments, the CAR according to the present disclosure comprises, or consists of:
(i) a first interaction domain comprising or consisting of an amino acid sequence having at least 60%, 65%, 70%, 75%, 80%, 85%, 86%, 87%, 88%, 89%, 90%, 91%, 92%, 93%, 94%, 95%, 96%, 97%, 98%, 99%, or 100% sequence identity to the amino acid sequence of SEQ ID NO:1, 2, 3, 4, 5, 6, 7, or 8,
(ii) an antigen-binding domain which binds specifically to a target antigen,
(iii) a second interaction domain comprising or consisting of an amino acid sequence having at least 60%, 65%, 70%, 75%, 80%, 85%, 86%, 87%, 88%, 89%, 90%, 91%, 92%, 93%, 94%, 95%, 96%, 97%, 98%, 99%, or 100% sequence identity to the amino acid sequence of SEQ ID NO:1, 2, 3, 4, 5, 6, 7, or 8,
(iv) a hinge comprising or consisting of an amino acid sequence having at least 60%, 65%, 70%, 75%, 80%, 85%, 86%, 87%, 88%, 89%, 90%, 91%, 92%, 93%, 94%, 95%, 96%, 97%, 98%, 99%, or 100% sequence identity to the amino acid sequence of SEQ ID NO:41,
(v) a transmembrane domain comprising or consisting of an amino acid sequence having at least 60%, 65%, 70%, 75%, 80%, 85%, 86%, 87%, 88%, 89%, 90%, 91%, 92%, 93%, 94%, 95%, 96%, 97%, 98%, 99%, or 100% sequence identity to the amino acid sequence of SEQ ID NO:42, and
(vi) a signalling domain comprising or consisting of an amino acid sequence having at least 60%, 65%, 70%, 75%, 80%, 85%, 86%, 87%, 88%, 89%, 90%, 91%, 92%, 93%, 94%, 95%, 96%, 97%, 98%, 99%, or 100% sequence identity to the amino acid sequence of SEQ ID NO:43, 44, or 46, and/or an amino acid sequence having at least 60%, 65%, 70%, 75%, 80%, 85%, 86%, 87%, 88%, 89%, 90%, 91%, 92%, 93%, 94%, 95%, 96%, 97%, 98%, 99%, or 100% sequence identity to the amino acid sequence of SEQ ID NO:45.

In some embodiments, the CAR according to the present disclosure comprises, or consists of:
(i) a first interaction domain comprising or consisting of an amino acid sequence having at least 60%, 65%, 70%, 75%, 80%, 85%, 86%, 87%, 88%, 89%, 90%, 91%, 92%, 93%, 94%, 95%, 96%, 97%, 98%, 99%, or 100% sequence identity to the amino acid sequence of SEQ ID NO:1, 2, 3, 4, 5, 6, 7, or 8,
(ii) an antigen-binding domain comprising or consisting of an amino acid sequence having at least 60%, 65%, 70%, 75%, 80%, 85%, 86%, 87%, 88%, 89%, 90%, 91%, 92%, 93%, 94%, 95%, 96%, 97%, 98%, 99%, or 100% sequence identity to the amino acid sequence(s) of: SEQ ID NO:58 and 62; SEQ ID NO:67 and 71; SEQ ID NO:66 or SEQ ID NO:75,
(iii) a second interaction domain comprising or consisting of an amino acid sequence having at least 60%, 65%, 70%, 75%, 80%, 85%, 86%, 87%, 88%, 89%, 90%, 91%, 92%, 93%, 94%, 95%, 96%, 97%, 98%, 99%, or 100% sequence identity to the amino acid sequence of SEQ ID NO:1, 2, 3, 4, 5, 6, 7, or 8,
(iv) a hinge comprising or consisting of an amino acid sequence having at least 60%, 65%, 70%, 75%, 80%, 85%, 86%, 87%, 88%, 89%, 90%, 91%, 92%, 93%, 94%, 95%, 96%, 97%, 98%, 99%, or 100% sequence identity to the amino acid sequence of SEQ ID NO:41,
(v) a transmembrane domain comprising or consisting of an amino acid sequence having at least 60%, 65%, 70%, 75%, 80%, 85%, 86%, 87%, 88%, 89%, 90%, 91%, 92%, 93%, 94%, 95%, 96%, 97%, 98%, 99%, or 100% sequence identity to the amino acid sequence of SEQ ID NO:42, and
(vi) a signalling domain comprising or consisting of an amino acid sequence having at least 60%, 65%, 70%, 75%, 80%, 85%, 86%, 87%, 88%, 89%, 90%, 91%, 92%, 93%, 94%, 95%, 96%, 97%, 98%, 99%, or 100% sequence identity to the amino acid sequence of SEQ ID NO:43, 44, or 46, and/or an amino acid sequence having at least 60%, 65%, 70%, 75%, 80%, 85%, 86%, 87%, 88%, 89%, 90%, 91%, 92%, 93%, 94%, 95%, 96%, 97%, 98%, 99%, or 100% sequence identity to the amino acid sequence of SEQ ID NO:45.

In some embodiments, the CAR comprises, or consists of an amino acid sequence having at least 60%, 65%, 70%, 75%, 80%, 85%, 86%, 87%, 88%, 89%, 90%, 91%, 92%, 93%, 94%, 95%, 96%, 97%, 98%, 99%, or 100% sequence identity to the amino acid sequence of SEQ ID NO:9, 10, 11, 12, 13, 14, 15, 49, 50, 51, 52, 53, 54, or 55.

In some embodiments, the CAR comprises, or consists of an amino acid sequence having at least 60%, 65%, 70%, 75%, 80%, 85%, 86%, 87%, 88%, 89%, 90%, 91%, 92%, 93%, 94%, 95%, 96%, 97%, 98%, 99%, or 100% sequence identity to the amino acid sequence of SEQ ID NO:9, 10, 11, 12, 13, 14, or 15.

In some embodiments, the CAR comprises, or consists of an amino acid sequence having at least 60%, 65%, 70%, 75%, 80%, 85%, 86%, 87%, 88%, 89%, 90%, 91%, 92%, 93%, 94%, 95%, 96%, 97%, 98%, 99%, or 100% sequence identity to the amino acid sequence of SEQ ID NO: 49, 50, 51, 52, 53, 54, or 55.

### Nucleic acids and vectors

The present disclosure provides a nucleic acid, or a plurality of nucleic acids, encoding a CAR according to the present disclosure. In some embodiments, the nucleic acid(s) comprise or consist of DNA and/or RNA.

In some embodiments, the nucleic acid(s) may be, or may be comprised in, a vector, or a plurality of vectors. That is, the nucleotide sequence(s) of the nucleic acid(s) may be contained in vector(s). The CAR according to the present disclosure may be produced within a cell by transcription from a vector encoding the antigen-binding molecule, polypeptide or CAR, and subsequent translation of the transcribed RNA.

Accordingly, the present disclosure also provides a vector, or plurality of vectors, comprising the nucleic acid or plurality of nucleic acids according to the present disclosure. The vector may facilitate delivery of the nucleic acid(s) encoding an CAR according to the present disclosure. The vector may be an expression vector comprising elements required for expressing nucleic acid(s) comprising/encoding an CAR according to the present disclosure.

Nucleic acids and vectors according to the present disclosure may be provided in purified or isolated form, i.e. from other nucleic acid, or naturally-occurring biological material.

The nucleotide sequence may be contained in a vector, e.g. an expression vector. A 'vector' as used herein is a nucleic acid molecule used as a vehicle to transfer exogenous nucleic acid into a cell. The vector may be a vector for expression of the nucleic acid in the cell. Such vectors may include a promoter sequence operably linked to the nucleotide sequence encoding the sequence to be expressed. A vector may also include a termination codon and expression enhancers. Any suitable vectors, promoters, enhancers and termination codons known in the art may be used to express a peptide or polypeptide from a vector according to the present disclosure.

The term 'operably linked' may include the situation where a selected nucleic acid sequence and regulatory nucleic acid sequence (e.g. promoter and/or enhancer) are covalently linked in such a way as to place the expression of nucleic acid sequence under the influence or control of the regulatory sequence (thereby forming an expression cassette). Thus a regulatory sequence is operably linked to the selected nucleic acid sequence if the regulatory sequence is capable of effecting transcription of the nucleic acid sequence. The resulting transcript(s) may then be translated into a desired peptide(s)/polypeptide(s).

Suitable vectors include plasmids, binary vectors, DNA vectors, mRNA vectors, viral vectors (e.g. retroviral vectors, e.g. gammaretroviral vectors (e.g. murine Leukemia virus (MLV)-derived vectors, e.g. SFG vector), lentiviral vectors, adenovirus vectors, adeno-associated virus vectors, vaccinia virus vectors and herpesvirus vectors), transposon-based vectors, and artificial chromosomes (e.g. yeast artificial chromosomes), e.g. as described in Maus et al., Annu Rev Immunol (2014) 32:189-225 or Morgan and Boyerinas, Biomedicines (2016) 4:9, which are both hereby incorporated by reference in their entirety.

In some embodiments, the vector may be a eukaryotic vector, e.g. a vector comprising the elements necessary for expression of protein from the vector in a eukaryotic cell. In some embodiments, the vector may be a mammalian vector, e.g. comprising a cytomegalovirus (CMV) or SV40 promoter to drive protein expression.

### Cells expressing the CARs of the disclosure

In some aspects and embodiments, the present disclosure provides a cell comprising a CAR according to the present disclosure. The CAR according to the present disclosure may be used to generate a CAR-expressing cell, *e.g.* a CAR-expressing immune cells(*e.g.* CAR-T or CAR-NK cell).

It will be appreciated that where cells are referred to herein in the singular (*i.e.* 'a/the cell'), pluralities/populations of such cells are also contemplated.

CAR-expressing cells may comprise or express nucleic acid encoding a CAR according to the present disclosure. It will be appreciated that a CAR-expressing cell comprises the CAR it expresses. It will also be appreciated that a cell expressing nucleic acid encoding a CAR also expresses and comprises the CAR encoded by the nucleic acid.

The cell may be a eukaryotic cell, *e.g.* a mammalian cell. The mammal may be a primate (rhesus, cynomolgous, non-human primate or human) or a non-human mammal (*e.g.* rabbit, guinea pig, rat, mouse or other rodent (including any animal in the order Rodentia), cat, dog, pig, sheep, goat, cattle (including cows, *e.g.* dairy cows, or any animal in the order Bos), horse (including any animal in the order Equidae), donkey, and non-human primate).

CAR-expressing cell is preferably an immune cell. An immune cell may be a cell of hematopoietic origin, *e.g.* a neutrophil, eosinophil, basophil, dendritic cell, lymphocyte, or monocyte. A lymphocyte may be *e.g.* a T cell, B cell, NK cell, NKT cell or innate lymphoid cell (ILC), or a precursor thereof. The immune cell may express *e.g.* CD3 polypeptides (*e.g.* CD3γ CD3ε CD3ζ or CD3δ), TCR polypeptides (TCRα or TCRβ), CD27, CD28, CD4 or CD8. In some embodiments, the immune cell is a T cell, *e.g.* a CD3+ T cell. In some embodiments, the T cell is a CD3+, CD4+ T cell. In some embodiments, the T cell is a CD3+, CD8+ T cell. In some embodiments, the T cell is a T helper cell (T_{H} cell). In some embodiments, the T cell is a cytotoxic T cell (*e.g.* a cytotoxic T lymphocyte (CTL)).

In some embodiments in accordance with the various aspects of the present disclosure, cells may comprise/express more than one (*e.g.* 2, 3, 4, *etc.*) CAR.

In some embodiments, the cells may comprise/express more than one, non-identical CAR. Cells comprising/expressing more than one non-identical CAR may comprise/express CARs specific for non-identical target antigens. In some embodiments, each non-identical target antigen is independently a cancer cell antigen as described herein.

### Functional properties of cells expressing the CARs of the disclosure

Cells (*e.g.* immune cells, *e.g.* T cells) expressing a CAR according to the present disclosure may display certain functional properties in response to the target antigen for the CAR, or in response a cell comprising/expressing the target antigen for the CAR. In some embodiments, the properties are functional properties associated with effector T cells, *e.g.* cytotoxic T cells.

Cells comprising a CAR/nucleic acid encoding a CAR according to the present disclosure may display one or more of the following properties:
expression of one or more cytotoxic/effector factors (*e.g.* IFNγ, TNFα, GM-CSF),
secretion of cytokines (e.g., IL-2, and/or IFNγ);
proliferation/population expansion in response to cells expressing the target antigen for the CAR;
cytotoxicity to cells expressing the target antigen for the CAR;
anti-cancer activity (*e.g.* cytotoxicity to cancer cells, tumor growth inhibition, reduction of metastasis, *etc.*) against cancer cells expressing the target antigen for the CAR.

In some embodiments, CAR-expressing cell according to the present disclosure may display one or more of the following properties: cytotoxicity to a cell comprising/expressing the target antigen for the CAR; proliferation, IFNγ expression/secretion and/or IL-2 expression/secretion in response to stimulation with the target antigen for the CAR, or in response to exposure to a cell comprising/expressing the target antigen for the CAR; anti-cancer activity (*e.g.* cytotoxicity to cancer cells, tumor growth inhibition, reduction of metastasis, *etc.*) against cancer comprising cells expressing the target antigen for the CAR.

Cell proliferation/population expansion can be investigated by analysing cell division or the number of cells over a period of time. Cell division can be analysed, for example, by *in vitro* analysis of incorporation of ³H-thymidine or by CFSE dilution assay, *e.g.* as described in Fulcher and Wong, Immunol Cell Biol (1999) 77(6): 559-564, hereby incorporated by reference in entirety. Proliferating cells can also be identified by analysis of incorporation of 5-ethynyl-2'-deoxyuridine (EdU) by an appropriate assay, as described e.g. in Buck et al., Biotechniques. 2008 Jun; 44(7):927-9, and Sali and Mitchison, PNAS USA 2008 Feb 19; 105(7): 2415-2420, both hereby incorporated by reference in their entirety.

As used herein, 'expression' may be gene or protein expression. Gene expression encompasses transcription of DNA to RNA, and can be measured by various means known to those skilled in the art, for example by measuring levels of mRNA by quantitative real-time PCR (qRT-PCR), or by reporter-based methods. Similarly, protein expression can be measured by various methods well known in the art, *e.g.* by antibody-based methods, for example by western blot, immunohistochemistry, immunocytochemistry, flow cytometry, ELISA, ELISPOT, or reporter-based methods.

Cytotoxicity and cell killing can be investigated, for example, using any of the methods reviewed in Zaritskaya et al., Expert Rev Vaccines (2011), 9(6):601-616, hereby incorporated by reference in its entirety. Examples of *in vitro* assays of cytotoxicity/cell killing assays include release assays such as the ⁵¹Cr release assay, the lactate dehydrogenase (LDH) release assay, the 3-(4,5-dimethylthiazol-2-yl)-2,5-diphenyl tetrazolium bromide (MTT) release assay, and the calcein-acetoxymethyl (calcein-AM) release assay. These assays measure cell killing based on the detection of factors released from lysed cells. Cell killing of a given test cell type by a given effector immune cell type can be analysed *e.g.* by co-culturing the test cells with the effector immune cells, and measuring the number/proportion of viable/dead (*e.g.* lysed) test cells after a suitable period of time. Other suitable assays include the xCELLigence real-time cytolytic *in vitro* potency assay described in Cerignoli et al., PLoS One. (2018) 13(3): e0193498 (hereby incorporated by reference in its entirety). In some embodiments, cell killing of cells expressing the target antigen for the CAR by CAR-expressing cells may be evaluated through the assay described in Example 3 herein.

In some embodiments, cytokine production analysis may be evaluated through the assay described in Example 4 herein.

In some embodiments, the survival of subjects having a cancer may be evaluated based on methods in line with the experiments described in Example 4 herein.

Cells may be evaluated for anti-cancer activity by analysis in an appropriate *in vitro* assays or *in vivo* models of the relevant cancer.

Cells comprising a CAR/nucleic acid encoding a CAR according to the present disclosure preferably possess novel and/or improved properties compared to cells comprising/comprising nucleic acid encoding a known CAR, *e.g.* a CAR that does not comprise two interaction domains as defined herein.

In particular, the cells comprising a CAR/nucleic acid encoding a CAR according to the present disclosure preferably possess novel and/or improved properties compared to cells comprising a CAR/nucleic acid encoding an equivalent CAR construct identical in all respects, with the exception of the presence of an interaction domain defined herein.

In some embodiments, cells comprising a CAR/nucleic acid encoding a CAR according to the present disclosure:
kill cells expressing the target antigen for the CAR with potency/rate which is similar to, or greater than, the potency/rate of killing of such cells by cells comprising a reference CAR;
secrete cytokines (e.g., IL-2, and/or IFNy) in response to stimulation with cells expressing the target antigen for the CAR to an extent which is similar to, or greater than, the secretion level of cells comprising a reference CAR;
inhibit tumor growth, *e.g.* of a cancer expressing the target antigen for the CAR with a potency/to an extent which is similar to, or greater than, tumor growth inhibition by cells comprising a reference CAR;
increase survival of subjects having a cancer, *e.g.* a cancer expressing the target antigen for the CAR to an extent which is similar to, or greater than, the extent to which survival is increased by cells comprising a reference CAR;
display persistence *in vivo* which is similar to, or greater than, the *in vivo* persistence of cells comprising a reference CAR;
display off-target cytotoxicity which is similar to, or less than, the off-target cytotoxicity displayed by cells comprising a reference CAR;

The reference CAR may be a CAR which is identical to the CAR according to the present disclosure to which it is compared to, except for the presence of interactions domain defined herein. By way of illustration, the CAR construct of SEQ ID NO:9 is identical to that of SEQ ID NO:56, with the exception that the CAR of SEQ ID NO:9 comprises a first interaction domain according to SEQ ID NO:1 and a second interaction domain according to SEQ ID NO:2, whereas the CAR of SEQ ID NO:56 does not comprise an interaction domain according to the current invention.

In some embodiments, the level of secretion of one or more cytokines (*e.g.* IL-2, and/or IFNy) by cells comprising a CAR/nucleic acid encoding a CAR according to the present disclosure in response to stimulation with cells expressing the target antigen for the CAR is greater than 1 times, *e.g.* one of ≥1.01 times, ≥1.02 times, ≥1.03 times, ≥1.04 times, ≥1.05 times, ≥1.1 times, ≥1.2 times, ≥1.3 times, ≥1.4 times, ≥1.5 times, ≥1.6 times, ≥1.7 times, ≥1.8 times, ≥1.9 times, ≥2 times, ≥3 times, ≥4 times, ≥5 times, ≥6 times, ≥7 times, ≥8 times, ≥9 times or ≥10 times the level of expression of such factor(s) by cells comprising a reference CAR/nucleic acid encoding a reference CAR in response to stimulation with cells expressing the target antigen for the CAR, as determined in a given assay.

In some embodiments, the potency or rate of cell killing of cells expressing the target antigen for the CAR by cells comprising a CAR/nucleic acid encoding a CAR according to the present disclosure (*e.g. in vitro* or *in vivo*) is greater than 1 times, e.g. one of ≥1.01 times, ≥1.02 times, ≥1.03 times, ≥1.04 times, ≥1.05 times, ≥1.1 times, ≥1.2 times, ≥1.3 times, ≥1.4 times, ≥1.5 times, ≥1.6 times, ≥1.7 times, ≥1.8 times, ≥1.9 times, ≥2 times, ≥3 times, ≥4 times, ≥5 times, ≥6 times, ≥7 times, ≥8 times, ≥9 times or ≥10 times the potency or rate of cell killing of such cells by cells comprising a reference CAR/nucleic acid encoding a reference CAR, as determined in a given assay.

In some embodiments, the cells comprising a CAR/nucleic acid encoding a CAR according to the present disclosure induce cell killing of cells not expressing the target antigen for the CAR to an extent which is similar to or less than the extent to which cells comprising a reference CAR/nucleic acid encoding a reference CAR induce cell killing of cells not expressing the target antigen for the CAR.

In some embodiments, the cells comprising a CAR/nucleic acid encoding a CAR according to the present disclosure induce cell killing of cells not expressing the target antigen for the CAR (*e.g. in vitro* or *in vivo*) to a level which is less than 1 times, e.g. ≤0.99 times, ≤0.95 times, ≤0.9 times, ≤0.85 times, ≤0.8 times, ≤0.75 times, ≤0.7 times, ≤0.65 times, ≤0.6 times, 50.55 times, ≤0.5 times, ≤0.45 times, ≤0.4 times, ≤0.35 times, ≤0.3 times, ≤0.25 times, ≤0.2 times, ≤0.15 times, ≤0.1 times, ≤0.05 times, or ≤0.01 times the level of cell killing of such cells by cells comprising a reference CAR/nucleic acid encoding a reference CAR, as determined in a given assay.

In some embodiments, the level of inhibition of tumor growth (*e.g.* of a tumor expressing the target antigen for the CAR) achieved by cells comprising a CAR/nucleic acid encoding a CAR according to the present disclosure is greater than 1 times, *e.g.* one of ≥1.01 times, ≥1.02 times, ≥1.03 times, ≥1.04 times, ≥1.05 times, ≥1.1 times, ≥1.2 times, ≥1.3 times, ≥1.4 times, ≥1.5 times, ≥1.6 times, ≥1.7 times, ≥1.8 times, ≥1.9 times, ≥2 times, ≥3 times, ≥4 times, ≥5 times, ≥6 times, ≥7 times, ≥8 times, ≥9 times or ≥10 times the level achieved by administration of a comparable quantity of cells comprising a reference CAR/nucleic acid encoding a reference CAR, as determined in a given assay.

In some embodiments, administration of cells comprising a CAR/nucleic acid encoding a CAR according to the present disclosure increases survival of recipient subjects having a cancer expressing the target antigen for the CAR to greater than 1 times, e.g. one of ≥1.01 times, ≥1.02 times, ≥1.03 times, ≥1.04 times, ≥1.05 times, ≥1.1 times, ≥1.2 times, ≥1.3 times, ≥1.4 times, ≥1.5 times, ≥1.6 times, ≥1.7 times, ≥1.8 times, ≥1.9 times, ≥2 times, ≥3 times, ≥4 times, ≥5 times, ≥6 times, ≥7 times, ≥8 times, ≥9 times or ≥10 times the level of survival achieved by administration of a comparable quantity of cells comprising a reference CAR/nucleic acid encoding a reference CAR, as determined in a given assay.

In some embodiments, the *in vivo* persistence of cells comprising a CAR/nucleic acid encoding a CAR according to the present disclosure following administration to a subject (*e.g.* a subject having a cancer, *e.g.* a cancer expressing the target antigen for the CAR) is greater than 1 times, e.g. one of ≥1.01 times, ≥1.02 times, ≥1.03 times, ≥1.04 times, ≥1.05 times, ≥1.1 times, ≥1.2 times, ≥1.3 times, ≥1.4 times, ≥1.5 times, ≥1.6 times, ≥1.7 times, ≥1.8 times, ≥1.9 times, ≥2 times, ≥3 times, ≥4 times, ≥5 times, ≥6 times, ≥7 times, ≥8 times, ≥9 times or ≥10 times the *in vivo* persistence observed for cells comprising a reference CAR/nucleic acid encoding a reference CAR, as determined in a given assay.

### Producing cells expressing the CARs of the disclosure

The present disclosure provides methods for producing a cell comprising a nucleic acid(s) or vector(s) according to the present disclosure, comprising introducing a nucleic acid, a plurality of nucleic acids, a vector or a plurality of vectors according to the present disclosure into a cell. In some embodiments, introducing an isolated nucleic acid(s) or vector(s) according to the present disclosure into a cell comprises transformation, transfection, electroporation or transduction (*e.g.* retroviral transduction).

The present disclosure also provides methods for producing a cell expressing/comprising a CAR according to the present disclosure, comprising introducing a nucleic acid, a plurality of nucleic acids, a vector or a plurality of vectors according to the present disclosure in a cell. In some embodiments, the methods additionally comprise culturing the cell under conditions suitable for expression of the nucleic acid(s) or vector(s) by the cell. In some embodiments, the methods are performed *in vitro.*

Methods for producing CAR-expressing cells are well known to the skilled person. They generally involve modifying cells (*e.g.* immune cells, *e.g.* T cells or NK cells) to express/comprise a CAR, *e.g.* introducing nucleic acid encoding a CAR into the immune cells.

Immune cells may be modified to comprise/express a CAR or nucleic acid encoding a CAR described herein according to methods that are well known to the skilled person. The methods generally comprise nucleic acid transfer for permanent (stable) or transient expression of the transferred nucleic acid.

Any suitable genetic engineering platform may be used to modify a cell according to the present disclosure. Suitable methods for modifying a cell include the use of genetic engineering platforms such as gammaretroviral vectors, lentiviral vectors, adenovirus vectors, DNA transfection, transposon-based gene delivery and RNA transfection, for example as described in Maus et al., Annu Rev Immunol (2014) 32:189-225, hereby incorporated by reference in its entirety.

Methods also include those described *e.g.* in Wang and Rivière Mol Ther Oncolytics. (2016) 3:16015, which is hereby incorporated by reference in its entirety. Suitable methods for introducing nucleic acid(s)/vector(s) into cells include transduction, transfection and electroporation.

Methods for generating/expanding populations of CAR-expressing immune cells *in vitro*/*ex vivo* are well known to the skilled person. Suitable culture conditions (*i.e.* cell culture media, additives, stimulations, temperature, gaseous atmosphere), cell numbers, culture periods and methods for introducing nucleic acid encoding a CAR into cells, *etc.* can be determined by reference *e.g.* to Hombach et al. J Immunol (2001) 167:6123-6131, Ramos et al. J. Clin. Invest. (2017) 127(9):3462-3471 and WO 2015/028444 A1, all of which are hereby incorporated by reference in their entirety.

Conveniently, cultures of cells according to the present disclosure may be maintained at 37°C in a humidified atmosphere containing 5% CO₂. The cells of cell cultures can be established and/or maintained at any suitable density, as can readily be determined by the skilled person.

Cultures can be performed in any vessel suitable for the volume of the culture, *e.g.* in wells of a cell culture plate, cell culture flasks, a bioreactor, *etc.* In some embodiments cells are cultured in a bioreactor, *e.g.* a bioreactor described in Somerville and Dudley, Oncoimmunology (2012) 1(8):1435-1437, which is hereby incorporated by reference in its entirety. In some embodiments cells are cultured in a GRex cell culture vessel, *e.g.* a GRex flask or a GRex 100 bioreactor.

Immune cells (*e.g.* T cells) may be activated prior to introduction of nucleic acid encoding the CAR. For example, T cells within a population of PBMCs may be non-specifically activated by stimulation *in vitro* with agonist anti-CD3 and agonist anti-CD28 antibodies, in the presence of IL-2.

Introducing nucleic acid(s)/vector(s) into a cell may comprise transduction, *e.g.* retroviral transduction. Accordingly, in some embodiments the nucleic acid(s) is/are comprised in a viral vector(s), or the vector(s) is/are a viral vector(s). Transduction of immune cells with viral vectors is described *e.g.* in Simmons and Alberola-Ila, Methods Mol Biol. (2016) 1323:99-108, which is hereby incorporated by reference in its entirety.

Agents may be employed to enhance the efficiency of transduction. Hexadimethrine bromide (polybrene) is a cationic polymer which is commonly used to improve transduction, through neutralising charge repulsion between virions and sialic acid residues expressed on the cell surface. Other agents commonly used to enhance transduction include *e.g.* the poloxamer-based agents such as LentiBOOST (Sirion Biotech), Retronectin (Takara), Vectofusin (Miltenyi Biotech) and also SureENTRY (Qiagen) and ViraDuctin (Cell Biolabs).

In some embodiments the methods comprise centrifuging the cells into which it is desired to introduce nucleic acid encoding the CAR in the presence of cell culture medium comprising viral vector comprising the nucleic acid (referred to in the art as 'spinfection').

In some embodiments, the methods comprise introducing a nucleic acid or vector according to the present disclosure into an immune cell by electroporation, *e.g.* as described in Koh et al., Molecular Therapy - Nucleic Acids (2013) 2, e114, which is hereby incorporated by reference in its entirety.

The methods generally comprise introducing a nucleic acid encoding a CAR into a cell, and culturing the cell under conditions suitable for expression of the nucleic acid/CAR by the cell. In some embodiments, the methods comprise culturing immune cells into which nucleic acid encoding a CAR has been introduced in order to expand their number. In some embodiments, the methods comprise culturing immune cells into which nucleic acid encoding a CAR has been introduced in the presence of IL-7 and/or IL-15 (*e.g.* recombinant IL-7 and/or IL-15).

In some embodiments the methods further comprise purifying/isolating CAR-expressing cells, *e.g.* from other cells (*e.g.* cells which do not express the CAR). Methods for purifying/isolating immune cells from heterogeneous populations of cells are well known in the art, and may employ *e.g.* FACS- or MACS-based methods for sorting populations of cells based on the expression of markers of the immune cells.

In some embodiments the methods purifying/isolating cells of a particular type, *e.g.* CAR-expressing CD8+ T cells, CAR-expressing CTLs.

In preferred embodiments, CAR-expressing T cells may be generated from T cells within populations of PBMCs by a process comprising: stimulating PBMCs with antagonist anti-CD3 and andti-CD28 antibodies, transducing the cells with a viral vector (*e.g.* a gamma-retroviral vector) encoding the CAR, and subsequently culturing the cells in the presence of IL-7 and IL-15.

The present disclosure also provides CAR-expressing cells obtained or obtainable by the methods according to the present disclosure.

### Compositions

The present disclosure also provides compositions comprising the CARs, nucleic acids, expression vectors and cells described herein.

The cells, CARs, nucleic acids and expression vectors described herein may be formulated as pharmaceutical compositions or medicaments for clinical use and may comprise a pharmaceutically acceptable carrier, diluent, excipient or adjuvant.

The compositions of the present disclosure may comprise one or more pharmaceutically-acceptable carriers (*e.g.* liposomes, micelles, microspheres, nanoparticles), diluents/excipients (*e.g.* starch, cellulose, a cellulose derivative, a polyol, dextrose, maltodextrin, magnesium stearate), adjuvants, fillers, buffers, preservatives (*e.g.* vitamin A, vitamin E, vitamin C, retinyl palmitate, selenium, cysteine, methionine, citric acid, sodium citrate, methyl paraben, propyl paraben), anti-oxidants (*e.g.* vitamin A, vitamin E, vitamin C, retinyl palmitate, selenium), lubricants (*e.g.* magnesium stearate, talc, silica, stearic acid, vegetable stearin), binders (*e.g.* sucrose, lactose, starch, cellulose, gelatin, polyethylene glycol (PEG), polyvinylpyrrolidone (PVP), xylitol, sorbitol, mannitol), stabilisers, solubilisers, surfactants (*e.g.,* wetting agents), masking agents or colouring agents (*e.g.* titanium oxide).

The term 'pharmaceutically-acceptable' as used herein pertains to compounds, ingredients, materials, compositions, dosage forms, *etc.,* which are, within the scope of sound medical judgment, suitable for use in contact with the tissues of the subject in question (*e.g.* a human subject) without excessive toxicity, irritation, allergic response, or other problem or complication, commensurate with a reasonable benefit/risk ratio. Each carrier, diluent, excipient, adjuvant, filler, buffer, preservative, anti-oxidant, lubricant, binder, stabiliser, solubiliser, surfactant, masking agent, colouring agent, flavouring agent or sweetening agent of a composition according to the present disclosure must also be 'acceptable' in the sense of being compatible with the other ingredients of the formulation. Suitable carriers, diluents, excipients, adjuvants, fillers, buffers, preservatives, anti-oxidants, lubricants, binders, stabilisers, solubilisers, surfactants, masking agents, colouring agents, flavouring agents or sweetening agents can be found in standard pharmaceutical texts, for example, Remington's 'The Science and Practice of Pharmacy' (Ed. A. Adejare), 23rd Edition (2020), Academic Press.

Compositions may be formulated for topical, parenteral, systemic, intracavitary, intravenous, intra-arterial, intramuscular, intrathecal, intraocular, intraconjunctival, intratumoral, subcutaneous, intradermal, intrathecal, oral or transdermal routes of administration. In some embodiments, a pharmaceutical composition/medicament may be formulated for administration by injection or infusion, or administration by ingestion.

Suitable formulations may comprise the relevant article in a sterile or isotonic medium. Medicaments and pharmaceutical compositions may be formulated in fluid, including gel, form. Fluid formulations may be formulated for administration by injection or infusion (*e.g.* via catheter) to a selected region of the human or animal body.

In some embodiments, the composition is formulated for injection or infusion, *e.g.* into a blood vessel, tissue/organ of interest, or tumor.

The present disclosure also provides methods for the production of pharmaceutically useful compositions, such methods of production may comprise one or more steps selected from: producing a CAR or a cell comprising/expressing a CAR according to the present disclosure; isolating a CAR or a cell comprising/expressing a CAR according to the present disclosure; and/or mixing a CAR or a cell comprising/expressing a CAR according to the present disclosure with a pharmaceutically acceptable carrier, adjuvant, excipient or diluent.

For example, a further aspect the present disclosure relates to a method of formulating or producing a medicament or pharmaceutical composition for use in the treatment of a disease/condition (*e.g.* a cancer), the method comprising formulating a pharmaceutical composition or medicament by mixing a CAR or a cell comprising/expressing a CAR according to the present disclosure with a pharmaceutically acceptable carrier, adjuvant, excipient or diluent.

### Therapeutic and prophylactic applications

The CARs, nucleic acids, expression vectors, cells and compositions described herein find use in therapeutic and prophylactic methods.

The present disclosure provides a CAR, nucleic acid (or plurality thereof), expression vector (or plurality thereof), cell or composition described herein for use in a method of medical treatment or prophylaxis. Also provided is a CAR, nucleic acid (or plurality thereof), expression vector (or plurality thereof), cell or composition described herein for use in a method of treating or preventing a disease or condition described herein. Also provided is the use of a CAR, nucleic acid (or plurality thereof), expression vector (or plurality thereof), cell or composition described herein in the manufacture of a medicament for treating or preventing a disease or condition described herein. Also provided is a method of treating or preventing a disease or condition described herein, comprising administering to a subject a therapeutically or prophylactically effective amount of a CAR, nucleic acid (or plurality thereof), expression vector (or plurality thereof), cell or composition described herein.

The methods may be effective to reduce the development or progression of a disease/condition, alleviation of the symptoms of a disease/condition or reduction in the pathology of a disease/condition. The methods may be effective to prevent progression of the disease/condition, *e.g.* to prevent worsening of, or to slow the rate of development of, the disease/condition. In some embodiments, the methods may lead to an improvement in the disease/condition, *e.g.* a reduction in the symptoms of the disease/condition or reduction in some other correlate of the severity/activity of the disease/condition. In some embodiments, the methods may prevent development of the disease/condition a later stage (*e.g.* a chronic stage or metastasis).

It will be appreciated that the articles of the present disclosure may be used for the treatment/prevention of any disease/condition that would derive therapeutic or prophylactic benefit from a reduction in the level/activity of the target antigen for the CAR, or a reduction in the number or activity of cells comprising/expressing the target antigen for the CAR.

For example, the disease/condition may be a disease/condition in which the target antigen for the CAR, or cells comprising/expressing the target antigen for the CAR are pathologically-implicated, *e.g.* a disease/condition in which an increased level/activity of the target antigen for the CAR, or an increase in the number/proportion of cells comprising/expressing the target antigen for the CAR is positively associated with the onset, development or progression of the disease/condition, and/or severity of one or more symptoms of the disease/condition. In some embodiments, an increased level/activity of the target antigen for the CAR, or an increase in the number/proportion of cells comprising/expressing the target antigen for the CAR may be a risk factor for the onset, development or progression of the disease/condition.

In some embodiments, the disease/condition to be treated/prevented in accordance with the present disclosure is a disease/condition characterised by an increase in the level of expression or activity of the target antigen for the CAR (*e.g.* CD19 or BCMA), *e.g.* as compared to the level of expression/activity in the absence of the disease/condition. In some embodiments, the disease/condition to be treated/prevented is a disease/condition characterised by an increase in the number/proportion/activity of cells expressing the target antigen for the CAR, *e.g.* as compared to the level/number/proportion/activity in the absence of the disease/condition (*e.g.* in a healthy subject, or in equivalent non-diseased tissue). Where the disease/condition is a cancer, the level of expression or activity of the target antigen for the CAR may be greater than the level of expression or activity of the target antigen for the CAR in equivalent non-cancerous cells/non-tumor tissue. A cancer/cell thereof may comprise one or more mutations (*e.g.* relative to equivalent non-cancerous cells/non-tumor tissue) causing upregulation of expression or activity of the target antigen for the CAR.

Treatment in accordance with the methods of the present disclosure may achieve one or more of the following in a subject (compared to an equivalent untreated subject, or subject treated with an appropriate control): a reduction in the level of the target antigen; a reduction in the activity of the target antigen; and/or a reduction in the number/proportion of cells comprising/expressing the target antigen.

In aspects and embodiments according to the present disclosure, cells (particularly immune cells, more particularly T cells) comprising/expressing a CAR according to the present disclosure are provided for therapeutic and prophylactic use. It will be appreciated that the methods generally comprise administering a population of immune cells expressing a CAR according to the present disclosure to a subject. In some embodiments, immune cells expressing a CAR according to the present disclosure may be administered in the form of a pharmaceutical composition comprising such cells.

In particular, use of immune cells expressing a CAR according to the present disclosure in methods to treat/prevent diseases/conditions by adoptive cell transfer (ACT) is contemplated.

Adoptive cell transfer generally refers to a process by which cells (*e.g.* immune cells) are obtained from a subject, typically by drawing a blood sample from which the cells are isolated. The cells are then typically modified and/or expanded, and then administered either to the same subject (in the case of adoptive transfer of autologous/autogeneic cells) or to a different subject (in the case of adoptive transfer of allogeneic cells). The treatment is typically aimed at providing a population of cells with certain desired characteristics to a subject, or increasing the frequency of such cells with such characteristics in that subject. Adoptive transfer may be performed with the aim of introducing a cell or population of cells into a subject, and/or increasing the frequency of a cell or population of cells in a subject.

Adoptive transfer of immune cells is described, for example, in Kalos and June (2013), Immunity 39(1): 49-60, and Davis et al. (2015), Cancer J. 21(6): 486-491, both of which are hereby incorporated by reference in their entirety. The skilled person is able to determine appropriate reagents and procedures for adoptive transfer of cells according to the present disclosure, for example by reference to Dai et al., 2016 J Nat Cancer Inst 108(7): djv439, which is incorporated by reference in its entirety.

The immune cells expressing a CAR according to the present disclosure may be employed in the treatment/prevention of diseases/conditions by allotransplantation or autotransplantation.

As used herein, 'allotransplantation' refers to the transplantation to a recipient subject of cells, tissues or organs which are genetically non-identical to the recipient subject. The cells, tissues or organs may be from, or may be derived from, cells, tissues or organs of a donor subject that is genetically non-identical to the recipient subject. Allotransplantation is distinct from autotransplantation, which refers to the transplantation of cells, tissues or organs which are from/derived from a donor subject genetically identical to the recipient subject (*i.e.* autologous material). It will be appreciated that adoptive transfer of allogeneic immune cells is a form of allotransplantation, and that adoptive transfer of autologous immune cells is a form of autotransplantation.

The present disclosure provides methods comprising administering immune cells comprising/expressing a CAR according to the present disclosure, or immune cells comprising/expressing nucleic acid encoding a CAR according to the present disclosure, to a subject.

In some embodiments, the methods comprise modifying an immune cell to comprise/express a CAR according to the present disclosure. In some embodiments, the methods comprise modifying an immune cell specific for a virus to comprise/express nucleic acid encoding a CAR according to the present disclosure.

In some embodiments, the methods comprise:
(a) modifying an immune cell to express or comprise a CAR according to the present disclosure, or to express or comprise nucleic acid encoding a CAR according to the present disclosure, and
(b) administering the immune cell specific for a virus modified to express or comprise a CAR according to the present disclosure, or modified to express or comprise a nucleic acid encoding a CAR according to the present disclosure, to a subject.

In some embodiments, the methods comprise:
(a) isolating or obtaining immune cells;
(b) modifying an immune cell to express or comprise a CAR according to the present disclosure, or to express or comprise nucleic acid encoding a CAR according to the present disclosure, and
(c) administering the immune cell modified to express or comprise a CAR according to the present disclosure, or modified to express or comprise a nucleic acid encoding a CAR according to the present disclosure, to a subject.

In some embodiments, the subject from which the immune cells (e.g. PBMCs) are isolated is the same subject to which cells are administered (i.e., adoptive transfer may be of autologous/autogeneic cells). In some embodiments, the subject from which the immune cells (e.g. PBMCs) are isolated is a different subject to the subject to which cells are administered (i.e., adoptive transfer may be of allogeneic cells).

In some embodiments the methods may comprise one or more of:
obtaining a blood sample from a subject;
isolating immune cells (e.g. PBMCs) from a blood sample which has been obtained from a subject; generating/expanding a population of immune cells;
culturing the immune cells in *in vitro* or *ex vivo* cell culture;
modifying an immune cell to express or comprise a CAR according to the present disclosure, or to express or comprise a nucleic acid encoding a CAR according to the present disclosure (e.g. by transduction with a viral vector encoding such CAR, or a viral vector comprising such nucleic acid); culturing immune cells expressing/comprising a CAR according to the present disclosure, or expressing/comprising a nucleic acid encoding a CAR according to the present disclosure in *in vitro* or *ex vivo* cell culture;
collecting/isolating immune cells expressing/comprising a CAR according to the present disclosure, or expressing/comprising a nucleic acid encoding a CAR according to the present disclosure;
formulating immune cells expressing/comprising a CAR according to the present disclosure, or a nucleic acid encoding a CAR according to the present disclosure to a pharmaceutical composition, *e.g.* by mixing the cells with a pharmaceutically acceptable adjuvant, diluent, or carrier;
administering immune cells expressing/comprising a CAR according to the present disclosure, or expressing/comprising a nucleic acid encoding a CAR according to the present disclosure, or a pharmaceutical composition comprising such cells, to a subject.

In some embodiments, the methods may additionally comprise treating the cells or subject to induce/enhance expression of CAR.

In some embodiments, the disease to be treated/prevented in accordance with the present disclosure is a cancer.

Cancer may refer to any unwanted cell proliferation (or any disease manifesting itself by unwanted cell proliferation), neoplasm or tumor. The cancer may be benign or malignant and may be primary or secondary (metastatic). A neoplasm or tumor may be any abnormal growth or proliferation of cells and may be located in any tissue. The cancer may be of tissues/cells derived from *e.g.* the adrenal gland, adrenal medulla, anus, appendix, bladder, blood, bone, bone marrow, brain, breast, cecum, central nervous system (including or excluding the brain) cerebellum, cervix, colon, duodenum, endometrium, epithelial cells (*e.g.* renal epithelia), gallbladder, oesophagus, glial cells, heart, ileum, jejunum, kidney, lacrimal glad, larynx, liver, lung, lymph, lymph node, lymphoblast, maxilla, mediastinum, mesentery, myometrium, nasopharynx, omentum, oral cavity, ovary, pancreas, parotid gland, peripheral nervous system, peritoneum, pleura, prostate, salivary gland, sigmoid colon, skin, small intestine, soft tissues, spleen, stomach, testis, thymus, thyroid gland, tongue, tonsil, trachea, uterus, vulva, and/or white blood cells.

Tumors may be nervous or non-nervous system tumors. Nervous system tumors may originate either in the central or peripheral nervous system, *e.g.* glioma, medulloblastoma, meningioma, neurofibroma, ependymoma, Schwannoma, neurofibrosarcoma, astrocytoma and oligodendroglioma. Non-nervous system cancers/tumors may originate in any other non-nervous tissue, examples include melanoma, mesothelioma, lymphoma, myeloma, leukemia, Non-Hodgkin's lymphoma (NHL), Hodgkin's lymphoma, chronic myelogenous leukemia (CML), acute myeloid leukemia (AML), myelodysplastic syndrome (MDS), cutaneous T cell lymphoma (CTCL), chronic lymphocytic leukemia (CLL), hepatoma, epidermoid carcinoma, prostate carcinoma, breast cancer, lung cancer , colon cancer, ovarian cancer, pancreatic cancer, thymic carcinoma, NSCLC, hematologic cancer and sarcoma.

In some embodiments the cancer is selected from the group consisting of: a solid cancer, a hematological cancer, gastric cancer (*e.g.* gastric carcinoma, gastric adenocarcinoma, gastrointestinal adenocarcinoma), liver cancer (hepatocellular carcinoma, cholangiocarcinoma), head and neck cancer (*e.g.* head and neck squamous cell carcinoma), oral cavity cancer (*e.g.* oropharyngeal cancer (*e.g.* oropharyngeal carcinoma), oral cancer, laryngeal cancer, nasopharyngeal carcinoma, oesophageal cancer), colorectal cancer (*e.g.* colorectal carcinoma), colon cancer, colon carcinoma, cervical carcinoma, prostate cancer, lung cancer (*e.g.* NSCLC, small cell lung cancer, lung adenocarcinoma, squamous lung cell carcinoma), bladder cancer, urothelial carcinoma, skin cancer (*e.g.* melanoma, advanced melanoma), renal cell cancer (*e.g.* renal cell carcinoma), ovarian cancer (*e.g.* ovarian carcinoma), mesothelioma, breast cancer, brain cancer (*e.g.* glioblastoma), prostate cancer, pancreatic cancer, a myeloid hematologic malignancy, a lymphoblastic hematologic malignancy, myelodysplastic syndrome (MDS), acute myeloid leukemia (AML), chronic myeloid leukemia (CML), acute lymphoblastic leukemia (ALL), lymphoma, non-Hodgkin's lymphoma (NHL), thymoma or multiple myeloma (MM).

In some embodiments, the cancer is a cancer in which the target antigen for the CAR (*e.g.,* CD19 or BCMA) is pathologically implicated. That is, in some embodiments the cancer is a cancer which is caused or exacerbated by the expression of the target antigen for the CAR, a cancer for which expression of the target antigen for the CAR is a risk factor and/or a cancer for which expression of the target antigen for the CAR is positively associated with onset, development, progression, severity or metastasis of the cancer. The cancer may be characterised by expression of the target antigen for the CAR, *e.g.* the cancer may comprise cells expressing the target antigen for the CAR. Such cancers may be referred to as being positive for the target antigen for the CAR. A cancer which is 'positive' for the target antigen for the CAR may be a cancer comprising cells expressing the target antigen for the CAR (*e.g.* at the cell surface). A cancer which is 'positive' for the target antigen for the CAR may overexpress the target antigen for the CAR. In some embodiments, the cancer is a CD19-positive cancer. In some embodiments, the cancer is a BCMA-positive cancer.

In some embodiments, the cancer may be a relapsed cancer. As used herein, a 'relapsed' cancer refers to a cancer which responded to a treatment (*e.g.* a first line therapy for the cancer), but which has subsequently re-emerged/progressed, *e.g.* after a period of remission. For example, a relapsed cancer may be a cancer whose growth/progression was inhibited by a treatment (*e.g.* a first line therapy for the cancer), and which has subsequently grown/progressed.

In some embodiments, the cancer may be a refractory cancer. As used herein, a 'refractory' cancer refers to a cancer which has not responded to a treatment (*e.g.* a first line therapy for the cancer). For example, a refractory cancer may be a cancer whose growth/progression was not inhibited by a treatment (*e.g.* a first line therapy for the cancer). In some embodiments a refractory cancer may be a cancer for which a subject receiving treatment for the cancer did not display a partial or complete response to the treatment.

In embodiments where the cancer is anaplastic large cell lymphoma, the cancer may be relapsed or refractory with respect to treatment with chemotherapy, brentuximab vedotin, or crizotinib. In embodiments where the cancer is peripheral T cell lymphoma, the cancer may be relapsed or refractory with respect to treatment with chemotherapy or brentuximab vedotin. In embodiments where the cancer is extranodal NK-T cell lymphoma, the cancer may be relapsed or refractory with respect to treatment with chemotherapy (with or without asparaginase) or brentuximab vedotin. In embodiments where the cancer is diffuse large B cell lymphoma, the cancer may be relapsed or refractory with respect to treatment with chemotherapy (with or without rituximab) or CD19 CAR-T therapy. In embodiments where the cancer is primary mediastinal B cell lymphoma, the cancer may be relapsed or refractory with respect to treatment with chemotherapy, or immune checkpoint inhibitor (*e.g.* PD-1 inhibitor).

Treatment of a cancer in accordance with the methods of the present disclosure achieves one or more of the following treatment effects: reduces the number of cancer cells in the subject, reduces the size of a cancerous tumor/lesion in the subject, inhibits (*e.g.* prevents or slows) growth of cancer cells in the subject, inhibits (*e.g.* prevents or slows) growth of a cancerous tumor/lesion in the subject, inhibits (*e.g.* prevents or slows) the development/progression of a cancer (*e.g.* to a later stage, or metastasis), reduces the severity of symptoms of a cancer in the subject, increases survival of the subject (*e.g.* progression free survival or overall survival), reduces a correlate of the number or activity of cancer cells in the subject, and/or reduces cancer burden in the subject.

Subjects may be evaluated in accordance with the Revised Criteria for Response Assessment: The Lugano Classification (described *e.g.* in Cheson et al., J Clin Oncol (2014) 32: 3059-3068, incorporated by reference hereinabove) in order to determine their response to treatment. In some embodiments, treatment of a subject in accordance with the methods of the present disclosure achieves one of the following: complete response, partial response, or stable disease.

CAR T cell therapy have shown excellent results for the treatment of cancer, but their application is not limited to the treatment of cancer. evidence from both clinical and preclinical investigations emphasize the broader potential of CAR T therapy, extending beyond oncology to address autoimmune disorders, persistent infections, cardiac fibrosis, age-related ailments and other conditions (Yang et al., Life Sciences. Volume 344, 1 May 2024, 122556).

In some embodiments, the disease/condition to be treated/prevented in accordance with the present disclosure is an autoimmune disease/disorder.

In some embodiments, the disease/condition to be treated/prevented in accordance with the present disclosure is an autoimmune disease. In some embodiments, the autoimmune disease is systemic lupus erythematosus (SLE), type 1 diabetes, Crohn's disease, Sjögren's syndrome, rheumatoid arthritis (RA), multiple sclerosis (MS), alopecia areata, psoriasis, psoriatic arthritis, myasthenia gravis, sarcoidosis, ulcerative colitis, Addison's disease, Graves' disease, Hashimoto's thyroiditis, Autoimmune vasculitis, Pernicious anemia, and/or Celiac disease.

Autoimmune diseases develop when the auto-reactive B lymphocytes (autoantibodies) and T lymphocytes cause pathological and/or functional damage to the organ/tissue containing the target autoantigen(s).

In some aspects and embodiments, the disease/condition to be treated/prevented in accordance with the present disclosure may be lymphocytopenia, or a disease/condition characterised by lymphocytopenia. Lymphocytopenia may be defined as a total lymphocyte count of < 1000/mcL (1 x 109/L) in adults or < 3000/mcL (< 3 x 109/L) in children < 2 years. Disease/conditions characterised by lymphocytopenia include e.g.: T lymphocytopenia, B lymphocytopenia, NK lymphocytopenia, idiopathic CD4+ lymphocytopenia, Human immunodeficiency virus infection and acquired immunodeficiency syndrome (HIV/AIDS), COVID-19, systemic lupus erythematosus (SLE), rheumatoid arthritis (RA), myasthenia gravis, sarcoidosis, multiple sclerosis (MS), chemotherapy-associated lymphocytopenia, severe combined immunodeficiency (SCID), Omenn syndrome, Wiskott-Aldrich syndrome and cartilage-hair hypoplasia (CHH).

In some aspects and embodiments, the disease/condition to be treated/prevented in accordance with the present disclosure may be a T cell dysfunctional disorder, a cancer, an infectious disease or an autoimmune disease.

A T cell dysfunctional disorder may be a disease/condition in which normal T cell function is impaired causing downregulation of the subject's immune response to pathogenic antigens, *e.g.* generated by infection by exogenous agents such as microorganisms, bacteria and viruses, or generated by the host in some disease states such as in some forms of cancer (*e.g.* in the form of tumor-associated antigens). The T cell dysfunctional disorder may comprise T cell exhaustion or T cell anergy. T cell exhaustion comprises a state in which CD8+ T cells fail to proliferate or exert T cell effector functions such as cytotoxicity and cytokine (*e.g.* IFNy) secretion in response to antigen stimulation. Exhausted T cells may also be characterised by sustained expression of one or more markers of T cell exhaustion, *e.g.* PD-1, CTLA-4, LAG-3, TIM-3. The T cell dysfunctional disorder may manifest as an infection, or inability to mount an effective immune response against an infection. The infection may be chronic, persistent, latent or slow, and may be the result of bacterial, viral, fungal or parasitic infection. As such, treatment may be provided to patients having a bacterial, viral or fungal infection. Examples of bacterial infections include infection with Helicobacter pylori. Examples of viral infections include infection with HIV, hepatitis B or hepatitis C. The T cell dysfunctional disorder may be associated with a cancer, such as tumor immune escape. Many human tumors express tumor-associated antigens recognised by T cells and capable of inducing an immune response.

An infectious disease may be *e.g.* bacterial, viral, fungal, or parasitic infection. In some embodiments, it may be particularly desirable to treat chronic/persistent infections, *e.g.* where such infections are associated with T cell dysfunction or T cell exhaustion. It is well established that T cell exhaustion is a state of T cell dysfunction that arises during many chronic infections (including viral, bacterial and parasitic), as well as in cancer (Wherry Nature Immunology Vol.12, No.6, p492-499, June 2011). Examples of bacterial infections that may be treated include infection by Bacillus spp., Bordetella pertussis, Clostridium spp., Corynebacterium spp., Vibrio chloerae, Staphylococcus spp., Streptococcus spp. Escherichia, Klebsiella, Proteus, Yersinia, Erwina, Salmonella, Listeria sp, Helicobacter pylori, mycobacteria (*e.g.* Mycobacterium tuberculosis) and Pseudomonas aeruginosa. For example, the bacterial infection may be sepsis or tuberculosis. Examples of viral infections that may be treated include infection by influenza virus, measles virus, hepatitis B virus (HBV), hepatitis C virus (HCV), human immunodeficiency virus (HIV), lymphocytic choriomeningitis virus (LCMV), Herpes simplex virus and human papilloma virus (HPV). Examples of fungal infections that may be treated include infection by Alternaria sp, Aspergillus sp, Candida sp and Histoplasma sp. The fungal infection may be fungal sepsis or histoplasmosis. Examples of parasitic infections that may be treated include infection by Plasmodium species (*e.g.* Plasmodium falciparum, Plasmodium yoeli, Plasmodium ovale, Plasmodium vivax, or Plasmodium chabaudi chabaudi). The parasitic infection may be a disease such as malaria, leishmaniasis and toxoplasmosis.

In some embodiments, the disease/condition is an autoimmune disease. In such embodiments the treatment may be aimed at reducing the number of autoimmune effector cells. In some embodiments the autoimmune disease is selected from: diabetes mellitus type 1, celiac disease, Graves' disease, inflammatory bowel disease, multiple sclerosis, psoriasis, rheumatoid arthritis, and systemic lupus erythematosus.

The CAR-expressing immune cells and compositions of the present disclosure are also useful for the treatment/prevention of an alloreactive immune response, and diseases/conditions characterised by an alloreactive immune response.

Diseases and conditions characterised by an alloreactive immune response include diseases/conditions caused or exacerbated by alloreactive immune responses associated with allotransplantation. Such diseases/conditions include graft versus host disease (GVHD) and graft rejection, and are described in detail in Perkey and Maillard Annu Rev Pathol. (2018) 13:219-245, which is hereby incorporated by reference in its entirety.

Graft-versus-host disease (GVHD) can occur following allotransplantation of large numbers of donor immune cells, and involves reactivity of donor-derived immune cells against allogeneic recipient cells/tissues/organs. Graft rejection refers to the destruction of transplanted cells/tissue/organs by a recipient's immune system following transplantation. Where graft rejection is of an allotransplant, it may be referred to as allograft rejection.

The CAR-expressing immune cells and compositions of the present disclosure may be used to deplete alloreactive T cells in an allotransplant, which could otherwise lead to graft versus host disease (GVHD) in a recipient upon allotransplantation.

The CAR-expressing immune cells and compositions of the present disclosure may be used to deplete alloreactive T cells in a donor for an allotransplant (*e.g.* prior to harvesting/collecting the allotransplant), which could otherwise lead to GVHD in a recipient upon allotransplantation.

The CAR-expressing immune cells and compositions of the present disclosure may be used to deplete alloreactive T cells in the recipient for an allotransplant, which could otherwise cause/promote graft rejection.

The present disclosure provides methods of treating/preventing graft-versus-host disease (GVHD) following allotransplantation, comprising administering a CAR-expressing immune cell or composition according to the present disclosure to a donor subject for an allotransplant. The present disclosure also provides methods of treating/preventing graft-versus-host disease (GVHD) following allotransplantation, comprising contacting an allotransplant with a CAR-expressing immune cell or composition according to the present disclosure. The aim for such methods is to reduce/remove the ability of alloreactive immune cells in the allograft to mount an alloreactive immune response to cells, tissue and/or organs of the recipient for the allotransplant.

The present disclosure provides methods of treating/preventing graft rejection following allotransplantation, comprising administering a CAR-expressing immune cell or composition according to the present disclosure to a recipient subject for an allotransplant. The aim for such methods is to reduce/remove the ability of the receipt subject to mount an alloreactive immune response to the allotransplant. The CAR-expressing immune cells are useful to eliminate immune cells in the recipient that would otherwise effect an alloreactive immune response against donor cells, tissue and/or organs.

The present disclosure provides methods comprising depleting an allotransplant of alloreactive immune cells (*e.g.* alloreactive T cells), comprising contacting an allotransplant (*e.g.* a population of cells, tissue or an organ to be transplanted) with a CAR-expressing immune cell or composition of the present disclosure. The methods may comprise administering a CAR-expressing immune cell or composition of the present disclosure to a donor subject for the allotransplant. The aim for such methods is to reduce/remove the ability of alloreactive immune cells in the allograft to mount an alloreactive immune response to cells, tissue and/or organs of the recipient for the allotransplant.

In some embodiments the methods comprise one or more of:
administering a CAR-expressing immune cell or composition according to the present disclosure to a subject, in order to deplete alloreactive immune cells in the subject;
obtaining/collecting a population of cells, tissue or organ from a subject to which a CAR-expressing immune cell or composition according to the present disclosure has been administered; and transplanting/administering a population of cells, tissue or organ depleted of alloreactive immune cells to a subject.

In some embodiments the methods comprise one or more of:
administering a CAR-expressing immune cell or composition according to the present disclosure to a subject, in order to deplete alloreactive immune cells in the subject; and
transplanting/administering a population of cells, tissue or organ to a subject to which a CAR-expressing immune cell or composition according to the present disclosure have previously been administered.

Depletion of alloreactive immune cells may result in *e.g.* a 2-fold, 10-fold, 100-fold, 1000-fold, 10000-fold or greater reduction in the quantity of alloreactive immune cells in the allotransplant or subject.

The methods may be performed *in vitro* or ex *vivo,* or *in vivo* in a subject. Method steps performed *in vitro* or *ex vivo* may comprise *in vitro* or *ex vivo* cell culture.

The methods may further comprise method steps for the production of CAR-expressing immune cells and compositions according to the present disclosure.

In some embodiments, administration of a CAR-expressing immune cell or composition according to the present disclosure to a recipient subject for an allotransplantation and allotransplantation are performed simultaneously (*i.e.* at the same time, or within *e.g.* 1 hr, 2 hrs, 3 hrs, 4 hrs, 5 hrs, 6 hrs, 8 hrs, 12 hrs, 24 hrs, 36 hrs or 48 hrs).

In some embodiments, administration of a CAR-expressing immune cell or composition according to the present disclosure to a recipient subject for an allotransplantation and allotransplantation are performed sequentially. The time interval between administration of a CAR-expressing immune cell or composition and allotransplantation may be any time interval, including hours, days, weeks, months, or years. The CAR-expressing immune cell or composition may be administered to the recipient subject before or after allotransplantation. The CAR-expressing immune cell or composition are preferably administered to the recipient subject prior to allotransplantation.

In some embodiments, administration of a CAR-expressing immune cell or composition according to the present disclosure to a donor subject for an allotransplantation and collection of the allotransplant (*i.e.* collection of the cells, tissue and/or an organ) from the subject are performed simultaneously (*i.e.* at the same time, or within *e.g.* 1 hr, 2 hrs, 3 hrs, 4 hrs, 5 hrs, 6 hrs, 8 hrs, 12 hrs, 24 hrs, 36 hrs or 48 hrs). In some embodiments administration of a CAR-expressing immune cell or composition according to the present disclosure to a donor subject for an allotransplantation and collection of the allotransplant (*i.e.* collection of the cells, tissue and/or an organ) from the subject are performed sequentially. The time interval between administration of a CAR-expressing immune cell or composition and collection of the allotransplant may be any time interval, including hours, days, weeks, months, or years. The CAR-expressing immune cell or composition may be administered to the donor subject before or after collection of the allotransplant. The CAR-expressing immune cell or composition are preferably administered to the donor subject prior to collection of the allotransplant.

In some embodiments, the methods comprise additional intervention to treat/prevent an alloreactive immune response, graft rejection and/or GVHD.

In some embodiments, the methods to treat/prevent alloreactivity, graft rejection and/or GVHD comprise administration of immunosuppressive and/or lymphodepletive therapy such as treatment with corticosteroids (*e.g.* prednisolone, hydrocortisone), calcineurin inhibitors (*e.g.* cyclosporin, tacrolimus) anti-proliferative agents (*e.g.* azathioprinem, mycophenolic acid) and/or mTOR inhibitors (*e.g.* sirolimus, everolimus).

In some embodiments, the methods to treat/prevent alloreactivity and/or graft rejection comprise antibody therapy, such as treatment with monoclonal anti-lL-2Rα receptor antibodies (*e.g.* basiliximab, daclizumab), anti-T cell antibodies (*e.g.* anti-thymocyte globulin, anti-lymphocyte globulin) and/or anti-CD20 antibodies (*e.g.* rituximab).

In some embodiments, the methods to treat/prevent alloreactivity and/or graft rejection comprise blood transfusion and/or bone marrow transplantation.

Where a method is disclosed herein, the present disclosure also provides the CAR-expressing immune cells and compositions of the present disclosure for use in such methods. Also provided is the use of the CAR-expressing immune cells or compositions of the present disclosure in the manufacture of products (*e.g.* medicaments) for use in such methods.

In some embodiments, the methods of various aspects of the present disclosure cause less depletion and/or increased survival of non-alloreactive immune cells as compared to methods employing immunosuppressive agent(s). For example, the present methods are useful for preserving/maintaining the non-alloreactive immune cell compartment in a recipient subject for an allotransplant, or in an allotransplant.

In some embodiments of the methods of the present disclosure comprising allotransplantation, the present methods are associated with an increased number/proportion of non-alloreactive immune cells in the recipient subject for the allotransplant as compared to methods involving treatment with an immunosuppressive agent. In some embodiments of the methods of the present disclosure comprising adoptive transfer of allogeneic immune cells, the present methods are associated with an increased number/proportion of non-alloreactive immune cells in the recipient subject for the allogeneic immune cells as compared to methods involving treatment with an immunosuppressive agent.

In some embodiments of the methods of the present disclosure comprising allotransplantation, the present methods are associated with an increased number/proportion of non-alloreactive immune cells in the allotransplant as compared to methods involving treatment with an immunosuppressive agent.

The present disclosure also provides the CAR-expressing immune cell or composition of the present disclosure for use in a method of:
killing a cell expressing the target antigen for which the CAR is specific; and/or
killing an alloreactive immune cell (*e.g.* a T cell a target antigen).

The present disclosure also provides the use of such CAR-expressing immune cells and compositions in such methods, and methods using the CAR-expressing immune cell and compositions to such ends.

### Administration

Administration of the articles of the present disclosure is preferably in a 'therapeutically-effective' or 'prophylactically-effective' amount, this being sufficient to show therapeutic or prophylactic benefit to the subject. The actual amount administered, and rate and time-course of administration, will depend on the nature and severity of the disease/condition and the particular article administered. Prescription of treatment, *e.g.* decisions on dosage *etc.,* is within the responsibility of general practitioners and other medical doctors, and typically takes account of the disease/disorder to be treated, the condition of the individual subject, the site of delivery, the method of administration and other factors known to practitioners. Examples of the techniques and protocols mentioned above can be found in Remington's 'The Science and Practice of Pharmacy' (ed. A. Adejare), 23rd Edition (2020), Academic Press.

Administration of the articles of the present disclosure may be topical, parenteral, systemic, intracavitary, intravenous, intra-arterial, intramuscular, intrathecal, intraocular, intravitreal, intraconjunctival, subretinal, suprachoroidal, subcutaneous, intradermal, intrathecal, oral, nasal or transdermal. Administration may be by injection or infusion. Administration of the articles of the present disclosure may be intratumoral.

In some aspects and embodiments in accordance with the present disclosure there may be targeted delivery of articles of the present disclosure, *i.e.* wherein the concentration of the relevant agent in the subject is increased in some parts of the body relative to other parts of the body. In some embodiments, the methods comprise intravenous, intra-arterial, intramuscular or subcutaneous administration and wherein the relevant article is formulated in a targeted agent delivery system. Suitable targeted delivery systems include, for example, nanoparticles, liposomes, micelles, beads, polymers, metal particles, dendrimers, antibodies, aptamers, nanotubes or micro-sized silica rods. Such systems may comprise a magnetic element to direct the agent to the desired organ or tissue. Suitable nanocarriers and delivery systems will be apparent to one skilled in the art.

In some cases, the articles of the present disclosure are formulated for targeted delivery to specific cells, a tissue, an organ and/or a tumor.

### Further intervention

Administration may be alone or in combination with other treatments, either simultaneously or sequentially dependent upon the disease/condition to be treated. The antigen-binding molecule, CAR, cell or composition described herein and another prophylactic/therapeutic agent may be administered simultaneously or sequentially.

In some embodiments, the methods comprise additional therapeutic or prophylactic intervention, *e.g.* for the treatment/prevention of a cancer. In some embodiments, the therapeutic or prophylactic intervention is selected from chemotherapy, immunotherapy, radiotherapy, surgery, vaccination and/or hormone therapy. In some embodiments, the therapeutic or prophylactic intervention comprises leukapheresis. In some embodiments, the therapeutic or prophylactic intervention comprises a stem cell transplant.

Simultaneous administration refers to administration of the antigen-binding molecule, polypeptide, CAR, nucleic acid (or plurality thereof), expression vector (or plurality thereof), cell or composition and therapeutic agent together, for example as a pharmaceutical composition containing both agents (combined preparation), or immediately after each other and optionally via the same route of administration, *e.g.* to the same artery, vein or other blood vessel. Sequential administration refers to administration of one of the antigen-binding molecule/composition or therapeutic agent followed after a given time interval by separate administration of the other agent. It is not required that the two agents are administered by the same route, although this is the case in some embodiments. The time interval may be any time interval.

In some embodiments, treatment of cancer further comprises chemotherapy and/or radiotherapy. Chemotherapy and radiotherapy respectively refer to treatment of a cancer with a drug or with ionising radiation (*e.g.* radiotherapy using X-rays or γ-rays). The drug may be a chemical entity, *e.g.* small molecule pharmaceutical, antibiotic, DNA intercalator, protein inhibitor (*e.g.* kinase inhibitor), or a biological agent, *e.g.* antibody, antibody fragment, aptamer, nucleic acid (*e.g*. DNA, RNA), peptide, polypeptide, or protein. The drug may be formulated as a pharmaceutical composition or medicament. The formulation may comprise one or more drugs (*e.g*. one or more active agents) together with one or more pharmaceutically acceptable diluents, excipients or carriers.

Chemotherapy may involve administration of more than one drug. A drug may be administered alone or in combination with other treatments, either simultaneously or sequentially dependent upon the condition to be treated.

The chemotherapy may be administered by one or more routes of administration, *e.g.* parenteral, intravenous injection, oral, subcutaneous, intradermal or intratumoral.

The chemotherapy may be administered according to a treatment regime. The treatment regime may be a pre-determined timetable, plan, scheme or schedule of chemotherapy administration which may be prepared by a physician or medical practitioner and may be tailored to suit the patient requiring treatment. The treatment regime may indicate one or more of: the type of chemotherapy to administer to the patient; the dose of each drug or radiation; the time interval between administrations; the length of each treatment; the number and nature of any treatment holidays, if any *etc.* For a co-therapy a single treatment regime may be provided which indicates how each drug is to be administered.

Chemotherapeutic drugs may be selected from: Abemaciclib, Abiraterone Acetate, Abitrexate (Methotrexate), Abraxane (Paclitaxel Albumin-stabilized Nanoparticle Formulation), ABVD, ABVE, ABVE-PC, AC, Acalabrutinib, AC-T, Adcetris (Brentuximab Vedotin), ADE, Ado-Trastuzumab Emtansine, Adriamycin (Doxorubicin Hydrochloride), Afatinib Dimaleate, Afinitor (Everolimus), Akynzeo (Netupitant and Palonosetron Hydrochloride), Aldara (Imiquimod), Aldesleukin, Alecensa (Alectinib), Alectinib, Alemtuzumab, Alimta (Pemetrexed Disodium), Aliqopa (Copanlisib Hydrochloride), Alkeran for Injection (Melphalan Hydrochloride), Alkeran Tablets (Melphalan), Aloxi (Palonosetron Hydrochloride), Alunbrig (Brigatinib), Ambochlorin (Chlorambucil), Amboclorin (Chlorambucil), Amifostine, Aminolevulinic Acid, Anastrozole, Aprepitant, Aredia (Pamidronate Disodium), Arimidex (Anastrozole), Aromasin (Exemestane), Arranon (Nelarabine), Arsenic Trioxide, Arzerra (Ofatumumab), Asparaginase Erwinia chrysanthemi, Atezolizumab, Avastin (Bevacizumab), Avelumab, Axicabtagene Ciloleucel, Axitinib, Azacitidine, Bavencio (Avelumab), BEACOPP, Becenum (Carmustine), Beleodaq (Belinostat), Belinostat, Bendamustine Hydrochloride, BEP, Besponsa (Inotuzumab Ozogamicin) , Bevacizumab, Bexarotene, Bexxar (Tositumomab and Iodine I 131 Tositumomab), Bicalutamide, BiCNU (Carmustine), Bleomycin, Blinatumomab, Blincyto (Blinatumomab), Bortezomib, Bosulif (Bosutinib), Bosutinib, Brentuximab Vedotin, Brigatinib, BuMel, Busulfan, Busulfex (Busulfan), Cabazitaxel, Cabometyx (Cabozantinib-S-Malate), Cabozantinib-S-Malate, CAF, Calquence (Acalabrutinib), Campath (Alemtuzumab), Camptosar (Irinotecan Hydrochloride), Capecitabine, CAPOX, Carac (Fluorouracil--Topical), Carboplatin, CARBOPLATIN-TAXOL, Carfilzomib, Carmubris (Carmustine), Carmustine, Carmustine Implant, Casodex (Bicalutamide), CEM, Ceritinib, Cerubidine (Daunorubicin Hydrochloride), Cervarix (Recombinant HPV Bivalent Vaccine), Cetuximab, CEV, Chlorambucil, CHLORAMBUCIL-PREDNISONE, CHOP, Cisplatin, Cladribine, Clafen (Cyclophosphamide), Clofarabine, Clofarex (Clofarabine), Clolar (Clofarabine), CMF, Cobimetinib, Cometriq (Cabozantinib-S-Malate), Copanlisib Hydrochloride, COPDAC, COPP, COPP-ABV, Cosmegen (Dactinomycin), Cotellic (Cobimetinib), Crizotinib, CVP, Cyclophosphamide, Cyfos (Ifosfamide), Cyramza (Ramucirumab), Cytarabine, Cytarabine Liposome, Cytosar-U (Cytarabine), Cytoxan (Cyclophosphamide), Dabrafenib, Dacarbazine, Dacogen (Decitabine), Dactinomycin, Daratumumab, Darzalex (Daratumumab), Dasatinib, Daunorubicin Hydrochloride, Daunorubicin Hydrochloride and Cytarabine Liposome, Decitabine, Defibrotide Sodium, Defitelio (Defibrotide Sodium), Degarelix, Denileukin Diftitox, Denosumab, DepoCyt (Cytarabine Liposome), Dexamethasone, Dexrazoxane Hydrochloride, Dinutuximab, Docetaxel, Doxil (Doxorubicin Hydrochloride Liposome), Doxorubicin Hydrochloride, Doxorubicin Hydrochloride Liposome, Dox-SL (Doxorubicin Hydrochloride Liposome), DTIC-Dome (Dacarbazine), Durvalumab, Efudex (Fluorouracil--Topical), Elitek (Rasburicase), Ellence (Epirubicin Hydrochloride), Elotuzumab, Eloxatin (Oxaliplatin), Eltrombopag Olamine, Emend (Aprepitant), Empliciti (Elotuzumab), Enasidenib Mesylate, Enzalutamide, Epirubicin Hydrochloride, EPOCH, Erbitux (Cetuximab), Eribulin Mesylate, Erivedge (Vismodegib), Erlotinib Hydrochloride, Erwinaze (Asparaginase Erwinia chrysanthemi), Ethyol (Amifostine), Etopophos (Etoposide Phosphate), Etoposide, Etoposide Phosphate, Evacet (Doxorubicin Hydrochloride Liposome), Everolimus, Evista (Raloxifene Hydrochloride), Evomela (Melphalan Hydrochloride), Exemestane, 5-FU (Fluorouracil Injection), 5-FU (Fluorouracil--Topical), Fareston (Toremifene), Farydak (Panobinostat), Faslodex (Fulvestrant), FEC, Femara (Letrozole), Filgrastim, Fludara (Fludarabine Phosphate), Fludarabine Phosphate, Fluoroplex (Fluorouracil--Topical), Fluorouracil Injection, Fluorouracil--Topical, Flutamide, Folex (Methotrexate), Folex PFS (Methotrexate), FOLFIRI, FOLFIRI-BEVACIZUMAB, FOLFIRI-CETUXIMAB, FOLFIRINOX, FOLFOX, Folotyn (Pralatrexate), FU-LV, Fulvestrant, Gardasil (Recombinant HPV Quadrivalent Vaccine), Gardasil 9 (Recombinant HPV Nonavalent Vaccine), Gazyva (Obinutuzumab), Gefitinib, Gemcitabine Hydrochloride, GEMCITABINE-CISPLATIN, GEMCITABINE-OXALIPLATIN, Gemtuzumab Ozogamicin, Gemzar (Gemcitabine Hydrochloride), Gilotrif (Afatinib Dimaleate), Gleevec (Imatinib Mesylate), Gliadel (Carmustine Implant), Gliadel wafer (Carmustine Implant), Glucarpidase, Goserelin Acetate, Halaven (Eribulin Mesylate), Hemangeol (Propranolol Hydrochloride), Herceptin (Trastuzumab), HPV Bivalent Vaccine, Recombinant, HPV Nonavalent Vaccine, Recombinant, HPV Quadrivalent Vaccine, Recombinant, Hycamtin (Topotecan Hydrochloride), Hydrea (Hydroxyurea), Hydroxyurea, Hyper-CVAD, Ibrance (Palbociclib), Ibritumomab Tiuxetan, Ibrutinib, ICE, Iclusig (Ponatinib Hydrochloride), Idamycin (Idarubicin Hydrochloride), Idarubicin Hydrochloride, Idelalisib, Idhifa (Enasidenib Mesylate), Ifex (Ifosfamide), Ifosfamide, Ifosfamidum (Ifosfamide), IL-2 (Aldesleukin), Imatinib Mesylate, Imbruvica (Ibrutinib), Imfinzi (Durvalumab), Imiquimod, Imlygic (Talimogene Laherparepvec), Inlyta (Axitinib), Inotuzumab Ozogamicin, Interferon Alfa-2b, Recombinant, Interleukin-2 (Aldesleukin), Intron A (Recombinant Interferon Alfa-2b), Iodine I 131 Tositumomab and Tositumomab, Ipilimumab, Iressa (Gefitinib), Irinotecan Hydrochloride, Irinotecan Hydrochloride Liposome, Istodax (Romidepsin), Ixabepilone, Ixazomib Citrate, Ixempra (Ixabepilone), Jakafi (Ruxolitinib Phosphate), JEB, Jevtana (Cabazitaxel), Kadcyla (Ado-Trastuzumab Emtansine), Keoxifene (Raloxifene Hydrochloride), Kepivance (Palifermin), Keytruda (Pembrolizumab), Kisqali (Ribociclib), Kymriah (Tisagenlecleucel), Kyprolis (Carfilzomib), Lanreotide Acetate, Lapatinib Ditosylate, Lartruvo (Olaratumab), Lenalidomide, Lenvatinib Mesylate, Lenvima (Lenvatinib Mesylate), Letrozole, Leucovorin Calcium, Leukeran (Chlorambucil), Leuprolide Acetate, Leustatin (Cladribine), Levulan (Aminolevulinic Acid), Linfolizin (Chlorambucil), LipoDox (Doxorubicin Hydrochloride Liposome), Lomustine, Lonsurf (Trifluridine and Tipiracil Hydrochloride), Lupron (Leuprolide Acetate), Lupron Depot (Leuprolide Acetate), Lupron Depot-Ped (Leuprolide Acetate), Lynparza (Olaparib), Marqibo (Vincristine Sulfate Liposome), Matulane (Procarbazine Hydrochloride), Mechlorethamine Hydrochloride, Megestrol Acetate, Mekinist (Trametinib), Melphalan, Melphalan Hydrochloride, Mercaptopurine, Mesna, Mesnex (Mesna), Methazolastone (Temozolomide), Methotrexate, Methotrexate LPF (Methotrexate), Methylnaltrexone Bromide, Mexate (Methotrexate), Mexate-AQ (Methotrexate), Midostaurin, Mitomycin C, Mitoxantrone Hydrochloride, Mitozytrex (Mitomycin C), MOPP, Mozobil (Plerixafor), Mustargen (Mechlorethamine Hydrochloride), Mutamycin (Mitomycin C), Myleran (Busulfan), Mylosar (Azacitidine), Mylotarg (Gemtuzumab Ozogamicin), Nanoparticle Paclitaxel (Paclitaxel Albumin-stabilized Nanoparticle Formulation), Navelbine (Vinorelbine Tartrate), Necitumumab, Nelarabine, Neosar (Cyclophosphamide), Neratinib Maleate, Nerlynx (Neratinib Maleate), Netupitant and Palonosetron Hydrochloride, Neulasta (Pegfilgrastim), Neupogen (Filgrastim), Nexavar (Sorafenib Tosylate), Nilandron (Nilutamide), Nilotinib, Nilutamide, Ninlaro (Ixazomib Citrate), Niraparib Tosylate Monohydrate, Nivolumab, Nolvadex (Tamoxifen Citrate), Nplate (Romiplostim), Obinutuzumab, Odomzo (Sonidegib), OEPA, Ofatumumab, OFF, Olaparib, Olaratumab, Omacetaxine Mepesuccinate, Oncaspar (Pegaspargase), Ondansetron Hydrochloride, Onivyde (Irinotecan Hydrochloride Liposome), Ontak (Denileukin Diftitox), Opdivo (Nivolumab), OPPA, Osimertinib, Oxaliplatin, Paclitaxel, Paclitaxel Albumin-stabilized Nanoparticle Formulation, PAD, Palbociclib, Palifermin, Palonosetron Hydrochloride, Palonosetron Hydrochloride and Netupitant, Pamidronate Disodium, Panitumumab, Panobinostat, Paraplat (Carboplatin), Paraplatin (Carboplatin), Pazopanib Hydrochloride, PCV, PEB, Pegaspargase, Pegfilgrastim, Peginterferon Alfa-2b, PEG-Intron (Peginterferon Alfa-2b), Pembrolizumab, Pemetrexed Disodium, Perjeta (Pertuzumab), Pertuzumab, Platinol (Cisplatin), Platinol-AQ (Cisplatin), Plerixafor, Pomalidomide, Pomalyst (Pomalidomide), Ponatinib Hydrochloride, Portrazza (Necitumumab), Pralatrexate, Prednisone, Procarbazine Hydrochloride, Proleukin (Aldesleukin), Prolia (Denosumab), Promacta (Eltrombopag Olamine), Propranolol Hydrochloride, Provenge (Sipuleucel-T), Purinethol (Mercaptopurine), Purixan (Mercaptopurine), Radium 223 Dichloride, Raloxifene Hydrochloride, Ramucirumab, Rasburicase, R-CHOP, R-CVP, Recombinant Human Papillomavirus (HPV) Bivalent Vaccine, Recombinant Human Papillomavirus (HPV) Nonavalent Vaccine, Recombinant Human Papillomavirus (HPV) Quadrivalent Vaccine, Recombinant Interferon Alfa-2b, Regorafenib, Relistor (Methylnaltrexone Bromide), R-EPOCH, Revlimid (Lenalidomide), Rheumatrex (Methotrexate), Ribociclib, R-ICE, Rituxan (Rituximab), Rituxan Hycela (Rituximab and Hyaluronidase Human), Rituximab, Rituximab and Hyaluronidase Human, Rolapitant Hydrochloride, Romidepsin, Romiplostim, Rubidomycin (Daunorubicin Hydrochloride), Rubraca (Rucaparib Camsylate), Rucaparib Camsylate, Ruxolitinib Phosphate, Rydapt (Midostaurin), Sclerosol Intrapleural Aerosol (Talc), Siltuximab, Sipuleucel-T, Somatuline Depot (Lanreotide Acetate), Sonidegib, Sorafenib Tosylate, Sprycel (Dasatinib), STANFORD V, Sterile Talc Powder (Talc), Steritalc (Talc), Stivarga (Regorafenib), Sunitinib Malate, Sutent (Sunitinib Malate), Sylatron (Peginterferon Alfa-2b), Sylvant (Siltuximab), Synribo (Omacetaxine Mepesuccinate), Tabloid (Thioguanine), TAC, Tafinlar (Dabrafenib), Tagrisso (Osimertinib), Talc, Talimogene Laherparepvec, Tamoxifen Citrate, Tarabine PFS (Cytarabine), Tarceva (Erlotinib Hydrochloride), Targretin (Bexarotene), Tasigna (Nilotinib), Taxol (Paclitaxel), Taxotere (Docetaxel), Tecentriq (Atezolizumab), Temodar (Temozolomide), Temozolomide, Temsirolimus, Thalidomide, Thalomid (Thalidomide), Thioguanine, Thiotepa, Tisagenlecleucel, Tolak (Fluorouracil--Topical), Topotecan Hydrochloride, Toremifene, Torisel (Temsirolimus), Tositumomab and Iodine I 131 Tositumomab, Totect (Dexrazoxane Hydrochloride), TPF, Trabectedin, Trametinib, Trastuzumab, Treanda (Bendamustine Hydrochloride), Trifluridine and Tipiracil Hydrochloride, Trisenox (Arsenic Trioxide), Tykerb (Lapatinib Ditosylate), Unituxin (Dinutuximab), Uridine Triacetate, VAC, Valrubicin, Valstar (Valrubicin), Vandetanib, VAMP, Varubi (Rolapitant Hydrochloride), Vectibix (Panitumumab), VelP, Velban (Vinblastine Sulfate), Velcade (Bortezomib), Velsar (Vinblastine Sulfate), Vemurafenib, Venclexta (Venetoclax), Venetoclax, Verzenio (Abemaciclib), Viadur (Leuprolide Acetate), Vidaza (Azacitidine), Vinblastine Sulfate, Vincasar PFS (Vincristine Sulfate), Vincristine Sulfate, Vincristine Sulfate Liposome, Vinorelbine Tartrate, VIP, Vismodegib, Vistogard (Uridine Triacetate), Voraxaze (Glucarpidase), Vorinostat, Votrient (Pazopanib Hydrochloride), Vyxeos (Daunorubicin Hydrochloride and Cytarabine Liposome), Wellcovorin (Leucovorin Calcium), Xalkori (Crizotinib), Xeloda (Capecitabine), XELIRI, XELOX, Xgeva (Denosumab), Xofigo (Radium 223 Dichloride), Xtandi (Enzalutamide), Yervoy (Ipilimumab), Yescarta (Axicabtagene Ciloleucel), Yondelis (Trabectedin), Zaltrap (Ziv-Aflibercept), Zarxio (Filgrastim), Zejula (Niraparib Tosylate Monohydrate), Zelboraf (Vemurafenib), Zevalin (Ibritumomab Tiuxetan), Zinecard (Dexrazoxane Hydrochloride), Ziv-Aflibercept, Zofran (Ondansetron Hydrochloride), Zoladex (Goserelin Acetate), Zoledronic Acid, Zolinza (Vorinostat), Zometa (Zoledronic Acid), Zydelig (Idelalisib), Zykadia (Ceritinib) and Zytiga (Abiraterone Acetate).

In some embodiments, the treatment may comprise administration of a corticosteroid, e.g. dexamethasone and/or prednisone.

In some embodiments, a subject is administered lymphodepleting chemotherapy prior to administration of immune cells expressing/comprising a CAR described herein (or expressing/comprising nucleic acid encoding such a CAR).

That is, in some embodiments, methods of treating/preventing a disease/condition in accordance with the present disclosure comprise: (i) administering a lymphodepleting chemotherapy to a subject, and (ii) subsequently administering an immune cell expressing/comprising a CAR according to the present disclosure, or expressing/comprising a nucleic acid encoding a CAR according to the present disclosure.

As used herein, 'lymphodepleting chemotherapy' refers to treatment with a chemotherapeutic agent which results in depletion of lymphocytes (e.g. T cells, B cells, NK cells, NKT cells or innate lymphoid cell (ILCs), or precursors thereof) within the subject to which the treatment is administered. A 'lymphodepleting chemotherapeutic agent' refers to a chemotherapeutic agent which results in depletion of lymphocytes.

Lymphodepleting chemotherapy and its use in methods of treatment by adoptive cell transfer are described *e.g.* in Klebanoff et al., Trends Immunol. (2005) 26(2):111-7 and Muranski et al., Nat Clin Pract Oncol. (2006) (12):668-81, both of which are hereby incorporated by reference in their entirety. The aim of lymphodepleting chemotherapy is to deplete the recipient subject's endogenous lymphocyte population.

In the context of treatment of disease by adoptive transfer of immune cells, lymphodepleting chemotherapy is typically administered prior to adoptive cell transfer, to condition the recipient subject to receive the adoptively transferred cells. Lymphodepleting chemotherapy is thought to promote the persistence and activity of adoptively transferred cells by creating a permissive environment, *e.g.* through elimination of cells expressing immunosuppressive cytokines, and creating the 'lymphoid space' required for expansion and activity of adoptively transferred lymphoid cells.

Chemotherapeutic agents commonly used in lymphodepleting chemotherapy include *e.g.* fludarabine, cyclophosphamide, bedamustine and pentostatin.

Multiple doses of the antigen-binding molecule, polypeptide, CAR, nucleic acid (or plurality thereof), expression vector (or plurality thereof), cell or composition may be provided. One or more, or each, of the doses may be accompanied by simultaneous or sequential administration of another therapeutic agent.

Multiple doses may be separated by a predetermined time interval, which may be selected to be one of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, or 31 days, or 1, 2, 3, 4, 5, or 6 months. By way of example, doses may be given once every 7, 14, 21 or 28 days (plus or minus 3, 2, or 1 days).

In accordance with various aspects of the present disclosure, a method of treating and/or preventing a disease/condition may comprise one or more of the following: reducing the number/proportion of cells expressing the target antigen for the CAR (*e.g.* CD19); inhibiting tumor growth (*e.g.* of a tumor expressing the target antigen for the CAR); reducing metastasis of a cancer (*e.g.* a cancer expressing the target antigen for the CAR); increasing survival of a subject having a cancer (*e.g.* a cancer expressing the target antigen for the CAR).

### Subjects

A subject in accordance with the various aspects of the present disclosure may be any animal or human. Therapeutic and prophylactic applications may be in human or animals (veterinary use).

The subject to be administered with an article of the present disclosure (*e.g.* in accordance with therapeutic or prophylactic intervention) may be a subject in need of such intervention. The subject is preferably mammalian, more preferably human. The subject may be a non-human mammal, but is more preferably human. The subject may be male or female. The subject may be a patient.

A subject may have (*e.g.* may have been diagnosed with) a disease or condition described herein, may be suspected of having such a disease/condition, or may be at risk of developing/contracting such a disease/condition. In embodiments according to the present disclosure, a subject may be selected for treatment according to the methods based on characterisation for one or more markers of such a disease/condition.

In some embodiments, a subject may be selected for therapeutic or prophylactic intervention as described herein based on the detection of cells/tissue expressing the target antigen for the CAR (*e.g.* CD19), or of cells/tissue overexpressing the target antigen for the CAR, *e.g.* in a sample obtained from the subject.

A subject may be an allogeneic subject with respect to an intervention in accordance with the present disclosure. A subject to be treated/prevented in accordance with the present disclosure may be genetically non-identical to the subject from which the CAR-expressing immune cells are derived. A subject to be treated/prevented in accordance with the present disclosure may be HLA mismatched with respect to the subject from which the CAR-expressing immune cells are derived. A subject to be treated/prevented in accordance with the present disclosure may be HLA matched with respect to the subject from which the CAR-expressing immune cells are derived.

The subject to which cells are administered in accordance with the present disclosure may be allogeneic/non-autologous with respect to the source from which the cells are/were derived. The subject to which cells are administered may be a different subject to the subject from which cells are/were obtained for the production of the cells to be administered. The subject to which the cells are administered may be genetically non-identical to the subject from which cells are/were obtained for the production of the cells to be administered.

The subject to which cells are administered may comprise MHC/HLA genes encoding MHC/HLA molecules which are non-identical to the MHC/HLA molecules encoded by the MHC/HLA genes of the subject from which cells are/were obtained for the production of the cells to be administered. The subject to which cells are administered may comprise MHC/HLA genes encoding MHC/HLA molecules which are identical to the MHC/HLA molecules encoded by the MHC/HLA genes of the subject from which cells are/were obtained for the production of the cells to be administered.

In some embodiments, the subject to which cells are administered is HLA matched with respect to the subject from which cells are/were obtained for the production of the cells to be administered. In some embodiments, the subject to which cells are administered is a near or complete HLA match with respect to the subject from which cells are/were obtained for the production of the cells to be administered.

In some embodiments, the subject is a ≥4/8 (*i.e.* 4/8, 5/8, 6/8, 7/8 or 8/8) match across HLA-A, -B, -C, and -DRB1. In some embodiments, the subject is a ≥5/10 (*i.e.* 5/10, 6/10, 7/10, 8/10, 9/10 or 10/10) match across HLA-A, -B, -C, -DRB1 and -DQB1. In some embodiments, the subject is a ≥6/12 (*i.e.* 6/12, 7/12, 8/12, 9/12, 10/12, 11/12 or 12/12) match across HLA-A, -B, -C, -DRB1, -DQB1 and -DPB1. In some embodiments, the subject is an 8/8 match across HLA-A, -B, -C, and -DRB1. In some embodiments, the subject is a 10/10 match across HLA-A, -B, -C, -DRB1 and -DQB1. In some embodiments, the subject is a 12/12 match across HLA-A, -B, -C, -DRB1, -DQB1 and -DPB1.

### Kits

In some aspects of the present disclosure a kit of parts is provided. In some embodiments, the kit may have at least one container having a predetermined quantity of a CAR, nucleic acid (or plurality thereof), expression vector (or plurality thereof), cell or composition described herein.

In some embodiments, the kit may comprise materials for producing a CAR, nucleic acid (or plurality thereof), expression vector (or plurality thereof), cell or composition described herein.

The kit may provide the CAR, nucleic acid (or plurality thereof), expression vector (or plurality thereof), cell or composition together with instructions for administration to a patient in order to treat a specified disease/condition.

In some embodiments the kit may further comprise at least one container having a predetermined quantity of another therapeutic agent (*e.g.* as described herein). In such embodiments, the kit may also comprise a second medicament or pharmaceutical composition such that the two medicaments or pharmaceutical compositions may be administered simultaneously or separately such that they provide a combined treatment for the specific disease or condition.

Kits according to the present disclosure may include instructions for use, *e.g.* in the form of an instruction booklet or leaflet. The instructions may include a protocol for performing any one or more of the methods described herein.

### Sequence identity

As used herein, 'sequence identity' refers to the percent of nucleotides/amino acid residues in a subject sequence that are identical to nucleotides/amino acid residues in a reference sequence, after aligning the sequences and, if necessary, introducing gaps, to achieve the maximum percent sequence identity between the sequences. Pairwise and multiple sequence alignment for the purposes of determining percent sequence identity between two or more amino acid or nucleic acid sequences can be achieved in various ways known to a person of skill in the art, for instance, using publicly available computer software such as ClustalOmega (Söding, J. 2005, Bioinformatics 21, 951-960), T-coffee (Notredame et al. 2000, J. Mol. Biol. (2000) 302, 205-217), Kalign (Lassmann and Sonnhammer 2005, BMC Bioinformatics, 6(298)) and MAFFT (Katoh and Standley 2013, Molecular Biology and Evolution, 30(4) 772-780) software. When using such software, the default parameters, *e.g.* for gap penalty and extension penalty, are preferably used.

### Sequences

| SEQ ID NO: | DESCRIPTION | SEQUENCE |
|---|---|---|
| 1 | Interaction domain (3H1DA.1)) | EIAACEEEIAALEAKIAALKE |
| 2 | Interaction domain (3H1DA.2) | KIAACKAKIAALKAEIAALEA |
| 3 | Interaction domain (3H2DA.1) | EIAACEEEIAALEAKIAACKE |
| 4 | Interaction domain (3H2DA.2) | KIAACKAKIAALKAEIAACEE |
| 5 | Interaction domain (3H1DP.1) | EIQRCEQEIRRLEQKIERLKR |
| 6 | Interaction domain (3H1DP.2) | KIERCKEKIERLKEEIQRLEQ |
| 7 | Interaction domain (HD1.1) | ECTGHIEGPTCTCHCK |
| 8 | Interaction domain (HD1.2) | DDKDCDEYCKKTKE |
| 9 | Exemplary anti-CD19 CAR amino acid sequence (CD8 leader_Myc-Tag_3H1DA.1_anti-CD19 scFv_3H1DA.2_CD8 hinge_CD8 **TM_41BB_CD3ζ**) | ALPVTALLLPLALLLHAARPEQKLISEEDLEIAACEEEIAALEAKIAALKEGSGDIQMTQTTSSLS ASLGDRVTISCRASQDISKYLNWYQQKPDGTVKLLIYHTSRLHSGVPSRFSGSGSGTDYSLTI SNLEQEDIATYFCQQGNTLPYTFGGGTKLEITGGGGSGGGGSGGGGSEVKLQESGPGLVAP SQSLSVTCTVSGVSLPDYGVSWIRQPPRKGLEWLGVIWGSETTYYNSALKSRLTIIKDNSKSQ VFLKMNSLQTDDTAIYYCAKHYYYGGSYAMDYWGQGTSVTVSSGSGKIAACKAKIAALKAEIA ALEATTTPAPRPPTPAPTIASQPLSLRPEACRPAAGGAVHTRGLDFACDIYIWAPLAGTCGVLL LSLVITLYCKRGRKKLLYIFKQPFMRPVQTTQEEDGCSCRFPEEEEGGCELRVKFSRSADAPA YQQGQNQLYNELNLGRREEYDVLDKRRGRDPEMGGKPRRKNPQEGLYNELQKDKMAEAYS EIGMKGERRRGKGHDGLYQGLSTATKDTYDALHMQALPPR |
| 10 | Exemplary anti-CD19 CAR amino acid sequence (CD8 leader_Myc-Tag_3H2DA.1_anti-CD19 scFv_3H2DA.2_CD8 hinge_CD8 **TM_41BB_CD3ζ**) | ALPVTALLLPLALLLHAARPEQKLISEEDLEIAACEEEIAALEAKIAACKEGSGDIQMTQTTSSLS ASLGDRVTISCRASQDISKYLNWYQQKPDGTVKLLIYHTSRLHSGVPSRFSGSGSGTDYSLTI SNLEQEDIATYFCQQGNTLPYTFGGGTKLEITGGGGSGGGGSGGGGSEVKLQESGPGLVAP SQSLSVTCTVSGVSLPDYGVSWIRQPPRKGLEWLGVIWGSETTYYNSALKSRLTIIKDNSKSQ VFLKMNSLQTDDTAIYYCAKHYYYGGSYAMDYWGQGTSVTVSSGSGKIAACKAKIAALKAEIA ACEETTTPAPRPPTPAPTIASQPLSLRPEACRPAAGGAVHTRGLDFACDIYIWAPLAGTCGVLL LSLVITLYCKRGRKKLLYIFKQPFMRPVQTTQEEDGCSCRFPEEEEGGCELRVKFSRSADAPA YQQGQNQLYNELNLGRREEYDVLDKRRGRDPEMGGKPRRKNPQEGLYNELQKDKMAEAYS EIGMKGERRRGKGHDGLYQGLSTATKDTYDALHMQALPPR |
| 11 | Exemplary anti-CD19 CAR amino acid sequence (CD8 leader_Myc-Tag_3H1DP.1_anti-CD19 scFv_3H1DP.2_CD8 hinge_CD8 TM_41BB_CD3ζ) | ALPVTALLLPLALLLHAARPEQKLISEEDLEIQRCEQEIRRLEQKIERLKRGSGDIQMTQTTSSL SASLGDRVTISCRASQDISKYLNWYQQKPDGTVKLLIYHTSRLHSGVPSRFSGSGSGTDYSLT ISNLEQEDIATYFCQQGNTLPYTFGGGTKLEITGGGGSGGGGSGGGGSEVKLQESGPGLVAP SQSLSVTCTVSGVSLPDYGVSWIRQPPRKGLEWLGVIWGSETTYYNSALKSRLTIIKDNSKSQ VFLKMNSLQTDDTAIYYCAKHYYYGGSYAMDYWGQGTSVTVSSGSGKIERCKEKIERLKEEIQ RLEQTTTPAPRPPTPAPTIASQPLSLRPEACRPAAGGAVHTRGLDFACDIYIWAPLAGTCGVLL LSLVITLYCKRGRKKLLYIFKQPFMRPVQTTQEEDGCSCRFPEEEEGGCELRVKFSRSADAPA |
| | | YQQGQNQLYNELNLGRREEYDVLDKRRGRDPEMGGKPRRKNPQEGLYNELQKDKMAEAYS EIGMKGERRRGKGHDGLYQGLSTATKDTYDALHMQALPPR |
| 12 | Exemplary anti-CD19 CAR amino acid sequence (CD8 leader_Myc-Tag_HD1.1_anti-CD19 scFv_HD1.2_CD8 hinge_CD8 **TM_41BB_CD3ζ**) | ALPVTALLLPLALLLHAARPEQKLISEEDLECTGHIEGPTCTCHCKGSGDIQMTQTTSSLSASL GDRVTISCRASQDISKYLNWYQQKPDGTVKLLIYHTSRLHSGVPSRFSGSGSGTDYSLTISNL EQEDIATYFCQQGNTLPYTFGGGTKLEITGGGGSGGGGSGGGGSEVKLQESGPGLVAPSQS LSVTCTVSGVSLPDYGVSWIRQPPRKGLEWLGVIWGSETTYYNSALKSRLTIIKDNSKSQVFL KMNSLQTDDTAIYYCAKHYYYGGSYAMDYWGQGTSVTVSSGSGDDKDCDEYCKKTKETTTP APRPPTPAPTIASQPLSLRPEACRPAAGGAVHTRGLDFACDIYIWAPLAGTCGVLLLSLVITLY CKRGRKKLLYIFKQPFMRPVQTTQEEDGCSCRFPEEEEGGCELRVKFSRSADAPAYQQGQN QLYNELNLGRREEYDVLDKRRGRDPEMGGKPRRKNPQEGLYNELQKDKMAEAYSEIGMKG ERRRGKGHDGLYQGLSTATKDTYDALHMQALPPR |
| 13 | Exemplary anti-BCMA CAR amino acid sequence (CD8 leader_Myc-Tag_3H1DA.1_anti-BCMA scFv_3H1DA.2_CD8 hinge_CD8 **TM_41BB_CD3ζ**) | ALPVTALLLPLALLLHAARPEQKLISEEDLEIAACEEEIAALEAKIAALKEGSGQVQLVQSGAEV KKPGSSVKVSCKASGYTFTSYVMHWVRQAPGQGLEWMGYIIPYNDATKYNEKFKGRVTITAD KSTSTAYMELSSLRSEDTAVYYCARYNYDGYFDVWGQGTLVTVSSGGGGSGGGGSGGGGS EIVLTQSPATLSLSPGERATLSCRASQSISDYLHWYQQKPGQAPRLLIYYASQSITGIPARFSG SGSGTDFTLTISSLEPEDFAVYYCQNGHSFPPTFGGGTKVEIKGSGKIAACKAKIAALKAEIAAL EATTTPAPRPPTPAPTIASQPLSLRPEACRPAAGGAVHTRGLDFACDIYIWAPLAGTCGVLLLS LVITLYCKRGRKKLLYIFKQPFMRPVQTTQEEDGCSCRFPEEEEGGCELRVKFSRSADAPAY QQGQNQLYNELNLGRREEYDVLDKRRGRDPEMGGKPRRKNPQEGLYNELQKDKMAEAYSE IGMKGERRRGKGHDGLYQGLSTATKDTYDALHMQALPPR |
| 14 | Exemplary anti-BCMA CAR amino acid sequence (CD8 leader_Myc-Tag_3H2DA.1_anti-BCMA scFv_3H2DA.2_CD8 hinge_CD8 **TM_41BB_CD3ζ**) | ALPVTALLLPLALLLHAARPEQKLISEEDLEIAACEEEIAALEAKIAACKEGSGQVQLVQSGAEV KKPGSSVKVSCKASGYTFTSYVMHWVRQAPGQGLEWMGYIIPYNDATKYNEKFKGRVTITAD KSTSTAYMELSSLRSEDTAVYYCARYNYDGYFDVWGQGTLVTVSSGGGGSGGGGSGGGGS EIVLTQSPATLSLSPGERATLSCRASQSISDYLHWYQQKPGQAPRLLIYYASQSITGIPARFSG SGSGTDFTLTISSLEPEDFAVYYCQNGHSFPPTFGGGTKVEIKGSGKIAACKAKIAALKAEIAAC EETTTPAPRPPTPAPTIASQPLSLRPEACRPAAGGAVHTRGLDFACDIYIWAPLAGTCGVLLLS LVITLYCKRGRKKLLYIFKQPFMRPVQTTQEEDGCSCRFPEEEEGGCELRVKFSRSADAPAY QQGQNQLYNELNLGRREEYDVLDKRRGRDPEMGGKPRRKNPQEGLYNELQKDKMAEAYSE IGMKGERRRGKGHDGLYQGLSTATKDTYDALHMQALPPR |
| 15 | Exemplary anti-BCMA CAR amino acid sequence (CD8 leader_Myc-Tag_3H1DP.1_anti-BCMA scFv_3H1DP.2_CD8 hinge_CD8 **TM_41BB_CD3ζ**) | ALPVTALLLPLALLLHAARPEQKLISEEDLEIQRCEQEIRRLEQKIERLKRGSGQVQLVQSGAEV KKPGSSVKVSCKASGYTFTSYVMHWVRQAPGQGLEWMGYIIPYNDATKYNEKFKGRVTITAD KSTSTAYMELSSLRSEDTAVYYCARYNYDGYFDVWGQGTLVTVSSGGGGSGGGGSGGGGS EIVLTQSPATLSLSPGERATLSCRASQSISDYLHWYQQKPGQAPRLLIYYASQSITGIPARFSG SGSGTDFTLTISSLEPEDFAVYYCQNGHSFPPTFGGGTKVEIKGSGKIERCKEKIERLKEEIQR LEQTTTPAPRPPTPAPTIASQPLSLRPEACRPAAGGAVHTRGLDFACDIYIWAPLAGTCGVLLL SLVITLYCKRGRKKLLYIFKQPFMRPVQTTQEEDGCSCRFPEEEEGGCELRVKFSRSADAPAY QQGQNQLYNELNLGRREEYDVLDKRRGRDPEMGGKPRRKNPQEGLYNELQKDKMAEAYSE IGMKGERRRGKGHDGLYQGLSTATKDTYDALHMQALPPR |
| 16 | Exemplary anti-CD19 CAR nucleic acid sequence (CD8 leader_Myc-Tag_3H1DA.1_anti-CD19 scFv_3H1DA.2_CD8 | gccctgcctgtgacagctctgctgctgcctctggccctgctgctgcatgccgccagacccgagcagaagctgatcagcgaggagg acctgGAGATTGCGGCATGCGAGGAGGAAATAGCGGCACTGGAGGCCAAGATCGCAGCAT TGAAAGAGGGGAGCGGTgacattcaaatgactcaaacaacttcatctttgtctgcaagcctgggagaccgcgtgacaat aagctgccgagccagtcaagatatcagcaaatatctcaattggtatcagcaaaagcctgatgggactgttaaattgttgatataccat acgagtcgcctccacagcggagttccttctaggttttctggatcaggctccgggaccgactactccctcacgattagcaatcttgagca ggaggatatagccacatatttttgtcaacaaggaaacacacttccctacaccttcggtggggggaacgaaattggaaattactggtgg cggcgggtctggaggtggcggtagtggaggcgggggctctgaagtcaagcttcaagaatcaggtccgggacttgtagcgccaagt cagtctctttccgtaacttgtaccgttagtggggtttccttgccagattacggagtaagttggattcgccaaccgcccagaaagggcttg |
| | hinge_CD8 **TM_41BB_CD3ζ**) | gaatggttgggtgttatctggggaagcgaaacaacatactataacagcgcactcaagtctcgcctcacgattattaaagataactcta aatctcaggtgttcctcaaaatgaactcattgcaaacggacgacactgccatctattactgtgcaaaacactattactacggcggctct tacgctatggattactggggccagggaacttcagtgacagtttctagtGGTTCTGGGAAGATCGCAGCCTGCAAG GCTAAGATTGCTGCTCTTAAAGCGGAGATCGCGGCGCTGGAAGCAacaaccacccctgcccctag acctccaaccccagcccctacaatcgccagccagcctctgtctctgaggcccgaggcttgtagaccagctgctggcggagccgtg cacaccagaggactggatttcgcctgcgacatctacatctgggcccctctggccggcacatgtggcgtgctgctgctgagcctcgtg atcaccctgtactgcaagcggggcagaaagaaactgctgtacatctttaagcagcccttcatgcggcccgtgcagaccacccagg aagaggacggctgctcctgcagattccccgaggaagaagaaggcggctgcgagctgagagtgaagttcagcagatccgccgac gcccctgcctaccagcagggacagaaccagctgtacaacgagctgaacctgggcagacgggaagagtacgacgtgctggaca agcggagaggccgggatcctgaaatgggcggcaagcccagacggaagaacccccaggaaggcctgtataacgaactgcaga aagacaagatggccgaggcctacagcgagatcggaatgaagggcgagcggagaagaggcaagggccacgatggcctgtac cagggcctgagcaccgccaccaaggacacctatgacgccctgcacatgcaggctttgccgcctaga |
| 17 | Exemplary anti-CD19 CAR nucleic acid sequence (CD8 leader_Myc-Tag_3H2DA.1_anti-CD19 scFv_3H2DA.2_CD8 hinge_CD8 **TM_41BB_CD3ζ**) | gccctgcctgtgacagctctgctgctgcctctggccctgctgctgcatgccgccagacccgagcagaagctgatcagcgaggagg acctgGAGATTGCGGCTTGCGAAGAGGAGATAGCAGCCTTGGAAGCAAAGATTGCTGCGTG TAAAGAGGGCAGCGGTgacattcaaatgactcaaacaacttcatctttgtctgcaagcctgggagaccgcgtgacaata agctgccgagccagtcaagatatcagcaaatatctcaattggtatcagcaaaagcctgatgggactgttaaattgttgatataccata cgagtcgcctccacagcggagttccttctaggttttctggatcaggctccgggaccgactactccctcacgattagcaatcttgagcag gaggatatagccacatatttttgtcaacaaggaaacacacttccctacaccttcggtgggggaacgaaattggaaattactggtggc ggcgggtctggaggtggcggtagtggaggcgggggctctgaagtcaagcttcaagaatcaggtccgggacttgtagcgccaagtc agtctctttccgtaacttgtaccgttagtggggtttccttgccagattacggagtaagttggattcgccaaccgcccagaaagggcttgg aatggttgggtgttatctggggaagcgaaacaacatactataacagcgcactcaagtctcgcctcacgattattaaagataactctaa atctcaggtgttcctcaaaatgaactcattgcaaacggacgacactgccatctattactgtgcaaaacactattactacggcggctctt acgctatggattactggggccagggaacttcagtgacagtttctagtGGCTCAGGCAAGATTGCTGCATGTAAAG CTAAGATTGCCGCGCTGAAAGCGGAGATAGCCGCGTGTGAGGAGacaaccacccctgcccctaga cctccaaccccagcccctacaatcgccagccagcctctgtctctgaggcccgaggcttgtagaccagctgctggcggagccgtgc acaccagaggactggatttcgcctgcgacatctacatctgggcccctctggccggcacatgtggcgtgctgctgctgagcctcgtgat caccctgtactgcaagcggggcagaaagaaactgctgtacatctttaagcagcccttcatgcggcccgtgcagaccacccaggaa gaggacggctgctcctgcagattccccgaggaagaagaaggcggctgcgagctgagagtgaagttcagcagatccgccgacgc ccctgcctaccagcagggacagaaccagctgtacaacgagctgaacctgggcagacgggaagagtacgacgtgctggacaag cggagaggccgggatcctgaaatgggcggcaagcccagacggaagaacccccaggaaggcctgtataacgaactgcagaaa gacaagatggccgaggcctacagcgagatcggaatgaagggcgagcggagaagaggcaagggccacgatggcctgtacca gggcctgagcaccgccaccaaggacacctatgacgccctgcacatgcaggctttgccgcctaga |
| 18 | Exemplary anti-CD19 CAR nucleic acid sequence (CD8 leader_Myc-Tag_3H1DP.1_anti-CD19 scFv_3H1DP.2_CD8 hinge_CD8 **TM_41BB_CD3ζ**) | gccctgcctgtgacagctctgctgctgcctctggccctgctgctgcatgccgccagacccgagcagaagctgatcagcgaggagg acctgGAGATCCAGCGCTGTGAGCAGGAGATCCGAAGACTTGAGCAAAAGATCGAGCGCC TGAAACGAGGATCAGGTgacattcaaatgactcaaacaacttcatctttgtctgcaagcctgggagaccgcgtgacaata agctgccgagccagtcaagatatcagcaaatatctcaattggtatcagcaaaagcctgatggactgttaaattgttgatataccata cgagtcgcctccacagcggagttccttctaggttttctggatcaggctccgggaccgactactccctcacgattagcaatcttgagcag gaggatatagccacatatttttgtcaacaaggaaacacacttccctacaccttcggtgggggaacgaaattggaaattactggtggc ggcgggtctggaggtggcggtagtggaggcgggggctctgaagtcaagcttcaagaatcaggtccgggacttgtagcgccaagtc agtctctttccgtaacttgtaccgttagtggggtttccttgccagattacggagtaagttggattcgccaaccgcccagaaagggcttgg aatggttgggtgttatctggggaagcgaaacaacatactataacagcgcactcaagtctcgcctcacgattattaaagataactctaa atctcaggtgttcctcaaaatgaactcattgcaaacggacgacactgccatctattactgtgcaaaacactattactacggcggctctt acgctatggattactggggccagggaacttcagtgacagtttctagtGGAAGTGGCAAAATTGAAAGATGTAAGG AGAAGATCGAGCGGCTGAAAGAAGAGATCCAAAGACTTGAGCAGacaaccacccctgccccctaga cctccaaccccagcccctacaatcgccagccagcctctgtctctgaggcccgaggcttgtagaccagctgctggcggagccgtgc acaccagaggactggatttcgcctgcgacatctacatctgggcccctctggccggcacatgtggcgtgctgctgctgagcctcgtgat |
| | | caccctgtactgcaagcggggcagaaagaaactgctgtacatctttaagcagcccttcatgcggcccgtgcagaccacccaggaa gaggacggctgctcctgcagattccccgaggaagaagaaggcggctgcgagctgagagtgaagttcagcagatccgccgacgc ccctgcctaccagcagggacagaaccagctgtacaacgagctgaacctgggcagacgggaagagtacgacgtgctggacaag cggagaggccgggatcctgaaatgggcggcaagcccagacggaagaacccccaggaaggcctgtataacgaactgcagaaa gacaagatggccgaggcctacagcgagatcggaatgaagggcgagcggagaagaggcaagggccacgatggcctgtacca gggcctgagcaccgccaccaaggacacctatgacgccctgcacatgcaggctttgccgcctaga |
| 19 | Exemplary anti-CD19 CAR nucleic acid sequence (CD8 leader_Myc-Tag_HD1.1_anti-CD19 scFv_HD1.2_CD8 hinge_CD8 **TM_41BB_CD3ζ**) | gccctgcctgtgacagctctgctgctgcctctggccctgctgctgcatgccgccagacccgagcagaagctgatcagcgaggagg acctgGAATGCACTGGCCATATTGAAGGTCCTACTTGCACGTGCCACTGTAAAGGAGCGG Tgacattcaaatgactcaaacaacttcatctttgtctgcaagcctgggagaccgcgtgacaataagctgccgagccagtcaagatat cagcaaatatctcaattggtatcagcaaaagcctgatgggactgttaaattgttgatataccatacgagtcgcctccacagcggagtt ccttctaggttttctggatcaggctccgggaccgactactccctcacgattagcaatcttgagcaggaggatatagccacatatttttgtc aacaaggaaacacacttccctacaccttcggtgggggaacgaaattggaaattactggtggcggcgggtctggaggtggcggtag tggaggcgggggctctgaagtcaagcttcaagaatcaggtccgggacttgtagcgccaagtcagtctctttccgtaacttgtaccgtta gtggggtttccttgccagattacggagtaagttggattcgccaaccgcccagaaagggcttggaatggttgggtgttatctggggaag cgaaacaacatactataacagcgcactcaagtctcgcctcacgattattaaagataactctaaatctcaggtgttcctcaaaatgaac tcattgcaaacggacgacactgccatctattactgtgcaaaacactattactacggcggctcttacgctatggattactggggccagg gaacttcagtgacagtttctagtGGTTCTGGGGACGATAAGGATTGCGACGAGTACTGTAAGAAAACC AAGGAAacaaccacccctgcccctagacctccaaccccagcccctacaatcgccagccagcctctgtctctgaggcccgagg cttgtagaccagctgctggcggagccgtgcacaccagaggactggatttcgcctgcgacatctacatctgggcccctctggccggc acatgtggcgtgctgctgctgagcctcgtgatcaccctgtactgcaagcggggcagaaagaaactgctgtacatctttaagcagccc ttcatgcggcccgtgcagaccacccaggaagaggacggctgctcctgcagattccccgaggaagaagaaggcggctgcgagct gagagtgaagttcagcagatccgccgacgcccctgcctaccagcagggacagaaccagctgtacaacgagctgaacctgggca gacgggaagagtacgacgtgctggacaagcggagaggccgggatcctgaaatgggcggcaagcccagacggaagaacccc caggaaggcctgtataacgaactgcagaaagacaagatggccgaggcctacagcgagatcggaatgaagggcgagcggaga agaggcaagggccacgatggcctgtaccagggcctgagcaccgccaccaaggacacctatgacgccctgcacatgcaggcttt gccgcctaga |
| 20 | Exemplary anti-CD19 BCMA nucleic acid sequence (CD8 leader_Myc-Tag_3H1DA.1_anti-BCMA scFv_3H1DA.2_CD8 hinge_CD8 **TM_41BB_CD3ζ**) | gccctgcctgtgacagctctgctgctgcctctggccctgctgctgcatgccgccagacccgagcagaagctgatcagcgaggagg acctgGAGATTGCGGCATGCGAGGAGGAAATAGCGGCACTGGAGGCCAAGATCGCAGCAT TGAAAGAGGGGAGCGGTcaggtgcagctggtgcagagcggcgcggaagtgaaaaaaccgggcagcagcgtgaaa gtgagctgcaaagcgagcggctatacctttaccagctatgtgatgcattgggtgcgccaggcgccgggccagggcctggaatggat gggctatattattccgtataacgatgcgaccaaatataacgaaaaatttaaaggccgcgtgaccattaccgcggataaaagcacca gcaccgcgtatatggaactgagcagcctgcgcagcgaagataccgcggtgtattattgcgcgcgctataactatgatggctattttga tgtgtggggccagggcaccctggtgaccgtgagcagcggtggcggcgggtctggaggtggcggtagtggaggcgggggctctga aattgtgctgacccagagcccggcgaccctgagcctgagcccgggcgaacgcgcgaccctgagctgccgcgcgagccagagc attagcgattatctgcattggtatcagcagaaaccgggccaggcgccgcgcctgctgatttattatgcgagccagagcattaccggc attccggcgcgctttagcggcagcggcagcggcaccgattttaccctgaccattagcagcctggaaccggaagattttgcggtgtatt attgccagaacggccatagctttccgccgacctttggcggcggcaccaaagtggaaattaaaGGTTCTGGGAAGATCG CAGCCTGCAAGGCTAAGATTGCTGCTCTTAAAGCGGAGATCGCGGCGCTGGAAGCAacaa ccacccctgccccctagacctccaaccccagcccctacaatcgccagccagcctctgtctctgaggcccgaggcttgtagaccagct gctggcggagccgtgcacaccagaggactggatttcgcctgcgacatctacatctgggcccctctggccggcacatgtggcgtgct gctgctgagcctcgtgatcaccctgtactgcaagcggggcagaaagaaactgctgtacatctttaagcagcccttcatgcggcccgt gcagaccacccaggaagaggacggctgctcctgcagattccccgaggaagaagaaggcggctgcgagctgagagtgaagttc agcagatccgccgacgcccctgcctaccagcagggacagaaccagctgtacaacgagctgaacctgggcagacgggaagagt acgacgtgctggacaagcggagaggccgggatcctgaaatgggcggcaagcccagacggaagaacccccaggaaggcctgt ataacgaactgcagaaagacaagatggccgaggcctacagcgagatcggaatgaagggcgagcggagaagaggcaaggg ccacgatggcctgtaccagggcctgagcaccgccaccaaggacacctatgacgccctgcacatgcaggctttgccgcctaga |
| 21 | Exemplary anti-CD19 BCMA nucleic acid sequence (CD8 leader_Myc-Tag_3H2DA.1_anti-BCMA scFv_3H2DA.2_CD8 hinge_CD8 **TM_41BB_CD3ζ**) | gccctgcctgtgacagctctgctgctgcctctggccctgctgctgcatgccgccagacccgagcagaagctgatcagcgaggagg acctgGAGATTGCGGCTTGCGAAGAGGAGATAGCAGCCTTGGAAGCAAAGATTGCTGCGTG TAAAGAGGGCAGCGGTcaggtgcagctggtgcagagcggcgcggaagtgaaaaaaccgggcagcagcgtgaaagt gagctgcaaagcgagcggctatacctttaccagctatgtgatgcattgggtgcgccaggcgccgggccagggcctggaatggatg ggctatattattccgtataacgatgcgaccaaatataacgaaaaatttaaaggccgcgtgaccattaccgcggataaaagcaccag caccgcgtatatggaactgagcagcctgcgcagcgaagataccgcggtgtattattgcgcgcgctataactatgatggctattttgat gtgtggggccagggcaccctggtgaccgtgagcagcggtggcggcgggtctggaggtggcggtagtggaggcgggggctctga aattgtgctgacccagagcccggcgaccctgagcctgagcccgggcgaacgcgcgaccctgagctgccgcgcgagccagagc attagcgattatctgcattggtatcagcagaaaccgggccaggcgccgcgcctgctgatttattatgcgagccagagcattaccggc attccggcgcgctttagcggcagcggcagcggcaccgattttaccctgaccattagcagcctggaaccggaagattttgcggtgtatt attgccagaacggccatagctttccgccgacctttggcggcggcaccaaagtggaaattaaaGGCTCAGGCAAGATTG CTGCATGTAAAGCTAAGATTGCCGCGCTGAAAGCGGAGATAGCCGCGTGTGAGGAGacaa ccacccctgccccctagacctccaaccccagcccctacaatcgccagccagcctctgtctctgaggcccgaggcttgtagaccagct gctggcggagccgtgcacaccagaggactggatttcgcctgcgacatctacatctgggcccctctggccggcacatgtggcgtgct gctgctgagcctcgtgatcaccctgtactgcaagcggggcagaaagaaactgctgtacatctttaagcagcccttcatgcggcccgt gcagaccacccaggaagaggacggctgctcctgcagattccccgaggaagaagaaggcggctgcgagctgagagtgaagttc agcagatccgccgacgcccctgcctaccagcagggacagaaccagctgtacaacgagctgaacctgggcagacgggaagagt acgacgtgctggacaagcggagaggccgggatcctgaaatgggcggcaagcccagacggaagaacccccaggaaggcctgt ataacgaactgcagaaagacaagatggccgaggcctacagcgagatcggaatgaagggcgagcggagaagaggcaaggg ccacgatggcctgtaccagggcctgagcaccgccaccaaggacacctatgacgccctgcacatgcaggctttgccgcctaga |
| 22 | Exemplary anti-CD19 BCMA nucleic acid sequence (CD8 leader_Myc-Tag_3H1DP.1_anti-BCMA scFv_3H1DP.2_CD8 hinge_CD8 **TM_41BB_CD3ζ**) | gccctgcctgtgacagctctgctgctgcctctggccctgctgctgcatgccgccagacccgagcagaagctgatcagcgaggagg acctgGAGATCCAGCGCTGTGAGCAGGAGATCCGAAGACTTGAGCAAAAGATCGAGCGCC TGAAACGAGGATCAGGTcaggtgcagctggtgcagagcggcgcggaagtgaaaaaaccgggcagcagcgtgaaa gtgagctgcaaagcgagcggctatacctttaccagctatgtgatgcattgggtgcgccaggcgccgggccagggcctggaatggat gggctatattattccgtataacgatgcgaccaaatataacgaaaaatttaaaggccgcgtgaccattaccgcggataaaagcacca gcaccgcgtatatggaactgagcagcctgcgcagcgaagataccgcggtgtattattgcgcgcgctataactatgatggctattttga tgtgtggggccagggcaccctggtgaccgtgagcagcggtggcggcgggtctggaggtggcggtagtggaggcgggggctctga aattgtgctgacccagagcccggcgaccctgagcctgagcccgggcgaacgcgcgaccctgagctgccgcgcgagccagagc attagcgattatctgcattggtatcagcagaaaccgggccaggcgccgcgcctgctgatttattatgcgagccagagcattaccggc attccggcgcgctttagcggcagcggcagcggcaccgattttaccctgaccattagcagcctggaaccggaagattttgcggtgtatt attgccagaacggccatagctttccgccgacctttggcggcggcaccaaagtggaaattaaaGGAAGTGGCAAAATTG AAAGATGTAAGGAGAAGATCGAGCGGCTGAAAGAAGATCCAAAGACTTGAGCAGacaa ccacccctgcccctagacctccaaccccagcccctacaatcgccagccagcctctgtctctgaggcccgaggcttgtagaccagct gctggcggagccgtgcacaccagaggactggatttcgcctgcgacatctacatctgggcccctctggccggcacatgtggcgtgct gctgctgagcctcgtgatcaccctgtactgcaagcggggcagaaagaaactgctgtacatctttaagcagcccttcatgcggcccgt gcagaccacccaggaagaggacggctgctcctgcagattccccgaggaagaagaaggcggctgcgagctgagagtgaagttc agcagatccgccgacgcccctgcctaccagcagggacagaaccagctgtacaacgagctgaacctgggcagacggaagagt acgacgtgctggacaagcggagaggccgggatcctgaaatgggcggcaagcccagacggaagaacccccaggaaggcctgt ataacgaactgcagaaagacaagatggccgaggcctacagcgagatcggaatgaagggcgagcggagaagaggcaaggg ccacgatggcctgtaccagggcctgagcaccgccaccaaggacacctatgacgccctgcacatgcaggctttgccgcctaga |
| 23 | Human CD19 (UniProt: P15391-1) | |
| | | |
| 24 | Human CD19 (UniProt: P15391-2) | MPPPRLLFFLLFLTPMEVRPEEPLVVKVEEGDNAVLQCLKGTSDGPTQQLTWSRESPLKPFL KLSLGLPGLGIHMRPLAIWLFIFNVSQQMGGFYLCQPGPPSEKAWQPGWTVNVEGSGELFR WNVSDLGGLGCGLKNRSSEGPSSPSGKLMSPKLYVWAKDRPEIWEGEPPCLPPRDSLNQSL SQDLTMAPGSTLWLSCGVPPDSVSRGPLSWTHVHPKGPKSLLSLELKDDRPARDMWVMET GLLLPRATAQDAGKYYCHRGNLTMSFHLEITARPVLWHWLLRTGGWKVSAVTLAYLIFCLCSL VGILHLQRALVLRRKRKRMTDPTRRFFKVTPPPGSGPQNQYGNVLSLPTPTSGLGRAQRWA AGLGGTAPSYGNPSSDVQADGALGSRSPPGVGPEEEEGEGYEEPDSEEDSEFYENDSNLG QDQLSQDGSGYENPEDEPLGPEDEDSFSNAESYENEDEELTQPVARTMDFLSPHGSAWDP SREATSLAGSQSYEDMRGILYAAPQLRSIRGQPGPNHEEDADSYENMDNPDGPDPAWGGG GRMGTWSTR |
| 25 | Human CD19 (UniProt: P15391-1) signal domain | MPPPRLLFFLLFLTPMEVR |
| 26 | Human CD19 (UniProt: P15391-1) extracellular domain | PEEPLVVKVEEGDNAVLQCLKGTSDGPTQQLTWSRESPLKPFLKLSLGPLGIHMRPLAIWL FIFNVSQQMGGFYLCQPGPPSEKAWQPGWTVNVEGSGELFRWNVSDLGGLGCGLKNRSSE GPSSPSGKLMSPKLYVWAKDRPEIWEGEPPCLPPRDSLNQSLSQDLTMAPGSTLWLSCGVP PDSVSRGPLSWTHVHPKGPKSLLSLELKDDRPARDMWVMETGLLLPRATAQDAGKYYCHRG NLTMSFHLEITARPVLWHWLLRTGGWK |
| 27 | Human CD19 (UniProt: P15391-1) transmembrane domain | VSAVTLAYLIFCLCSLVGILHL |
| 28 | Human CD19 (UniProt: P15391-1) cytoplasmic domain | QRALVLRRKRKRMTDPTRRFFKVTPPPGSGPQNQYGNVLSLPTPTSGLGRAQRWAAGLGG TAPSYGNPSSDVQADGALGSRSPPGVGPEEEEGEGYEEPDSEEDSEFYENDSNLGQDQLS QDGSGYENPEDEPLGPEDEDSFSNAESYENEDEELTQPVARTMDFLSPHGSAWDPSREATS LGSQSYEDMRGILYAAPQLRSIRGQPGPNHEEDADSYENMDNPDGPDPAWGGGGRMGTW STR |
| 29 | Human BCMA (UniProt: Q02223-1) | MLQMAGQCSQNEYFDSLLHACIPCQLRCSSNTPPLTCQRYCNASVTNSVKGTNAILWTCLGL SLIISLAVFVLMFLLRKINSEPLKDEFKNTGSGLLGMANIDLEKSRTGDEIILPRGLEYTVEECTC EDCIKSKPKVDSDHCFPLPAMEEGATILVTTKTNDYCKSLPAALSATEIEKSISAR |
| 30 | Human BCMA (UniProt: Q02223-2) | MLQMAGQCSQNEYFDSLLHACIPCQLRCSSNTPPLTCQRYCNARSGLLGMANIDLEKSRTGD EIILPRGLEYTVEECTCEDCIKSKPKVDSDHCFPLPAMEEGATILVTTKTNDYCKSLPAALSATE IEKSISAR |
| 31 | Human BCMA (UniProt: Q02223-1) extracellular domain | MLQMAGQCSQNEYFDSLLHACIPCQLRCSSNTPPLTCQRYCNASVTNSVKGTNA |
| 32 | Human BCMA (UniProt: Q02223-1) transmembrane domain | ILWTCLGLSLIISLAVFVLMFLL |
| 33 | Human BCMA (UniProt: Q02223-1) cytoplasmic domain | RKINSEPLKDEFKNTGSGLLGMANIDLEKSRTGDEIILPRGLEYTVEECTCEDCIKSKPKVDSD HCFPLPAMEEGATILVTTKTNDYCKSLPAALSATEIEKSISAR |
| 34 | Linker sequence 1 | GGGS |
| 35 | Linker sequence 2 | GGGGS |
| 36 | Linker sequence 3 | GGGSG |
| 37 | Linker sequence 4 | GGGGSG |
| 38 | Linker sequence 5 | GGGGSGGGGSGGGGS |
| 39 | Linker sequence 6 | GGGGSGGGGSGGGGSGGGGS |
| 40 | Linker sequence 7 | GGGGSGGGGSGGGGSGGGGSGGGGSGGGGS |
| 41 | **CD8α hinge** | TTTPAPRPPTPAPTIASQPLSLRPEACRPAAGGAVHTRGLDFACDIY |
| 42 | **CD8α transmembrane** domain | IWAPLAGTCGVLLLSLVITLYC |
| 43 | ITAM consensus | YXXL/I |
| | | wherein X = any amino acid |
| 44 | Larger ITAM consensus | YXXL/I(X)₆₋₈YXXL/I |
| | | wherein X = any amino acid |
| 45 | Human 4-1BB intracellular domain | KRGRKKLLYIFKQPFMRPVQTTQEEDGCSCRFPEEEEGGCEL |
| 46 | **Human CD3ζ** intracellular domain | RVKFSRSADAPAYQQGQNQLYNELNLGRREEYDVLDKRRGRDPEMGGKPRRKNPQEGLYN ELQKDKMAEAYSEIGMKGERRRGKGHDGLYQGLSTATKDTYDALHMQALPPR |
| 47 | Myc tag | EQKLISEEDL |
| 48 | CD8 leader | ALPVTALLLPLALLLHAARP |
| 49 | Exemplary anti-CD19 CAR amino acid sequence (3H1DA.1_anti-CD19 scFv_3H1DA.2_CD8 hinge_CD8 **TM_41BB_CD3ζ)** | EIAACEEEIAALEAKIAALKEGSGDIQMTQTTSSLSASLGDRVTISCRASQDISKYLNWYQQKP DGTVKLLIYHTSRLHSGVPSRFSGSGSGTDYSLTISNLEQEDIATYFCQQGNTLPYTFGGGTK LEITGGGGSGGGGSGGGGSEVKLQESGPGLVAPSQSLSVTCTVSGVSLPDYGVSWIRQPPR KGLEWLGVIWGSETTYYNSALKSRLTIIKDNSKSQVFLKMNSLQTDDTAIYYCAKHYYYGGSY AMDYWGQGTSVTVSSGSGKIAACKAKIAALKAEIAALEATTTPAPRPPTPAPTIASQPLSLRPE ACRPAAGGAVHTRGLDFACDIYIWAPLAGTCGVLLLSLVITLYCKRGRKKLLYIFKQPFMRPVQ TTQEEDGCSCRFPEEEEGGCELRVKFSRSADAPAYQQGQNQLYNELNLGRREEYDVLDKRR GRDPEMGGKPRRKNPQEGLYNELQKDKMAEAYSEIGMKGERRRGKGHDGLYQGLSTATKD TYDALHMQALPPR |
| 50 | Exemplary anti-CD19 CAR amino acid sequence (3H2DA.1_anti-CD19 scFv_3H2DA.2_CD8 hinge_CD8 **TM_41BB_CD3ζ)** | EIAACEEEIAALEAKIAACKEGSGDIQMTQTTSSLSASLGDRVTISCRASQDISKYLNWYQQKP DGTVKLLIYHTSRLHSGVPSRFSGSGSGTDYSLTISNLEQEDIATYFCQQGNTLPYTFGGGTK LEITGGGGSGGGGSGGGGSEVKLQESGPGLVAPSQSLSVTCTVSGVSLPDYGVSWIRQPPR KGLEWLGVIWGSETTYYNSALKSRLTIIKDNSKSQVFLKMNSLQTDDTAIYYCAKHYYYGGSY AMDYWGQGTSVTVSSGSGKIAACKAKIAALKAEIAACEETTTPAPRPPTPAPTIASQPLSLRPE ACRPAAGGAVHTRGLDFACDIYIWAPLAGTCGVLLLSLVITLYCKRGRKKLLYIFKQPFMRPVQ TTQEEDGCSCRFPEEEEGGCELRVKFSRSADAPAYQQGQNQLYNELNLGRREEYDVLDKRR GRDPEMGGKPRRKNPQEGLYNELQKDKMAEAYSEIGMKGERRRGKGHDGLYQGLSTATKD TYDALHMQALPPR |
| 51 | Exemplary anti-CD19 CAR amino acid sequence (3H1DP.1_anti-CD19 scFv_3H1DP.2_CD8 hinge_CD8 **TM_41BB_CD3ζ)** | EIQRCEQEIRRLEQKIERLKRGSGDIQMTQTTSSLSASLGDRVTISCRASQDISKYLNWYQQKP DGTVKLLIYHTSRLHSGVPSRFSGSGSGTDYSLTISNLEQEDIATYFCQQGNTLPYTFGGGTK LEITGGGGSGGGGSGGGGSEVKLQESGPGLVAPSQSLSVTCTVSGVSLPDYGVSWIRQPPR KGLEWLGVIWGSETTYYNSALKSRLTIIKDNSKSQVFLKMNSLQTDDTAIYYCAKHYYYGGSY AMDYWGQGTSVTVSSGSGKIERCKEKIERLKEEIQRLEQTTTPAPRPPTPAPTIASQPLSLRP EACRPAAGGAVHTRGLDFACDIYIWAPLAGTCGVLLLSLVITLYCKRGRKKLLYIFKQPFMRPV QTTQEEDGCSCRFPEEEEGGCELRVKFSRSADAPAYQQGQNQLYNELNLGRREEYDVLDK |
| | | RRGRDPEMGGKPRRKNPQEGLYNELQKDKMAEAYSEIGMKGERRRGKGHDGLYQGLSTAT KDTYDALHMQALPPR |
| 52 | Exemplary anti-CD19 CAR amino acid sequence (HD1.1_anti-CD19 scFv_HD1.2_CD8 hinge_CD8 **TM_41BB_CD3ζ)** | ECTGHIEGPTCTCHCKGSGDIQMTQTTSSLSASLGDRVTISCRASQDISKYLNWYQQKPDGT VKLLIYHTSRLHSGVPSRFSGSGSGTDYSLTISNLEQEDIATYFCQQGNTLPYTFGGGTKLEIT GGGGSGGGGSGGGGSEVKLQESGPGLVAPSQSLSVTCTVSGVSLPDYGVSWIRQPPRKGL EWLGVIWGSETTYYNSALKSRLTIIKDNSKSQVFLKMNSLQTDDTAIYYCAKHYYYGGSYAMD YWGQGTSVTVSSGSGDDKDCDEYCKKTKETTTPAPRPPTPAPTIASQPLSLRPEACRPAAG GAVHTRGLDFACDIYIWAPLAGTCGVLLLSLVITLYCKRGRKKLLYIFKQPFMRPVQTTQEEDG CSCRFPEEEEGGCELRVKFSRSADAPAYQQGQNQLYNELNLGRREEYDVLDKRRGRDPEM GGKPRRKNPQEGLYNELQKDKMAEAYSEIGMKGERRRGKGHDGLYQGLSTATKDTYDALH MQALPPR |
| 53 | Exemplary anti-BCMA CAR amino acid sequence (3H1DA.1_anti-BCMA scFv_3H1DA.2_CD8 hinge_CD8 **TM_41BB_CD3ζ)** | EIAACEEEIAALEAKIAALKEGSGQVQLVQSGAEVKKPGSSVKVSCKASGYTFTSYVMHWVR QAPGQGLEWMGYIIPYNDATKYNEKFKGRVTITADKSTSTAYMELSSLRSEDTAVYYCARYNY DGYFDVWGQGTLVTVSSGGGGSGGGGSGGGGSEIVLTQSPATLSLSPGERATLSCRASQSI SDYLHWYQQKPGQAPRLLIYYASQSITGIPARFSGSGSGTDFTLTISSLEPEDFAVYYCQNGH SFPPTFGGGTKVEIKGSGKIAACKAKIAALKAEIAALEATTTPAPRPPTPAPTIASQPLSLRPEA CRPAAGGAVHTRGLDFACDIYIWAPLAGTCGVLLLSLVITLYCKRGRKKLLYIFKQPFMRPVQT TQEEDGCSCRFPEEEEGGCELRVKFSRSADAPAYQQGQNQLYNELNLGRREEYDVLDKRR GRDPEMGGKPRRKNPQEGLYNELQKDKMAEAYSEIGMKGERRRGKGHDGLYQGLSTATKD TYDALHMQALPPR |
| 54 | Exemplary anti-BCMA CAR amino acid sequence (3H2DA.1_anti- BCMA scFv_3H2DA.2_CD8 hinge_CD8 **TM_41BB_CD3ζ)** | EIAACEEEIAALEAKIAACKEGSGQVQLVQSGAEVKKPGSSVKVSCKASGYTFTSYVMHWVR QAPGQGLEWMGYIIPYNDATKYNEKFKGRVTITADKSTSTAYMELSSLRSEDTAVYYCARYNY DGYFDVWGQGTLVTVSSGGGGSGGGGSGGGGSEIVLTQSPATLSLSPGERATLSCRASQSI SDYLHWYQQKPGQAPRLLIYYASQSITGIPARFSGSGSGTDFTLTISSLEPEDFAVYYCQNGH SFPPTFGGGTKVEIKGSGKIAACKAKIAALKAEIAACEETTTPAPRPPTPAPTIASQPLSLRPEA CRPAAGGAVHTRGLDFACDIYIWAPLAGTCGVLLLSLVITLYCKRGRKKLLYIFKQPFMRPVQT TQEEDGCSCRFPEEEEGGCELRVKFSRSADAPAYQQGQNQLYNELNLGRREEYDVLDKRR GRDPEMGGKPRRKNPQEGLYNELQKDKMAEAYSEIGMKGERRRGKGHDGLYQGLSTATKD TYDALHMQALPPR |
| 55 | Exemplary anti-BCMA CAR amino acid sequence (3H1DP.1_anti- BCMA scFv_3H1DP.2_CD8 hinge_CD8 **TM_41BB_CD3ζ)** | EIQRCEQEIRRLEQKIERLKRGSGQVQLVQSGAEVKKPGSSVKVSCKASGYTFTSYVMHWVR QAPGQGLEWMGYIIPYNDATKYNEKFKGRVTITADKSTSTAYMELSSLRSEDTAVYYCARYNY DGYFDVWGQGTLVTVSSGGGGSGGGGSGGGGSEIVLTQSPATLSLSPGERATLSCRASQSI SDYLHWYQQKPGQAPRLLIYYASQSITGIPARFSGSGSGTDFTLTISSLEPEDFAVYYCQNGH SFPPTFGGGTKVEIKGSGKIERCKEKIERLKEEIQRLEQTTTPAPRPPTPAPTIASQPLSLRPEA CRPAAGGAVHTRGLDFACDIYIWAPLAGTCGVLLLSLVITLYCKRGRKKLLYIFKQPFMRPVQT TQEEDGCSCRFPEEEEGGCELRVKFSRSADAPAYQQGQNQLYNELNLGRREEYDVLDKRR GRDPEMGGKPRRKNPQEGLYNELQKDKMAEAYSEIGMKGERRRGKGHDGLYQGLSTATKD TYDALHMQALPPR |
| 56 | Control anti-CD19 CAR amino acid sequence (CD8 leader_Myc-Tag_anti-CD19 scFv_CD8 hinge_CD8 **TM_41BB_CD3ζ)** | ALPVTALLLPLALLLHAARPEQKLISEEDLDIQMTQTTSSLSASLGDRVTISCRASQDISKYLNW YQQKPDGTVKLLIYHTSRLHSGVPSRFSGSGSGTDYSLTISNLEQEDIATYFCQQGNTLPYTF GGGTKLEITGGGGSGGGGSGGGGSEVKLQESGPGLVAPSQSLSVTCTVSGVSLPDYGVSWI RQPPRKGLEWLGVIWGSETTYYNSALKSRLTIIKDNSKSQVFLKMNSLQTDDTAIYYCAKHYY YGGSYAMDYWGQGTSVTVSSTTTPAPRPPTPAPTIASQPLSLRPEACRPAAGGAVHTRGLD FACDIYIWAPLAGTCGVLLLSLVITLYCKRGRKKLLYIFKQPFMRPVQTTQEEDGCSCRFPEEE EGGCELRVKFSRSADAPAYQQGQNQLYNELNLGRREEYDVLDKRRGRDPEMGGKPRRKNP QEGLYNELQKDKMAEAYSEIGMKGERRRGKGHDGLYQGLSTATKDTYDALHMQALPPR |
| 57 | Control anti-BCMA CAR amino acid sequence (CD8 leader_Myc-Tag_anti- BCMA scFv_CD8 hinge_CD8 **TM_41BB_CD3ζ)** | ALPVTALLLPLALLLHAARPEQKLISEEDLQVQLVQSGAEVKKPGSSVKVSCKASGYTFTSYV MHWVRQAPGQGLEWMGYIIPYNDATKYNEKFKGRVTITADKSTSTAYMELSSLRSEDTAVYY CARYNDGYFDVWGQGTLVTVSSGGGGSGGGGSGGGGSEIVLTQSPATLSLSPGERATLS CRASQSISDYLHWYQQKPGQAPRLLIYYASQSITGIPARFSGSGSGTDFTLTISSLEPEDFAVY YCQNGHSFPPTFGGGTKVEIKTTTPAPRPPTPAPTIASQPLSLRPEACRPAAGGAVHTRGLDF ACDIYIWAPLAGTCGVLLLSLVITLYCKRGRKKLLYIFKQPFMRPVQTTQEEDGCSCRFPEEEE GGCELRVKFSRSADAPAYQQGQNQLYNELNLGRREEYDVLDKRRGRDPEMGGKPRRKNPQ EGLYNELQKDKMAEAYSEIGMKGERRRGKGHDGLYQGLSTATKDTYDALHMQALPPR |
| 58 | Anti-CD19 VH | EVKLQESGPGLVAPSQSLSVTCTVSGVSLPDYGVSWIRQPPRKGLEWLGVIWGSETTYYNSA LKSRLTIIKDNSKSQVFLKMNSLQTDDTAIYYCAKHYYYGGSYAMDYWGQGTSVTVSS |
| 59 | Anti-CD19 HC-CDR1 | GVSLPDYG |
| 60 | Anti-CD19 HC-CDR2 | IWGSETT |
| 61 | Anti-CD19 HC-CDR3 | AKHYYYGGSYAMDY |
| 62 | Anti-CD19 VL | DIQMTQTTSSLSASLGDRVTISCRASQDISKYLNWYQQKPDGTVKLLIYHTSRLHSGVPSRFS GSGSGTDYSLTISNLEQEDIATYFCQQGNTLPYTFGGGTKLEIT |
| 63 | Anti-CD19 LC-CDR1 | QDISKY |
| 64 | Anti-CD19 LC-CDR2 | HTS |
| 65 | Anti-CD19 LC-CDR3 | QQGNTLPYT |
| 66 | Anti-CD19 scFv | DIQMTQTTSSLSASLGDRVTISCRASQDISKYLNWYQQKPDGTVKLLIYHTSRLHSGVPSRFS GSGSGTDYSLTISNLEQEDIATYFCQQGNTLPYTFGGGTKLEITGGGGSGGGGSGGGGSEVK LQESGPGLVAPSQSLSVTCTVSGVSLPDYGVSWIRQPPRKGLEWLGVIWGSETTYYNSALKS RLTIIKDNSKSQVFLKMNSLQTDDTAIYYCAKHYYYGGSYAMDYWGQGTSVTVSS |
| 67 | Anti-BCMA VH | QVQLVQSGAEVKKPGSSVKVSCKASGYTFTSYVMHWVRQAPGQGLEWMGYIIPYNDATKYN EKFKGRVTITADKSTSTAYMELSSLRSEDTAVYYCARYNYDGYFDVWGQGTLVTVSS |
| 68 | Anti-BCMA HC-CDR1 | GYTFTSYV |
| 69 | Anti-BCMA HC-CDR2 | IIPYNDAT |
| 70 | Anti-BCMA HC-CDR3 | ARYNYDGYFDV |
| 71 | Anti-BCMA VL | EIVLTQSPATLSLSPGERATLSCRASQSISDYLHWYQQKPGQAPRLLIYYASQSITGIPARFSG SGSGTDFTLTISSLEPEDFAVYYCQNGHSFPPTFGGGTKVEIKGSG |
| 72 | Anti-BCMA LC-CDR1 | QSISDY |
| 73 | Anti-BCMA LC-CDR2 | YAS |
| 74 | Anti-BCMA LC-CDR3 | QNGHSFPPT |
| 75 | Anti-BCMA scFv | QVQLVQSGAEVKKPGSSVKVSCKASGYTFTSYVMHWVRQAPGQGLEWMGYIIPYNDATKYN EKFKGRVTITADKSTSTAYMELSSLRSEDTAVYYCARYNYDGYFDVWGQGTLVTVSSGGGG SGGGGSGGGGSEIVLTQSPATLSLSPGERATLSCRASQSISDYLHWYQQKPGQAPRLLIYYA SQSITGIPARFSGSGSGTDFTLTISSLEPEDFAVYYCQNGHSFPPTFGGGTKVEIKGSG |

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

### Examples

### EXAMPLE 1 - Design and preparation of DNA constructs

Standard molecular biology techniques were used to prepare DNA constructs encoding disulfide-stabilized CARs. These techniques involved the polymerase chain reaction (PCR), agarose gel electrophoresis of DNA, isolation of DNA fragments from gels, nucleic acid concentration determination, chemical DNA synthesis, digestion with restriction enzymes, ligation of DNA fragments, Gibson assembly, chemical transformation of competent E. coli cells, plasmid DNA isolation and large-scale plasmid DNA purification. These methods are widely known to those skilled in the field and are thoroughly detailed in molecular biology handbooks.

All plasmids used for transfection into the Lenti-X 293T cell line were chemically transformed into E. coli NEB stable bacteria and isolated using the GeneJet Plasmid DNA Isolation Kit (Invitrogen).

The following CAR molecules were produced, and analysed in the subsequent examples:

| CAR ID | First interaction domain | Second interaction domain | CAR sequence |
|---|---|---|---|
| CD19 3H1DA | 3H1 DA.1 | 3H1DA.2 | SEQ ID NO:9 |
| CD19 3H2DA | 3H2DA.1 | 3H2DA.2 | SEQ ID NO:10 |
| CD19 3H1DP | 3H1DP.1 | 3H1DP.2 | SEQ ID NO:11 |
| CD19 HD1 | HD1.1 | HD1.2 | SEQ ID NO:12 |
| Control anti-CD19 CAR | - | - | SEQ ID NO:56 |
| BCMA 3H1DA | 3H1DA.1 | 3H1DA.2 | SEQ ID NO:13 |
| BCMA 3H2DA | 3H2DA.1 | 3H2DA.2 | SEQ ID NO:14 |
| BCMA 3H1DP | 3H1DP.1 | 3H1DP.2 | SEQ ID NO:15 |
| Control anti-BCMA CAR | - | - | SEQ ID NO:57 |

All of the CARs in the above table have the same hinge, transmembrane domain, and signalling domain.

All anti-CD19 CARs in the above table have the same anti-CD19 antigen-binding domains, and all anti-BCMA CARs in the above table have the same anti-BCMA antigen-binding domains.

### EXAMPLE 2 - Construction of CAR T Cells

Viral Production: Lentiviral particles were generated by transient transfection of plasmids into Lenti-X 293T cells. The cells were seeded at a density of 5 × 10⁸ cells per plate. After 24 hours, the cells were transfected with a mixture consisting of 10 µg of the pCDH vector encoding the protein of interest, 5 µg of the pMDLg/pRRe packaging vector (12). 2.5 µg of the VSV-G envelope vector (12), and 5 µg of the pRSV-Rev vector (12) using polyethylenimine MAX (PEl MAX). Forty-eight hours after transfection, the supernatant containing the lentiviral particles was collected and filtered through a 0.45-µm filter. The filtered supernatant was then ultracentrifuged at 4°C for 2 hours at 100,000g using ultracentrifuge tubes. The resulting viral pellet was resuspended in phosphate-buffered saline (PBS) and stored at -80°C until further use.

CAR T Cell Construction: Human T cells from multiple healthy donors were used in this process, and all procedures were conducted in compliance with ethical and biosafety standards. Peripheral blood mononuclear cells (PBMCs) were isolated from healthy donor buffy coats using Lymphoprep density gradient centrifugation. CD3+ T cells were isolated by negative selection with the Pan T Cell Isolation Kit (Miltenyi Biotec). The isolated T cells were cultured in RPMI medium supplemented with 10% heat-inactivated fetal bovine serum (FBS), 25 µL/mL ImmunoCult Human CD3/CD28 T cell activator, and 50 U/mL human recombinant IL-2 for 48 hours prior to transduction.

T cells were transduced with lentiviral particles at a multiplicity of infection (MOI) of 10-15. After transduction, the T cells were cultured and expanded for at least 5-10 days in the presence of 50 U/mL human recombinant IL-2. The efficiency of CAR expression was evaluated by flow cytometry, measuring Myc-tag expression on the cell surface.

### EXAMPLE 3 - In Vitro Cytotoxicity Assays

The cytotoxicity of stabilized CARs was assessed against the target antigens CD19 and BCMA to evaluate their effectiveness in comparison to the second-generation wild-type CAR. RAJI-FLuc cells expressing CD19 and BCMA antigens were used as the target cancer cell line.

CAR T cells were washed to remove human IL-2, then resuspended in RPMI medium and co-cultured with RAJI-FLuc cells at the specified effector-to-target (E:T) ratios. After 24 hours, bioluminescence imaging (BLI) was performed using the IVIS Lumina Series III system. Prior to measurement, D-luciferin was added to the co-cultures at a final concentration of 150 µg/mL. Data analysis was performed using Living Image 4.5.2 software. The percentage of specific target cell lysis was calculated from the average radiance values (ARV) using the formula: % specific lysis = 100 × (spontaneous death ARV - test ARV) / (spontaneous death ARV - background ARV).

Results: The cytotoxicity of stabilized CAR T cells was assessed in comparison to standard CAR T cells (Figure 4). The results indicate that CAR T cells incorporating coiled-coil peptide heterodimers with cysteine residues demonstrated superior cytotoxicity compared to wild-type CAR T cells. These coiled-coil heterodimers were designed to form either one (3H1DA) or two (3H2DA) disulfide bonds, both of which enhanced CAR T cell-mediated killing of CD19-expressing target cells. Disulfide-stabilized CAR T cells also showed superior performance when the coiled-coil interaction domains were modified to include polar and charged amino acid residues (3H1DP). The HD1 heterodimer also enhanced the cytotoxic activity of CAR T cells against CD19-postiive RAJI cells (Figure 7).

Additionally, coiled-coil-stabilized CAR T cells targeting BCMA exhibited greater tumor cell killing compared to control CAR T cells, reinforcing the effectiveness of this modification.

These findings highlight that the incorporation of peptide heterodimers with cysteine residues enhances CAR T cell cytotoxicity across multiple antigen targets.

### EXAMPLE 4 - Cytokine Production Analysis

The secretion of cytokines (IL-2, IFN-γ) was measured to compare the immune activation of CAR T cells containing a coiled-coil peptide heterodimer with that of wild-type CAR T cells.

Supernatants from co-cultures of CAR T cells and target cells, previously utilized in cytotoxicity assays, were collected following centrifugation at 1800 rpm for 5 minutes to pellet the cells. The concentrations of human IL-2 and IFN-γ in the supernatants were measured using commercially available ELISA kits (Thermo Fisher Scientific), following the manufacturer's protocols. ELISA washing steps were carried out with a HydroSpeed plate washer (Tecan), and absorbance was recorded using a SynergyMx microplate reader (BioTek). Endpoint readings were analyzed to determine cytokine concentrations.

Results: Cytokine release was evaluated following CAR T cell stimulation with CD19-expressing target cells (Figure 5). Disulfide-stabilized CAR T cells (3H1DA, 3H1DP) exhibited higher IL-2 and IFN-γ secretion than wild-type CAR T cells, which correlates with the enhanced cytotoxic activity observed in cytotoxicity assays, indicating stronger immune activation.

### EXAMPLE 5 - Animal Cancer Model Studies

The therapeutic efficacy of disulfide-stabilized CAR T cells was assessed in vivo using a xenograft mouse model of human lymphoma.

NOD.CB17-Prkdcscid IL2rgtm1/BcgenHsd mice, aged 8 to 12 weeks, were bred and maintained under controlled conditions, including a 12-hour light/dark cycle, relative humidity of 40-60%, and temperatures ranging from 20-24°C. The animals were housed in individually ventilated cages (IVCs) with access to standard chow and tap water ad libitum. Health certificates were provided for all animals, with their microbiological status confirmed via the FELASA-recommended Mouse Vivum panel (QM Diagnostics).

For the in vivo study, tumor xenografts were established by intravenously injecting 1 × 10⁸ RAJI-FLuc cells per mouse. On day 3 post-injection, CAR T cells were administered intravenously at a dose of 5 × 10⁶ cells per mouse. Tumor burden was monitored weekly using bioluminescent imaging. Prior to imaging, mice were injected intraperitoneally with D-luciferin (150 mg/kg; Sigma) and anesthetized with isoflurane inhalation. Bioluminescent imaging was performed 10 minutes after luciferin injection using the IVIS Lumina Series III system (PerkinElmer), with data acquisition and analysis conducted via Living Image 4.5.2 software (PerkinElmer). Mice were humanely sacrificed at a designated experimental endpoint or when ethical criteria were met, with relevant data collected for analysis.

Results: The Kaplan-Meier survival curve indicates that mice treated with disulfide-stabilized CAR T cells demonstrated prolonged survival compared to the control group (Figure 6A). In this experiment, each group consisted of six mice, and the duration of the experiment was 7 weeks. All six mice in the CD19 3H2DA group survived, five mice in the CD19 3H1DA group survived, and three mice survived in the CD19 3H1DP group. The clinically approved CD19 wild-type CAR prolonged the survival of mice compared to those treated with mock T cells; however, no mice survived until week 7, demonstrating clear therapeutic benefit of the interaction domain disulfide-stabilized CARs. Tumor burden was quantified by measuring total flux (photons/second) (Figure 6B). Coiled-coil-stabilized CAR T cells exhibited superior tumor clearance over time, as evidenced by a reduced bioluminescence signal relative to the control CAR T cells.

These results confirm that disulfide-stabilized CAR T cells effectively inhibit tumor growth and enhance the survival of mice. The 3H2DA CAR construct, stabilized by two disulfide bonds, was the most effective in prolonging survival, followed by the 3H1DA CAR construct with one disulfide bond, and then the 3H1DP CAR, which contains one disulfide bond and modified coiled-coil-forming segments with charged amino acid residues.

### References

A number of publications are cited above in order to more fully describe and disclose the invention and the state of the art to which the invention pertains. Full citations for a number of key references are provided below. The entirety of each of these references is incorporated herein by reference in their entirety.
1. Duan Y, Chen R, Huang Y, Meng X, Chen J, Liao C, et al. Tuning the ignition of CAR: optimizing the affinity of scFv to improve CAR-T therapy. Vol. 79, Cellular and Molecular Life Sciences. Springer Science and Business Media Deutschland GmbH; 2022.
2. Zheng Z, Li S, Liu M, Chen C, Zhang L, Zhou D. Fine-Tuning through Generations: Advances in Structure and Production of CAR-T Therapy. Vol. 15, Cancers. Multidisciplinary Digital Publishing Institute (MDPI); 2023.
3. Jayaraman J, Mellody MP, Hou AJ, Desai RP, Fung AW, Pham AHT, et al. CAR-T design: Elements and their synergistic function. Vol. 58, EBioMedicine. Elsevier B.V.; 2020.
4. Sadelain M, Brentjens R, Rivière I. The promise and potential pitfalls of chimeric antigen receptors. Vol. 21, Current Opinion in Immunology. 2009. p. 215-23.
5. MOzes G, Sipos F. CAR-Based Therapy for Autoimmune Diseases: A Novel Powerful Option. Vol. 12, Cells. MDPI; 2023.
6. Basu R, Whitlock BM, Husson J, Le Floc'h A, Jin W, Oyler-Yaniv A, et al. Cytotoxic T Cells Use Mechanical Force to Potentiate Target Cell Killing. Cell. 2016 Mar 24;165(1):100-10.
7. Gezehagn Kussia G, Tessema TS. The Potential of Single-Chain Variable Fragment Antibody: Role in Future Therapeutic and Diagnostic Biologics. Vol. 2024, Journal of Immunology Research. Hindawi Limited; 2024.
8. Kang TH, Seong BL. Solubility, Stability, and Avidity of Recombinant Antibody Fragments Expressed in Microorganisms. Vol. 11, Frontiers in Microbiology. Frontiers Media S.A.; 2020.
9. Zhao JX, Yang L, Gu ZN, Chen HQ, Tian FW, Chen YQ, et al. Stabilization of the single-chain fragment variable by an interdomain disulfide bond and its effect on antibody affinity. Int J Mol Sci. 2011 Jan;12(1):1-11.
10. Cai Y, Zhang J, Lao X, Jiang H, Yu Y, Deng Y, et al. Construction of a disulfide-stabilized diabody against fibroblast growth factor-2 and the inhibition activity in targeting breast cancer. Cancer Sci. 2016 Aug 1;107(8):1141-50.
11. Brinkmann U, Reiter Y, Jung SH, Lee B, Pastan I. A recombinant immunotoxin containing a disulfide-stabilized Fv fragment. Vol. 90, Proc. Natl. Acad. Sci. USA. 1993.
12. Dull T, Zufferey R, Kelly M, Mandel RJ, Nguyen M, Trono D, et al. A Third-Generation Lentivirus Vector with a Conditional Packaging System. Vol. 72, JOURNAL OF VIROLOGY. 1998.

For standard molecular biology techniques, see Sambrook, J., Russel, D.W. Molecular Cloning, A Laboratory Manual. 3 ed. 2001, Cold Spring Harbor, New York: Cold Spring Harbor Laboratory Press

## Claims

1. A polypeptide comprising (i) a first interaction domain comprising a cysteine, (ii) an antigen-binding domain which binds specifically to a target antigen, and (ii) a second interaction domain comprising a cysteine, wherein the first interaction domain and the second interaction domain interact through a disulfide bond between cysteine residues.

2. A chimeric antigen receptor (CAR) comprising:
(i) a first interaction domain comprising a cysteine,
(ii) an antigen-binding domain which binds specifically to a target antigen,
(iii) a second interaction domain comprising a cysteine,
(iv) a hinge,
(v) a transmembrane domain, and
(vi) a signalling domain,
wherein the first interaction domain and the second interaction domain interact through a disulfide bond between cysteine residues.

3. The polypeptide according to claim 1, or a CAR according to claim 2, wherein the first interaction domain comprises a coiled-coil peptide, and the second interaction domain comprises a coiled-coil peptide.

4. The polypeptide according to claim 1 or claim 3, or a CAR according to claim 2 or claim 3, wherein each interaction domain comprises a cysteine at position 'd' of a heptad repeat.

5. The polypeptide according to any one of claims 1, or 3 to 4, or a CAR according to any one of claims 2 to 4, wherein each interaction domain comprises less than 50 amino acid residues.

6. The polypeptide according to any one of claims 1, or 3 to 5, or a CAR according to any one of claims 2 to 5, wherein the first interaction domain and/or second interaction domain comprises an amino acid sequence having at least 70% amino acid sequence identity to any one of SEQ ID NOs: 1-8.

7. The polypeptide according to any one of claims 1, or 3 to 6, or a CAR according to any one of claims 2 to 6, wherein the antigen-binding domain comprises an antibody-derived antigen-binding molecule, optionally wherein the antibody-derived antigen-binding molecule is a single chain variable fragment (scFv) or a variable domain of the heavy chain of a heavy-chain antibody (VHH).

8. The CAR according to any one of claims 2 to 7, wherein the CAR is a multispecific CAR comprising two antigen binding domains, and each antigen binding domain is independently stabilized by two interaction domains.

9. The CAR according to any one of claims 2 to 8, wherein the hinge comprises an amino acid sequence derived from a CD8α hinge, and/or wherein the transmembrane domain comprises an amino acid sequence derived from a CD8α transmembrane domain.

10. The CAR according to any one of claims 2 to 9, wherein the signalling domain comprises an amino acid sequence derived from a CD3ζ signalling domain, and/or an amino acid sequence derived from a 4-1BB costimulatory domain.

11. The polypeptide according to any one of claims 1, or 3 to 10, or a CAR according to any one of claims 2 to 10, wherein the target antigen is a tumor associated antigen or an antigen expressed on the surface of a cell involved in autoimmune disease.

12. A nucleic acid, or a plurality of nucleic acids, optionally isolated, encoding a polypeptide according to any one of claims 1, or 3 to 11, or a CAR according to any one of claims 2 to 11.

13. A cell comprising a polypeptide according to any one of claims 1, or 3 to 11, a CAR according to any one of claims 2 to 11, or a nucleic acid or a plurality of nucleic acids according to claim 12.

14. A composition comprising a polypeptide according to any one of claims 1, or 3 to 11, a CAR according to any one of claims 2 to 11, a nucleic acid or a plurality of nucleic acids according to claim 12, or a cell according to claim 13, and a pharmaceutically acceptable carrier, diluent, excipient or adjuvant.

15. A polypeptide according to any one of claims 1, or 3 to 11, a CAR according to any one of claims 2 to 11, a nucleic acid or a plurality of nucleic acids according to claim 12, a cell according to claim 13, or a composition according to claim 14, for use in the treatment or prevention of a cancer or an autoimmune disease.
